(19) Europäisches Patentamt
European Patent Office
Office européen des brevets

(11) EP 3 892 333 A1

(12) **EUROPEAN PATENT APPLICATION**
published in accordance with Art. 153(4) EPC

(43) Date of publication:
**13.10.2021 Bulletin 2021/41**

(21) Application number: **19892717.0**

(22) Date of filing: **09.12.2019**

(51) Int Cl.:
***A61P 35/00*** *(2006.01)* ***A61K 35/12*** *(2015.01)*
***A61K 31/00*** *(2006.01)*

(86) International application number:
**PCT/CN2019/124066**

(87) International publication number:
**WO 2020/114518 (11.06.2020 Gazette 2020/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.12.2018 CN 201811497155**

(71) Applicants:
- **Carsgen Therapeutics Co., Ltd.**
  **Shanghai 200231 (CN)**
- **Shanghai Cancer Institute**
  **Shanghai 200032 (CN)**

(72) Inventors:
- **LI, Zonghai**
  **Shanghai 200231 (CN)**
- **SUN, Ruixin**
  **Shanghai 200231 (CN)**

(74) Representative: **Lavoix**
**Bayerstraße 83**
**80335 München (DE)**

# (54) TUMOR COMBINED IMMUNOTHERAPY

(57) Provided is a method for tumor treatment, comprising administering immune effector cells and a PARP inhibitor to an individual suffering from a tumor, the immune effector cell expresses a receptor for identifying a tumor antigen. Further provided is a kit for tumor treatment.

EP 3 892 333 A1

SEQUENCE LISTING

<110> CARSGEN THERAPEUTICS CO., LTD
Shanghai cancer institute

<120> TUMOR COMBINED IMMUNOTHERAPY

<130> BET21M2040

<140> EP19892717.0
<141> 2019-12-09

<150> CN201811497155.5
<151> 2018-12-07

<160> 87

<170> PatentIn version 3.5

<210> 1
<211> 81
<212> DNA
<213> Artificial Sequence

<220>
<223> Description of Artificial Sequence: artificially synthesized sequence

<400> 1
atggcctcac cgttgacccg ctttctgtcg ctgaacctgc tgctgctggg tgagtcgatt 60

atcctgggga gtggagaagc t 81

<210> 2
<211> 720
<212> DNA
<213> Artificial Sequence

<220>
<223> Description of Artificial Sequence: artificially synthesized sequence

<400> 2
gacatcctga tgacccaatc tccatcctcc atgtctgtat ctctgggaga cacagtcagc 60

atcacttgcc attcaagtca ggacattaac agtaatatag ggtggttgca gcagagacca 120

gggaaatcat ttaagggcct gatctatcat ggaaccaact tggacgatga agttccatca 180

aggttcagtg gcagtggatc tggagccgat tattctctca ccatcagcag cctggaatct 240

gaagattttg cagactatta ctgtgtacag tatgctcagt ttccgtggac gttcggtgga 300

ggcaccaagc tggaaatcaa acgtggtgga ggcggttcag gcggaggtgg ctctggcggt 360

ggcggatcgg ccgatgtgca gcttcaggag tcgggaccta gcctggtgaa accttctcag 420

tctctgtccc tcacctgcac tgtcactggc tactcaatca ccagtgattt tgcctggaac 480

tggatccggc agtttccagg aaacaagctg gagtggatgg gctacataag ttatagtggt 540

aacactaggt acaacccatc tctcaaaagt cgaatctcta tcactcgaga cacatccaag 600

aaccaattct tcctgcagtt gaattctgtg actattgagg acacagccac atattactgt 660

gtaacggcgg gacgcgggtt tccttattgg ggccaaggga ctctggtcac tgtctctgca 720

<210> 3
<211> 216
<212> DNA
<213> Artificial Sequence

<220>
<223> Description of Artificial Sequence: artificially synthesized sequence

<400> 3
actactacca agccagtgct gcgaactccc tcacctgtgc accctaccgg gacatctcag 60

ccccagagac cagaagattg tcggccccgt ggctcagtga aggggaccgg attggacttc 120

gcctgtgata tttacatctg ggcacccttg gccggaatct gcgtggccct tctgctgtcc 180

ttgatcatca ctctcatctg ctaccacagg agccga 216

<210> 4
<211> 123
<212> DNA
<213> Artificial Sequence

<220>
<223> Description of Artificial Sequence: artificially synthesized sequence

<400> 4
aatagtagaa ggaacagact ccttcaaagt gactacatga acatgactcc ccggaggcct 60

gggctcactc gaaagcctta ccagccctac gcccctgcca gagactttgc agcgtaccgc 120

ccc 123

<210> 5
<211> 321
<212> DNA
<213> Artificial Sequence

<220>
<223> Description of Artificial Sequence: artificially synthesized sequence

<400> 5
agcaggagtg cagagactgc tgccaacctg caggacccca accagctcta caatgagctc 60

aatctagggc gaagagagga atatgacgtc ttggagaaga agcgggctcg ggatccagag 120

atgggaggca aacagcagag gaggaggaac ccccaggaag gcgtatacaa tgcactgcag 180

aaagacaaga tggcagaagc ctacagtgag atcggcacaa aaggcgagag gcggagaggc 240

aaggggcacg atggccttta ccagggtctc agcactgcca ccaaggacac ctatgatgcc 300

ctgcatatgc agaccctggc c 321

<210> 6
<211> 63
<212> DNA
<213> Artificial Sequence

<220>
<223> Description of Artificial Sequence: artificially synthesized sequence

<400> 6
atggccttac cagtgaccgc cttgctcctg ccgctggcct tgctgctcca cgccgccagg 60

ccg 63

<210> 7
<211> 198
<212> DNA
<213> Artificial Sequence

<220>
<223> Description of Artificial Sequence: artificially synthesized sequence

<400> 7
accacgacgc cagcgccgcg accaccaaca ccggcgccca ccatcgcgtc gcagcccctg 60

tccctgcgcc cagaggcgtg ccggccagcg gcggggggcg cagtgcacac gagggggctg 120

gacttcgcct gtgatatcta catctgggcg cccttggccg ggacttgtgg ggtccttctc 180

ctgtcactgg ttatcacc 198

<210> 8
<211> 123
<212> DNA
<213> Artificial Sequence

<220>
<223> Description of Artificial Sequence: artificially synthesized sequence

<400> 8
aggagtaaga ggagcaggct cctgcacagt gactacatga acatgactcc ccgccgcccc 60

gggccaaccc gcaagcatta ccagccctat gccccaccac gcgacttcgc agcctatcgc 120

tcc 123

<210> 9
<211> 339
<212> DNA
<213> Artificial Sequence

<220>
<223> Description of Artificial Sequence: artificially synthesized sequence

<400> 9

agagtgaagt tcagcaggag cgcagacgcc cccgcgtacc agcagggcca gaaccagctc 60

tataacgagc tcaatctagg acgaagagag gagtacgatg ttttggacaa gagacgtggc 120

cgggaccctg agatgggggg aaagccgcag agaaggaaga accctcagga aggcctgtac 180

aatgaactgc agaaagataa gatggcggag gcctacagtg agattgggat gaaaggcgag 240

cgccggaggg gcaaggggca cgatggcctt taccagggtc tcagtacagc caccaaggac 300

acctacgacg cccttcacat gcaggccctg ccccctcgc 339

<210> 10
<211> 81
<212> DNA
<213> Artificial Sequence

<220>
<223> Description of Artificial Sequence: artificially synthesized sequence

<400> 10
ttttgggtgc tggtggtggt tggtggagtc ctggcttgct atagcttgct agtaacagtg 60

gcctttatta ttttctgggt g 81

<210> 11
<211> 240
<212> PRT
<213> Artificial Sequence

<220>
<223> Description of Artificial Sequence: artificially synthesized sequence

<400> 11

Asp Ile Leu Met Thr Gln Ser Pro Ser Ser Met Ser Val Ser Leu Gly
1 5 10 15

Asp Thr Val Ser Ile Thr Cys His Ser Ser Gln Asp Ile Asn Ser Asn
20 25 30

Ile Gly Trp Leu Gln Gln Arg Pro Gly Lys Ser Phe Lys Gly Leu Ile
35 40 45

Tyr His Gly Thr Asn Leu Asp Asp Glu Val Pro Ser Arg Phe Ser Gly
50 55 60

Ser Gly Ser Gly Ala Asp Tyr Ser Leu Thr Ile Ser Ser Leu Glu Ser
65 70 75 80

Glu Asp Phe Ala Asp Tyr Tyr Cys Val Gln Tyr Ala Gln Phe Pro Trp
85 90 95

Thr Phe Gly Gly Gly Thr Lys Leu Glu Ile Lys Arg Gly Gly Gly Gly
100 105 110

Ser Gly Gly Gly Gly Ser Gly Gly Gly Gly Ser Ala Asp Val Gln Leu
115 120 125

Gln Glu Ser Gly Pro Ser Leu Val Lys Pro Ser Gln Ser Leu Ser Leu
130 135 140

Thr Cys Thr Val Thr Gly Tyr Ser Ile Thr Ser Asp Phe Ala Trp Asn
145 150 155 160

Trp Ile Arg Gln Phe Pro Gly Asn Lys Leu Glu Trp Met Gly Tyr Ile
165 170 175

Ser Tyr Ser Gly Asn Thr Arg Tyr Asn Pro Ser Leu Lys Ser Arg Ile
180 185 190

Ser Ile Thr Arg Asp Thr Ser Lys Asn Gln Phe Phe Leu Gln Leu Asn
195 200 205

Ser Val Thr Ile Glu Asp Thr Ala Thr Tyr Tyr Cys Val Thr Ala Gly
210 215 220

Arg Gly Phe Pro Tyr Trp Gly Gln Gly Thr Leu Val Thr Val Ser Ala
225 230 235 240

<210> 12
<211> 239
<212> PRT
<213> Artificial Sequence

<220>
<223> Description of Artificial Sequence: artificially synthesized sequence

<400> 12

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Ser Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

```
Ser Gly Ser Gly Thr Asp Phe Thr Leu Thr Ile Ser Ser Leu Gln Pro
65                  70                  75                  80

Glu Asp Phe Ala Thr Tyr Tyr Cys Val Gln Tyr Ala Gln Phe Pro Tyr
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Ser Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser
225                 230                 235


<210>  13
<211>  239
<212>  PRT
<213>  Artificial Sequence

<220>
<223>  Description of Artificial Sequence: artificially synthesized
       sequence

<400>  13

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30
```

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Ile Pro Leu
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Gln Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Met Gly Lys
210 215 220

Asn Trp Asp Tyr Trp Gly Gln Gly Thr Leu Val Thr Val Ser Ser
225 230 235

<210> 14
<211> 239
<212> PRT
<213> Artificial Sequence

<220>
<223> Description of Artificial Sequence: artificially synthesized sequence

<400> 14

```
Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Lys Val Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Ile
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Ser Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser
225                 230                 235
```

<210> 15
<211> 239
<212> PRT

<213> Artificial Sequence

<220>
<223> Description of Artificial Sequence: artificially synthesized sequence

<400> 15

```
Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Pro Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Ile
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Ser Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220
```

```
Gly Phe Arg Tyr Trp Gly Gln Gly Thr Leu Val Thr Val Ser Ser
225                 230                 235


<210>  16
<211>  239
<212>  PRT
<213>  Artificial Sequence

<220>
<223>  Description of Artificial Sequence: artificially synthesized
       sequence

<400>  16

Asp Ile Gln Met Thr Gln Ser Pro Ser Ser Leu Ser Ala Ser Val Gly
1               5                   10                  15


Asp Arg Val Thr Ile Thr Cys His Ala Ser Gln Asp Ile Asn Val Asn
            20                  25                  30


Ile Gly Trp Leu Gln Gln Lys Pro Gly Lys Ala Phe Lys Gly Leu Ile
        35                  40                  45


Tyr His Gly Lys Lys Leu Glu Asp Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Asn Gln Tyr Glu Asn Asn Pro Ile
                85                  90                  95


Thr Phe Gly Gln Gly Thr Lys Val Glu Ile Lys Arg Gly Gly Gly Gly
            100                 105                 110


Ser Gly Gly Gly Gly Ser Gly Gly Gly Gly Ser Asp Val Gln Leu Val
        115                 120                 125


Glu Ser Gly Gly Gly Leu Val Gln Pro Gly Gly Ser Leu Arg Leu Ser
    130                 135                 140


Cys Ala Val Ser Gly Tyr Ser Ile Thr Ser Asp Tyr Ala Trp Asn Trp
145                 150                 155                 160


Ile Arg Gln Ala Pro Gly Lys Gly Leu Glu Trp Leu Gly Tyr Ile Ser
                165                 170                 175


Tyr Arg Gly Arg Thr Ser Tyr Asn Pro Ser Leu Lys Ser Arg Ile Ser
            180                 185                 190
```

```
Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser
225                 230                 235


<210>  17
<211>  239
<212>  PRT
<213>  Artificial Sequence

<220>
<223>  Description of Artificial Sequence: artificially synthesized
       sequence

<400>  17

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Lys Tyr Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Ile
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160
```

```
Ile Arg Gln Ala Pro Gly Lys Gly Leu Glu Trp Leu Gly Tyr Ile Ser
                165                 170                 175

Tyr Arg Gly Arg Thr Ser Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser
225                 230                 235


<210>  18
<211>  239
<212>  PRT
<213>  Artificial Sequence

<220>
<223>  Description of Artificial Sequence: artificially synthesized
       sequence

<400>  18

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Ile
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125
```

```
Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Ser Gly Asn Thr Ser Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser
225                 230                 235
```

<210> 19
<211> 239
<212> PRT
<213> Artificial Sequence

<220>
<223> Description of Artificial Sequence: artificially synthesized sequence

<400> 19

```
Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Glu Val Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Leu
                85                  90                  95
```

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Arg Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser
225 230 235

<210> 20
<211> 239
<212> PRT
<213> Artificial Sequence

<220>
<223> Description of Artificial Sequence: artificially synthesized sequence

<400> 20

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Ser Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Asn Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser
225 230 235

<210> 21
<211> 239
<212> PRT
<213> Artificial Sequence

<220>
<223> Description of Artificial Sequence: artificially synthesized sequence

<400> 21

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Ser Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser
225 230 235

<210> 22
<211> 239
<212> PRT
<213> Artificial Sequence

<220>
<223> Description of Artificial Sequence: artificially synthesized sequence

<400> 22

```
Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Ile Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Arg Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser
225                 230                 235
```

<210> 23
<211> 239

<212> PRT
<213> Artificial Sequence

<220>
<223> Description of Artificial Sequence: artificially synthesized sequence

<400> 23

```
Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Ile Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Gln Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220
```

Gly Phe Arg Tyr Trp Gly Gln Gly Thr Leu Val Thr Val Ser Ser
225 230 235

<210> 24
<211> 239
<212> PRT
<213> Artificial Sequence

<220>
<223> Description of Artificial Sequence: artificially synthesized sequence

<400> 24

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Thr Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Ser Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser
225 230 235

<210> 25
<211> 239
<212> PRT
<213> Artificial Sequence

<220>
<223> Description of Artificial Sequence: artificially synthesized sequence

<400> 25

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ser Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Ser Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser
225 230 235

<210> 26
<211> 239
<212> PRT
<213> Artificial Sequence

<220>
<223> Description of Artificial Sequence: artificially synthesized sequence

<400> 26

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Thr Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Leu
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Gln Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser
225 230 235

<210> 27
<211> 239
<212> PRT
<213> Artificial Sequence

<220>
<223> Description of Artificial Sequence: artificially synthesized sequence

<400> 27

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Thr Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Leu
85 90 95

```
Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Arg Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser
225                 230                 235


<210>  28
<211>  239
<212>  PRT
<213>  Artificial Sequence

<220>
<223>  Description of Artificial Sequence: artificially synthesized
       sequence

<400>  28

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ser Phe Lys Gly Leu Ile
        35                  40                  45

Tyr His Gly Lys Asn Leu Glu Asp Gly Val Pro Ser Arg Phe Ser Gly
    50                  55                  60
```

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Arg Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser
225                 230                 235

<210> 29
<211> 239
<212> PRT
<213> Artificial Sequence

<220>
<223> Description of Artificial Sequence: artificially synthesized sequence

<400> 29

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

```
Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Phe Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Arg Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser
225                 230                 235
```

<210> 30
<211> 460
<212> PRT
<213> Artificial Sequence

<220>
<223> Description of Artificial Sequence: artificially synthesized sequence

<400> 30

Asp Ile Leu Met Thr Gln Ser Pro Ser Ser Met Ser Val Ser Leu Gly
1 5 10 15

Asp Thr Val Ser Ile Thr Cys His Ser Ser Gln Asp Ile Asn Ser Asn
20 25 30

Ile Gly Trp Leu Gln Gln Arg Pro Gly Lys Ser Phe Lys Gly Leu Ile
35 40 45

Tyr His Gly Thr Asn Leu Asp Asp Glu Val Pro Ser Arg Phe Ser Gly
50 55 60

Ser Gly Ser Gly Ala Asp Tyr Ser Leu Thr Ile Ser Ser Leu Glu Ser
65 70 75 80

Glu Asp Phe Ala Asp Tyr Tyr Cys Val Gln Tyr Ala Gln Phe Pro Trp
85 90 95

Thr Phe Gly Gly Gly Thr Lys Leu Glu Ile Lys Arg Gly Gly Gly Gly
100 105 110

Ser Gly Gly Gly Gly Ser Gly Gly Gly Gly Ser Ala Asp Val Gln Leu
115 120 125

Gln Glu Ser Gly Pro Ser Leu Val Lys Pro Ser Gln Ser Leu Ser Leu
130 135 140

Thr Cys Thr Val Thr Gly Tyr Ser Ile Thr Ser Asp Phe Ala Trp Asn
145 150 155 160

Trp Ile Arg Gln Phe Pro Gly Asn Lys Leu Glu Trp Met Gly Tyr Ile
165 170 175

Ser Tyr Ser Gly Asn Thr Arg Tyr Asn Pro Ser Leu Lys Ser Arg Ile
180 185 190

Ser Ile Thr Arg Asp Thr Ser Lys Asn Gln Phe Phe Leu Gln Leu Asn
195 200 205

Ser Val Thr Ile Glu Asp Thr Ala Thr Tyr Tyr Cys Val Thr Ala Gly
210 215 220

Arg Gly Phe Pro Tyr Trp Gly Gln Gly Thr Leu Val Thr Val Ser Ala
225 230 235 240

Thr Thr Thr Lys Pro Val Leu Arg Thr Pro Ser Pro Val His Pro Thr

245 250 255

Gly Thr Ser Gln Pro Gln Arg Pro Glu Asp Cys Arg Pro Arg Gly Ser
260 265 270

Val Lys Gly Thr Gly Leu Asp Phe Ala Cys Asp Ile Tyr Ile Trp Ala
275 280 285

Pro Leu Ala Gly Ile Cys Val Ala Leu Leu Leu Ser Leu Ile Ile Thr
290 295 300

Leu Ile Cys Tyr His Arg Ser Arg Asn Ser Arg Arg Asn Arg Leu Leu
305 310 315 320

Gln Ser Asp Tyr Met Asn Met Thr Pro Arg Arg Pro Gly Leu Thr Arg
325 330 335

Lys Pro Tyr Gln Pro Tyr Ala Pro Ala Arg Asp Phe Ala Ala Tyr Arg
340 345 350

Pro Ser Arg Ser Ala Glu Thr Ala Ala Asn Leu Gln Asp Pro Asn Gln
355 360 365

Leu Tyr Asn Glu Leu Asn Leu Gly Arg Arg Glu Glu Tyr Asp Val Leu
370 375 380

Glu Lys Lys Arg Ala Arg Asp Pro Glu Met Gly Gly Lys Gln Gln Arg
385 390 395 400

Arg Arg Asn Pro Gln Glu Gly Val Tyr Asn Ala Leu Gln Lys Asp Lys
405 410 415

Met Ala Glu Ala Tyr Ser Glu Ile Gly Thr Lys Gly Glu Arg Arg Arg
420 425 430

Gly Lys Gly His Asp Gly Leu Tyr Gln Gly Leu Ser Thr Ala Thr Lys
435 440 445

Asp Thr Tyr Asp Ala Leu His Met Gln Thr Leu Ala
450 455 460

<210> 31
<211> 465
<212> PRT
<213> Artificial Sequence

<220>
<223> Description of Artificial Sequence: artificially synthesized sequence

<400> 31

```
Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Ile Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Gln Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Met Gly Lys
    210                 215                 220

Asn Trp Asp Tyr Trp Gly Gln Gly Thr Leu Val Thr Val Ser Ser Thr
225                 230                 235                 240
```

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Phe Trp Val Leu
275 280 285

Val Val Val Gly Gly Val Leu Ala Cys Tyr Ser Leu Leu Val Thr Val
290 295 300

Ala Phe Ile Ile Phe Trp Val Arg Ser Lys Arg Ser Arg Leu Leu His
305 310 315 320

Ser Asp Tyr Met Asn Met Thr Pro Arg Arg Pro Gly Pro Thr Arg Lys
325 330 335

His Tyr Gln Pro Tyr Ala Pro Pro Arg Asp Phe Ala Ala Tyr Arg Ser
340 345 350

Arg Val Lys Phe Ser Arg Ser Ala Asp Ala Pro Ala Tyr Gln Gln Gly
355 360 365

Gln Asn Gln Leu Tyr Asn Glu Leu Asn Leu Gly Arg Arg Glu Glu Tyr
370 375 380

Asp Val Leu Asp Lys Arg Arg Gly Arg Asp Pro Glu Met Gly Gly Lys
385 390 395 400

Pro Gln Arg Arg Lys Asn Pro Gln Glu Gly Leu Tyr Asn Glu Leu Gln
405 410 415

Lys Asp Lys Met Ala Glu Ala Tyr Ser Glu Ile Gly Met Lys Gly Glu
420 425 430

Arg Arg Arg Gly Lys Gly His Asp Gly Leu Tyr Gln Gly Leu Ser Thr
435 440 445

Ala Thr Lys Asp Thr Tyr Asp Ala Leu His Met Gln Ala Leu Pro Pro
450 455 460

Arg
465

<210> 32
<211> 462
<212> PRT

<213> Artificial Sequence

<220>
<223> Description of Artificial Sequence: artificially synthesized sequence

<400> 32

```
Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Ile Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Gln Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Met Gly Lys
    210                 215                 220
```

```
Asn Trp Asp Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Ile Tyr Ile Trp
        275                 280                 285

Ala Pro Leu Ala Gly Thr Cys Gly Val Leu Leu Leu Ser Leu Val Ile
    290                 295                 300

Thr Leu Tyr Cys Lys Arg Gly Arg Lys Lys Leu Leu Tyr Ile Phe Lys
305                 310                 315                 320

Gln Pro Phe Met Arg Pro Val Gln Thr Thr Gln Glu Glu Asp Gly Cys
                325                 330                 335

Ser Cys Arg Phe Pro Glu Glu Glu Glu Gly Gly Cys Glu Leu Arg Val
            340                 345                 350

Lys Phe Ser Arg Ser Ala Asp Ala Pro Ala Tyr Lys Gln Gly Gln Asn
        355                 360                 365

Gln Leu Tyr Asn Glu Leu Asn Leu Gly Arg Arg Glu Glu Tyr Asp Val
    370                 375                 380

Leu Asp Lys Arg Arg Gly Arg Asp Pro Glu Met Gly Gly Lys Pro Arg
385                 390                 395                 400

Arg Lys Asn Pro Gln Glu Gly Leu Tyr Asn Glu Leu Gln Lys Asp Lys
                405                 410                 415

Met Ala Glu Ala Tyr Ser Glu Ile Gly Met Lys Gly Glu Arg Arg Arg
            420                 425                 430

Gly Lys Gly His Asp Gly Leu Tyr Gln Gly Leu Ser Thr Ala Thr Lys
        435                 440                 445

Asp Thr Tyr Asp Ala Leu His Met Gln Ala Leu Pro Pro Arg
    450                 455                 460
```

<210> 33
<211> 507

<212> PRT
<213> Artificial Sequence

<220>
<223> Description of Artificial Sequence: artificially synthesized sequence

<400> 33

```
Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Ile Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Gln Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Met Gly Lys
    210                 215                 220
```

Asn Trp Asp Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Lys Arg Gly Arg Lys Lys Leu Leu Tyr Ile Phe Lys Gln Pro Phe Met
        355                 360                 365

Arg Pro Val Gln Thr Thr Gln Glu Glu Asp Gly Cys Ser Cys Arg Phe
    370                 375                 380

Pro Glu Glu Glu Glu Gly Gly Cys Glu Leu Arg Val Lys Phe Ser Arg
385                 390                 395                 400

Ser Ala Asp Ala Pro Ala Tyr Gln Gln Gly Gln Asn Gln Leu Tyr Asn
                405                 410                 415

Glu Leu Asn Leu Gly Arg Arg Glu Glu Tyr Asp Val Leu Asp Lys Arg
            420                 425                 430

Arg Gly Arg Asp Pro Glu Met Gly Gly Lys Pro Gln Arg Arg Lys Asn
        435                 440                 445

Pro Gln Glu Gly Leu Tyr Asn Glu Leu Gln Lys Asp Lys Met Ala Glu
    450                 455                 460

Ala Tyr Ser Glu Ile Gly Met Lys Gly Glu Arg Arg Arg Gly Lys Gly

465 470 475 480

His Asp Gly Leu Tyr Gln Gly Leu Ser Thr Ala Thr Lys Asp Thr Tyr
485 490 495

Asp Ala Leu His Met Gln Ala Leu Pro Pro Arg
500 505

<210> 34
<211> 465
<212> PRT
<213> Artificial Sequence

<220>
<223> Description of Artificial Sequence: artificially synthesized sequence

<400> 34

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Lys Val Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser

```
                165                 170                 175

Tyr Arg Gly Arg Thr Ser Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Arg Val Lys Phe Ser Arg Ser Ala Asp Ala Pro Ala Tyr Gln Gln Gly
        355                 360                 365

Gln Asn Gln Leu Tyr Asn Glu Leu Asn Leu Gly Arg Arg Glu Glu Tyr
    370                 375                 380

Asp Val Leu Asp Lys Arg Arg Gly Arg Asp Pro Glu Met Gly Gly Lys
385                 390                 395                 400

Pro Gln Arg Arg Lys Asn Pro Gln Glu Gly Leu Tyr Asn Glu Leu Gln
                405                 410                 415
```

Lys Asp Lys Met Ala Glu Ala Tyr Ser Glu Ile Gly Met Lys Gly Glu
420 425 430

Arg Arg Arg Gly Lys Gly His Asp Gly Leu Tyr Gln Gly Leu Ser Thr
435 440 445

Ala Thr Lys Asp Thr Tyr Asp Ala Leu His Met Gln Ala Leu Pro Pro
450 455 460

Arg
465

<210> 35
<211> 462
<212> PRT
<213> Artificial Sequence

<220>
<223> Description of Artificial Sequence: artificially synthesized sequence

<400> 35

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Lys Val Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Ser Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Ile Tyr Ile Trp
275 280 285

Ala Pro Leu Ala Gly Thr Cys Gly Val Leu Leu Leu Ser Leu Val Ile
290 295 300

Thr Leu Tyr Cys Lys Arg Gly Arg Lys Lys Leu Leu Tyr Ile Phe Lys
305 310 315 320

Gln Pro Phe Met Arg Pro Val Gln Thr Thr Gln Glu Glu Asp Gly Cys
325 330 335

Ser Cys Arg Phe Pro Glu Glu Glu Glu Gly Gly Cys Glu Leu Arg Val
340 345 350

Lys Phe Ser Arg Ser Ala Asp Ala Pro Ala Tyr Lys Gln Gly Gln Asn
355 360 365

Gln Leu Tyr Asn Glu Leu Asn Leu Gly Arg Arg Glu Glu Tyr Asp Val
370 375 380

Leu Asp Lys Arg Arg Gly Arg Asp Pro Glu Met Gly Gly Lys Pro Arg
385 390 395 400

Arg Lys Asn Pro Gln Glu Gly Leu Tyr Asn Glu Leu Gln Lys Asp Lys
405 410 415

Met Ala Glu Ala Tyr Ser Glu Ile Gly Met Lys Gly Glu Arg Arg Arg
420 425 430

Gly Lys Gly His Asp Gly Leu Tyr Gln Gly Leu Ser Thr Ala Thr Lys
435 440 445

Asp Thr Tyr Asp Ala Leu His Met Gln Ala Leu Pro Pro Arg
450 455 460

<210> 36
<211> 507
<212> PRT
<213> Artificial Sequence

<220>
<223> Description of Artificial Sequence: artificially synthesized sequence

<400> 36

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Lys Val Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Ser Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Phe Trp Val Leu
275 280 285

Val Val Val Gly Gly Val Leu Ala Cys Tyr Ser Leu Leu Val Thr Val
290 295 300

Ala Phe Ile Ile Phe Trp Val Arg Ser Lys Arg Ser Arg Leu Leu His
305 310 315 320

Ser Asp Tyr Met Asn Met Thr Pro Arg Arg Pro Gly Pro Thr Arg Lys
325 330 335

His Tyr Gln Pro Tyr Ala Pro Pro Arg Asp Phe Ala Ala Tyr Arg Ser
340 345 350

Lys Arg Gly Arg Lys Lys Leu Leu Tyr Ile Phe Lys Gln Pro Phe Met
355 360 365

Arg Pro Val Gln Thr Thr Gln Glu Glu Asp Gly Cys Ser Cys Arg Phe
370 375 380

Pro Glu Glu Glu Glu Gly Gly Cys Glu Leu Arg Val Lys Phe Ser Arg
385 390 395 400

Ser Ala Asp Ala Pro Ala Tyr Gln Gln Gly Gln Asn Gln Leu Tyr Asn
405 410 415

Glu Leu Asn Leu Gly Arg Arg Glu Glu Tyr Asp Val Leu Asp Lys Arg
420 425 430

Arg Gly Arg Asp Pro Glu Met Gly Gly Lys Pro Gln Arg Arg Lys Asn
435 440 445

Pro Gln Glu Gly Leu Tyr Asn Glu Leu Gln Lys Asp Lys Met Ala Glu
450 455 460

Ala Tyr Ser Glu Ile Gly Met Lys Gly Glu Arg Arg Arg Gly Lys Gly
465 470 475 480

His Asp Gly Leu Tyr Gln Gly Leu Ser Thr Ala Thr Lys Asp Thr Tyr
485 490 495

Asp Ala Leu His Met Gln Ala Leu Pro Pro Arg
500 505

<210> 37
<211> 465
<212> PRT
<213> Artificial Sequence

<220>
<223> Description of Artificial Sequence: artificially synthesized sequence

<400> 37

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Pro Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Ser Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Phe Trp Val Leu
275 280 285

Val Val Val Gly Gly Val Leu Ala Cys Tyr Ser Leu Leu Val Thr Val
290 295 300

Ala Phe Ile Ile Phe Trp Val Arg Ser Lys Arg Ser Arg Leu Leu His
305 310 315 320

Ser Asp Tyr Met Asn Met Thr Pro Arg Arg Pro Gly Pro Thr Arg Lys
325 330 335

His Tyr Gln Pro Tyr Ala Pro Pro Arg Asp Phe Ala Ala Tyr Arg Ser

340 345 350

Arg Val Lys Phe Ser Arg Ser Ala Asp Ala Pro Ala Tyr Gln Gln Gly
355 360 365

Gln Asn Gln Leu Tyr Asn Glu Leu Asn Leu Gly Arg Arg Glu Glu Tyr
370 375 380

Asp Val Leu Asp Lys Arg Arg Gly Arg Asp Pro Glu Met Gly Gly Lys
385 390 395 400

Pro Gln Arg Arg Lys Asn Pro Gln Glu Gly Leu Tyr Asn Glu Leu Gln
405 410 415

Lys Asp Lys Met Ala Glu Ala Tyr Ser Glu Ile Gly Met Lys Gly Glu
420 425 430

Arg Arg Arg Gly Lys Gly His Asp Gly Leu Tyr Gln Gly Leu Ser Thr
435 440 445

Ala Thr Lys Asp Thr Tyr Asp Ala Leu His Met Gln Ala Leu Pro Pro
450 455 460

Arg
465

<210> 38
<211> 462
<212> PRT
<213> Artificial Sequence

<220>
<223> Description of Artificial Sequence: artificially synthesized sequence

<400> 38

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Pro Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro

```
65                  70                  75                  80

Glu Asp Phe Ala Thr Tyr Tyr Cys Asn Gln Tyr Glu Asn Asn Pro Ile
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Ser Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Ile Tyr Ile Trp
        275                 280                 285

Ala Pro Leu Ala Gly Thr Cys Gly Val Leu Leu Leu Ser Leu Val Ile
    290                 295                 300

Thr Leu Tyr Cys Lys Arg Gly Arg Lys Lys Leu Leu Tyr Ile Phe Lys
305                 310                 315                 320
```

Gln Pro Phe Met Arg Pro Val Gln Thr Thr Gln Glu Glu Asp Gly Cys
325 330 335

Ser Cys Arg Phe Pro Glu Glu Glu Glu Gly Gly Cys Glu Leu Arg Val
340 345 350

Lys Phe Ser Arg Ser Ala Asp Ala Pro Ala Tyr Lys Gln Gly Gln Asn
355 360 365

Gln Leu Tyr Asn Glu Leu Asn Leu Gly Arg Arg Glu Glu Tyr Asp Val
370 375 380

Leu Asp Lys Arg Arg Gly Arg Asp Pro Glu Met Gly Gly Lys Pro Arg
385 390 395 400

Arg Lys Asn Pro Gln Glu Gly Leu Tyr Asn Glu Leu Gln Lys Asp Lys
405 410 415

Met Ala Glu Ala Tyr Ser Glu Ile Gly Met Lys Gly Glu Arg Arg Arg
420 425 430

Gly Lys Gly His Asp Gly Leu Tyr Gln Gly Leu Ser Thr Ala Thr Lys
435 440 445

Asp Thr Tyr Asp Ala Leu His Met Gln Ala Leu Pro Pro Arg
450 455 460

<210> 39
<211> 507
<212> PRT
<213> Artificial Sequence

<220>
<223> Description of Artificial Sequence: artificially synthesized sequence

<400> 39

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Pro Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Ser Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Phe Trp Val Leu
275 280 285

Val Val Val Gly Gly Val Leu Ala Cys Tyr Ser Leu Leu Val Thr Val
290 295 300

Ala Phe Ile Ile Phe Trp Val Arg Ser Lys Arg Ser Arg Leu Leu His
305 310 315 320

Ser Asp Tyr Met Asn Met Thr Pro Arg Arg Pro Gly Pro Thr Arg Lys
325 330 335

His Tyr Gln Pro Tyr Ala Pro Pro Arg Asp Phe Ala Ala Tyr Arg Ser
340 345 350

Lys Arg Gly Arg Lys Lys Leu Leu Tyr Ile Phe Lys Gln Pro Phe Met
355 360 365

Arg Pro Val Gln Thr Thr Gln Glu Glu Asp Gly Cys Ser Cys Arg Phe
370 375 380

Pro Glu Glu Glu Glu Gly Gly Cys Glu Leu Arg Val Lys Phe Ser Arg
385 390 395 400

Ser Ala Asp Ala Pro Ala Tyr Gln Gln Gly Gln Asn Gln Leu Tyr Asn
405 410 415

Glu Leu Asn Leu Gly Arg Arg Glu Glu Tyr Asp Val Leu Asp Lys Arg
420 425 430

Arg Gly Arg Asp Pro Glu Met Gly Gly Lys Pro Gln Arg Arg Lys Asn
435 440 445

Pro Gln Glu Gly Leu Tyr Asn Glu Leu Gln Lys Asp Lys Met Ala Glu
450 455 460

Ala Tyr Ser Glu Ile Gly Met Lys Gly Glu Arg Arg Arg Gly Lys Gly
465 470 475 480

His Asp Gly Leu Tyr Gln Gly Leu Ser Thr Ala Thr Lys Asp Thr Tyr
485 490 495

Asp Ala Leu His Met Gln Ala Leu Pro Pro Arg
500 505

<210> 40
<211> 465
<212> PRT
<213> Artificial Sequence

<220>
<223> Description of Artificial Sequence: artificially synthesized sequence

<400> 40

Asp Ile Gln Met Thr Gln Ser Pro Ser Ser Leu Ser Ala Ser Val Gly
1 5 10 15

```
Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Lys Lys Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Ile
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Ser Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270
```

```
Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Arg Val Lys Phe Ser Arg Ser Ala Asp Ala Pro Ala Tyr Gln Gln Gly
        355                 360                 365

Gln Asn Gln Leu Tyr Asn Glu Leu Asn Leu Gly Arg Arg Glu Glu Tyr
    370                 375                 380

Asp Val Leu Asp Lys Arg Arg Gly Arg Asp Pro Glu Met Gly Gly Lys
385                 390                 395                 400

Pro Gln Arg Arg Lys Asn Pro Gln Glu Gly Leu Tyr Asn Glu Leu Gln
                405                 410                 415

Lys Asp Lys Met Ala Glu Ala Tyr Ser Glu Ile Gly Met Lys Gly Glu
            420                 425                 430

Arg Arg Arg Gly Lys Gly His Asp Gly Leu Tyr Gln Gly Leu Ser Thr
        435                 440                 445

Ala Thr Lys Asp Thr Tyr Asp Ala Leu His Met Gln Ala Leu Pro Pro
    450                 455                 460

Arg
465
```

<210> 41
<211> 462
<212> PRT
<213> Artificial Sequence

<220>
<223> Description of Artificial Sequence: artificially synthesized sequence

<400> 41

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Lys Lys Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Ile
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Ser Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser

```
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Ile Tyr Ile Trp
        275                 280                 285

Ala Pro Leu Ala Gly Thr Cys Gly Val Leu Leu Leu Ser Leu Val Ile
    290                 295                 300

Thr Leu Tyr Cys Lys Arg Gly Arg Lys Lys Leu Leu Tyr Ile Phe Lys
305                 310                 315                 320

Gln Pro Phe Met Arg Pro Val Gln Thr Thr Gln Glu Glu Asp Gly Cys
                325                 330                 335

Ser Cys Arg Phe Pro Glu Glu Glu Glu Gly Gly Cys Glu Leu Arg Val
            340                 345                 350

Lys Phe Ser Arg Ser Ala Asp Ala Pro Ala Tyr Lys Gln Gly Gln Asn
        355                 360                 365

Gln Leu Tyr Asn Glu Leu Asn Leu Gly Arg Arg Glu Glu Tyr Asp Val
    370                 375                 380

Leu Asp Lys Arg Arg Gly Arg Asp Pro Glu Met Gly Gly Lys Pro Arg
385                 390                 395                 400

Arg Lys Asn Pro Gln Glu Gly Leu Tyr Asn Glu Leu Gln Lys Asp Lys
                405                 410                 415

Met Ala Glu Ala Tyr Ser Glu Ile Gly Met Lys Gly Glu Arg Arg Arg
            420                 425                 430

Gly Lys Gly His Asp Gly Leu Tyr Gln Gly Leu Ser Thr Ala Thr Lys
        435                 440                 445

Asp Thr Tyr Asp Ala Leu His Met Gln Ala Leu Pro Pro Arg
    450                 455                 460
```

<210> 42
<211> 507
<212> PRT
<213> Artificial Sequence

<220>
<223> Description of Artificial Sequence: artificially synthesized sequence

<400> 42

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Lys Lys Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Ser Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

```
Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Lys Arg Gly Arg Lys Lys Leu Leu Tyr Ile Phe Lys Gln Pro Phe Met
        355                 360                 365

Arg Pro Val Gln Thr Thr Gln Glu Glu Asp Gly Cys Ser Cys Arg Phe
    370                 375                 380

Pro Glu Glu Glu Glu Gly Gly Cys Glu Leu Arg Val Lys Phe Ser Arg
385                 390                 395                 400

Ser Ala Asp Ala Pro Ala Tyr Gln Gln Gly Gln Asn Gln Leu Tyr Asn
                405                 410                 415

Glu Leu Asn Leu Gly Arg Arg Glu Glu Tyr Asp Val Leu Asp Lys Arg
            420                 425                 430

Arg Gly Arg Asp Pro Glu Met Gly Gly Lys Pro Gln Arg Arg Lys Asn
        435                 440                 445

Pro Gln Glu Gly Leu Tyr Asn Glu Leu Gln Lys Asp Lys Met Ala Glu
    450                 455                 460

Ala Tyr Ser Glu Ile Gly Met Lys Gly Glu Arg Arg Arg Gly Lys Gly
465                 470                 475                 480

His Asp Gly Leu Tyr Gln Gly Leu Ser Thr Ala Thr Lys Asp Thr Tyr
                485                 490                 495
```

Asp Ala Leu His Met Gln Ala Leu Pro Pro Arg
500 505

<210> 43
<211> 465
<212> PRT
<213> Artificial Sequence

<220>
<223> Description of Artificial Sequence: artificially synthesized sequence

<400> 43

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Lys Tyr Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Ser Tyr Asn Pro Ser Leu Lys Ser Arg Ile Ser
180 185 190

```
Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Arg Val Lys Phe Ser Arg Ser Ala Asp Ala Pro Ala Tyr Gln Gln Gly
        355                 360                 365

Gln Asn Gln Leu Tyr Asn Glu Leu Asn Leu Gly Arg Arg Glu Glu Tyr
    370                 375                 380

Asp Val Leu Asp Lys Arg Arg Gly Arg Asp Pro Glu Met Gly Gly Lys
385                 390                 395                 400

Pro Gln Arg Arg Lys Asn Pro Gln Glu Gly Leu Tyr Asn Glu Leu Gln
                405                 410                 415

Lys Asp Lys Met Ala Glu Ala Tyr Ser Glu Ile Gly Met Lys Gly Glu
            420                 425                 430

Arg Arg Arg Gly Lys Gly His Asp Gly Leu Tyr Gln Gly Leu Ser Thr
        435                 440                 445
```

Ala Thr Lys Asp Thr Tyr Asp Ala Leu His Met Gln Ala Leu Pro Pro
    450                 455                 460

Arg
465

<210> 44
<211> 462
<212> PRT
<213> Artificial Sequence

<220>
<223> Description of Artificial Sequence: artificially synthesized sequence

<400> 44

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Lys Tyr Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Ile
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

```
Tyr Arg Gly Arg Thr Ser Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Ile Tyr Ile Trp
        275                 280                 285

Ala Pro Leu Ala Gly Thr Cys Gly Val Leu Leu Leu Ser Leu Val Ile
    290                 295                 300

Thr Leu Tyr Cys Lys Arg Gly Arg Lys Lys Leu Leu Tyr Ile Phe Lys
305                 310                 315                 320

Gln Pro Phe Met Arg Pro Val Gln Thr Thr Gln Glu Glu Asp Gly Cys
                325                 330                 335

Ser Cys Arg Phe Pro Glu Glu Glu Glu Gly Gly Cys Glu Leu Arg Val
            340                 345                 350

Lys Phe Ser Arg Ser Ala Asp Ala Pro Ala Tyr Lys Gln Gly Gln Asn
        355                 360                 365

Gln Leu Tyr Asn Glu Leu Asn Leu Gly Arg Arg Glu Glu Tyr Asp Val
    370                 375                 380

Leu Asp Lys Arg Arg Gly Arg Asp Pro Glu Met Gly Gly Lys Pro Arg
385                 390                 395                 400

Arg Lys Asn Pro Gln Glu Gly Leu Tyr Asn Glu Leu Gln Lys Asp Lys
                405                 410                 415

Met Ala Glu Ala Tyr Ser Glu Ile Gly Met Lys Gly Glu Arg Arg Arg
```

420 425 430

Gly Lys Gly His Asp Gly Leu Tyr Gln Gly Leu Ser Thr Ala Thr Lys
435 440 445

Asp Thr Tyr Asp Ala Leu His Met Gln Ala Leu Pro Pro Arg
450 455 460

<210> 45
<211> 507
<212> PRT
<213> Artificial Sequence

<220>
<223> Description of Artificial Sequence: artificially synthesized sequence

<400> 45

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Lys Tyr Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser

165 170 175

Tyr Arg Gly Arg Thr Ser Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Phe Trp Val Leu
275 280 285

Val Val Val Gly Gly Val Leu Ala Cys Tyr Ser Leu Leu Val Thr Val
290 295 300

Ala Phe Ile Ile Phe Trp Val Arg Ser Lys Arg Ser Arg Leu Leu His
305 310 315 320

Ser Asp Tyr Met Asn Met Thr Pro Arg Arg Pro Gly Pro Thr Arg Lys
325 330 335

His Tyr Gln Pro Tyr Ala Pro Pro Arg Asp Phe Ala Ala Tyr Arg Ser
340 345 350

Lys Arg Gly Arg Lys Lys Leu Leu Tyr Ile Phe Lys Gln Pro Phe Met
355 360 365

Arg Pro Val Gln Thr Thr Gln Glu Glu Asp Gly Cys Ser Cys Arg Phe
370 375 380

Pro Glu Glu Glu Glu Gly Gly Cys Glu Leu Arg Val Lys Phe Ser Arg
385 390 395 400

Ser Ala Asp Ala Pro Ala Tyr Gln Gln Gly Gln Asn Gln Leu Tyr Asn
405 410 415

```
Glu Leu Asn Leu Gly Arg Arg Glu Glu Tyr Asp Val Leu Asp Lys Arg
            420                 425                 430

Arg Gly Arg Asp Pro Glu Met Gly Gly Lys Pro Gln Arg Arg Lys Asn
        435                 440                 445

Pro Gln Glu Gly Leu Tyr Asn Glu Leu Gln Lys Asp Lys Met Ala Glu
    450                 455                 460

Ala Tyr Ser Glu Ile Gly Met Lys Gly Glu Arg Arg Arg Gly Lys Gly
465                 470                 475                 480

His Asp Gly Leu Tyr Gln Gly Leu Ser Thr Ala Thr Lys Asp Thr Tyr
                485                 490                 495

Asp Ala Leu His Met Gln Ala Leu Pro Pro Arg
            500                 505


<210>  46
<211>  465
<212>  PRT
<213>  Artificial Sequence

<220>
<223>  Description of Artificial Sequence: artificially synthesized
       sequence

<400>  46

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Ile
                85                  90                  95

Thr Phe Gly Gln Gly Thr Lys Val Glu Ile Lys Arg Gly Gly Gly Gly
            100                 105                 110
```

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Ser Gly Asn Thr Ser Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Phe Trp Val Leu
275 280 285

Val Val Val Gly Gly Val Leu Ala Cys Tyr Ser Leu Leu Val Thr Val
290 295 300

Ala Phe Ile Ile Phe Trp Val Arg Ser Lys Arg Ser Arg Leu Leu His
305 310 315 320

Ser Asp Tyr Met Asn Met Thr Pro Arg Arg Pro Gly Pro Thr Arg Lys
325 330 335

His Tyr Gln Pro Tyr Ala Pro Pro Arg Asp Phe Ala Ala Tyr Arg Ser
340 345 350

Arg Val Lys Phe Ser Arg Ser Ala Asp Ala Pro Ala Tyr Gln Gln Gly
355 360 365

Gln Asn Gln Leu Tyr Asn Glu Leu Asn Leu Gly Arg Arg Glu Glu Tyr
    370                 375                 380

Asp Val Leu Asp Lys Arg Arg Gly Arg Asp Pro Glu Met Gly Gly Lys
385                 390                 395                 400

Pro Gln Arg Arg Lys Asn Pro Gln Glu Gly Leu Tyr Asn Glu Leu Gln
                405                 410                 415

Lys Asp Lys Met Ala Glu Ala Tyr Ser Glu Ile Gly Met Lys Gly Glu
            420                 425                 430

Arg Arg Arg Gly Lys Gly His Asp Gly Leu Tyr Gln Gly Leu Ser Thr
        435                 440                 445

Ala Thr Lys Asp Thr Tyr Asp Ala Leu His Met Gln Ala Leu Pro Pro
    450                 455                 460

Arg
465

<210> 47
<211> 462
<212> PRT
<213> Artificial Sequence

<220>
<223> Description of Artificial Sequence: artificially synthesized sequence

<400> 47

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Ile
                85                  90                  95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Ser Gly Asn Thr Ser Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Ile Tyr Ile Trp
275 280 285

Ala Pro Leu Ala Gly Thr Cys Gly Val Leu Leu Leu Ser Leu Val Ile
290 295 300

Thr Leu Tyr Cys Lys Arg Gly Arg Lys Lys Leu Leu Tyr Ile Phe Lys
305 310 315 320

Gln Pro Phe Met Arg Pro Val Gln Thr Thr Gln Glu Glu Asp Gly Cys
325 330 335

Ser Cys Arg Phe Pro Glu Glu Glu Glu Gly Gly Cys Glu Leu Arg Val
340 345 350

Lys Phe Ser Arg Ser Ala Asp Ala Pro Ala Tyr Lys Gln Gly Gln Asn
355 360 365

Gln Leu Tyr Asn Glu Leu Asn Leu Gly Arg Arg Glu Glu Tyr Asp Val
370 375 380

Leu Asp Lys Arg Arg Gly Arg Asp Pro Glu Met Gly Gly Lys Pro Arg
385 390 395 400

Arg Lys Asn Pro Gln Glu Gly Leu Tyr Asn Glu Leu Gln Lys Asp Lys
405 410 415

Met Ala Glu Ala Tyr Ser Glu Ile Gly Met Lys Gly Glu Arg Arg Arg
420 425 430

Gly Lys Gly His Asp Gly Leu Tyr Gln Gly Leu Ser Thr Ala Thr Lys
435 440 445

Asp Thr Tyr Asp Ala Leu His Met Gln Ala Leu Pro Pro Arg
450 455 460

<210> 48
<211> 507
<212> PRT
<213> Artificial Sequence

<220>
<223> Description of Artificial Sequence: artificially synthesized sequence

<400> 48

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

```
Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Ser Gly Asn Thr Ser Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
```

340 345 350

Lys Arg Gly Arg Lys Lys Leu Leu Tyr Ile Phe Lys Gln Pro Phe Met
355 360 365

Arg Pro Val Gln Thr Thr Gln Glu Glu Asp Gly Cys Ser Cys Arg Phe
370 375 380

Pro Glu Glu Glu Glu Gly Gly Cys Glu Leu Arg Val Lys Phe Ser Arg
385 390 395 400

Ser Ala Asp Ala Pro Ala Tyr Gln Gln Gly Gln Asn Gln Leu Tyr Asn
405 410 415

Glu Leu Asn Leu Gly Arg Arg Glu Glu Tyr Asp Val Leu Asp Lys Arg
420 425 430

Arg Gly Arg Asp Pro Glu Met Gly Gly Lys Pro Gln Arg Arg Lys Asn
435 440 445

Pro Gln Glu Gly Leu Tyr Asn Glu Leu Gln Lys Asp Lys Met Ala Glu
450 455 460

Ala Tyr Ser Glu Ile Gly Met Lys Gly Glu Arg Arg Arg Gly Lys Gly
465 470 475 480

His Asp Gly Leu Tyr Gln Gly Leu Ser Thr Ala Thr Lys Asp Thr Tyr
485 490 495

Asp Ala Leu His Met Gln Ala Leu Pro Pro Arg
500 505

<210> 49
<211> 465
<212> PRT
<213> Artificial Sequence

<220>
<223> Description of Artificial Sequence: artificially synthesized sequence

<400> 49

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Glu Val Asn
20 25 30

Ile Gly Trp Leu Gln Gln Lys Pro Gly Lys Ala Phe Lys Gly Leu Ile

```
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Arg Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285
```

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Arg Val Lys Phe Ser Arg Ser Ala Asp Ala Pro Ala Tyr Gln Gln Gly
        355                 360                 365

Gln Asn Gln Leu Tyr Asn Glu Leu Asn Leu Gly Arg Arg Glu Glu Tyr
    370                 375                 380

Asp Val Leu Asp Lys Arg Arg Gly Arg Asp Pro Glu Met Gly Gly Lys
385                 390                 395                 400

Pro Gln Arg Arg Lys Asn Pro Gln Glu Gly Leu Tyr Asn Glu Leu Gln
                405                 410                 415

Lys Asp Lys Met Ala Glu Ala Tyr Ser Glu Ile Gly Met Lys Gly Glu
            420                 425                 430

Arg Arg Arg Gly Lys Gly His Asp Gly Leu Tyr Gln Gly Leu Ser Thr
        435                 440                 445

Ala Thr Lys Asp Thr Tyr Asp Ala Leu His Met Gln Ala Leu Pro Pro
    450                 455                 460

Arg
465

<210> 50
<211> 462
<212> PRT
<213> Artificial Sequence

<220>
<223> Description of Artificial Sequence: artificially synthesized sequence

<400> 50

Asp Ile Gln Met Thr Gln Ser Pro Ser Ser Leu Ser Ala Ser Val Gly
1               5                   10                  15

```
Asp Arg Val Thr Ile Thr Cys His Ala Ser Gln Asp Ile Glu Val Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Arg Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270
```

Ala Val His Thr Arg Gly Leu Asp Phe Ala Cys Asp Ile Tyr Ile Trp
275 280 285

Ala Pro Leu Ala Gly Thr Cys Gly Val Leu Leu Leu Ser Leu Val Ile
290 295 300

Thr Leu Tyr Cys Lys Arg Gly Arg Lys Lys Leu Leu Tyr Ile Phe Lys
305 310 315 320

Gln Pro Phe Met Arg Pro Val Gln Thr Thr Gln Glu Glu Asp Gly Cys
325 330 335

Ser Cys Arg Phe Pro Glu Glu Glu Glu Gly Gly Cys Glu Leu Arg Val
340 345 350

Lys Phe Ser Arg Ser Ala Asp Ala Pro Ala Tyr Lys Gln Gly Gln Asn
355 360 365

Gln Leu Tyr Asn Glu Leu Asn Leu Gly Arg Arg Glu Glu Tyr Asp Val
370 375 380

Leu Asp Lys Arg Arg Gly Arg Asp Pro Glu Met Gly Gly Lys Pro Arg
385 390 395 400

Arg Lys Asn Pro Gln Glu Gly Leu Tyr Asn Glu Leu Gln Lys Asp Lys
405 410 415

Met Ala Glu Ala Tyr Ser Glu Ile Gly Met Lys Gly Glu Arg Arg Arg
420 425 430

Gly Lys Gly His Asp Gly Leu Tyr Gln Gly Leu Ser Thr Ala Thr Lys
435 440 445

Asp Thr Tyr Asp Ala Leu His Met Gln Ala Leu Pro Pro Arg
450 455 460

<210> 51
<211> 507
<212> PRT
<213> Artificial Sequence

<220>
<223> Description of Artificial Sequence: artificially synthesized sequence

<400> 51

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Glu Val Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Leu
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Arg Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Phe Trp Val Leu
275 280 285

Val Val Val Gly Gly Val Leu Ala Cys Tyr Ser Leu Leu Val Thr Val
290 295 300

Ala Phe Ile Ile Phe Trp Val Arg Ser Lys Arg Ser Arg Leu Leu His
305 310 315 320

Ser Asp Tyr Met Asn Met Thr Pro Arg Arg Pro Gly Pro Thr Arg Lys
325 330 335

His Tyr Gln Pro Tyr Ala Pro Pro Arg Asp Phe Ala Ala Tyr Arg Ser
340 345 350

Lys Arg Gly Arg Lys Lys Leu Leu Tyr Ile Phe Lys Gln Pro Phe Met
355 360 365

Arg Pro Val Gln Thr Thr Gln Glu Glu Asp Gly Cys Ser Cys Arg Phe
370 375 380

Pro Glu Glu Glu Glu Gly Gly Cys Glu Leu Arg Val Lys Phe Ser Arg
385 390 395 400

Ser Ala Asp Ala Pro Ala Tyr Gln Gln Gly Gln Asn Gln Leu Tyr Asn
405 410 415

Glu Leu Asn Leu Gly Arg Arg Glu Glu Tyr Asp Val Leu Asp Lys Arg
420 425 430

Arg Gly Arg Asp Pro Glu Met Gly Gly Lys Pro Gln Arg Arg Lys Asn
435 440 445

Pro Gln Glu Gly Leu Tyr Asn Glu Leu Gln Lys Asp Lys Met Ala Glu
450 455 460

Ala Tyr Ser Glu Ile Gly Met Lys Gly Glu Arg Arg Arg Gly Lys Gly
465 470 475 480

His Asp Gly Leu Tyr Gln Gly Leu Ser Thr Ala Thr Lys Asp Thr Tyr
485 490 495

Asp Ala Leu His Met Gln Ala Leu Pro Pro Arg
500 505

<210> 52

<211> 20
<212> DNA
<213> Artificial Sequence

<220>
<223> Description of Artificial Sequence: artificially synthesized sequence

<400> 52
gacgttgggt taccttctgc 20

<210> 53
<211> 20
<212> DNA
<213> Artificial Sequence

<220>
<223> Description of Artificial Sequence: artificially synthesized sequence

<400> 53
ttcccaggtc acgatgtagg 20

<210> 54
<211> 20
<212> DNA
<213> Artificial Sequence

<220>
<223> Description of Artificial Sequence: artificially synthesized sequence

<400> 54
atggccgcga gacggcacct 20

<210> 55
<211> 465
<212> PRT
<213> Artificial Sequence

<220>
<223> Description of Artificial Sequence: artificially synthesized sequence

<400> 55

```
Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Ser Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60
```

```
Ser Gly Ser Gly Thr Asp Phe Thr Leu Thr Ile Ser Ser Leu Gln Pro
65                  70                  75                  80

Glu Asp Phe Ala Thr Tyr Tyr Cys Val Gln Tyr Ala Gln Phe Pro Tyr
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Ser Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
```

305 310 315 320

Ser Asp Tyr Met Asn Met Thr Pro Arg Arg Pro Gly Pro Thr Arg Lys
325 330 335

His Tyr Gln Pro Tyr Ala Pro Pro Arg Asp Phe Ala Ala Tyr Arg Ser
340 345 350

Arg Val Lys Phe Ser Arg Ser Ala Asp Ala Pro Ala Tyr Gln Gln Gly
355 360 365

Gln Asn Gln Leu Tyr Asn Glu Leu Asn Leu Gly Arg Arg Glu Glu Tyr
370 375 380

Asp Val Leu Asp Lys Arg Arg Gly Arg Asp Pro Glu Met Gly Gly Lys
385 390 395 400

Pro Gln Arg Arg Lys Asn Pro Gln Glu Gly Leu Tyr Asn Glu Leu Gln
405 410 415

Lys Asp Lys Met Ala Glu Ala Tyr Ser Glu Ile Gly Met Lys Gly Glu
420 425 430

Arg Arg Arg Gly Lys Gly His Asp Gly Leu Tyr Gln Gly Leu Ser Thr
435 440 445

Ala Thr Lys Asp Thr Tyr Asp Ala Leu His Met Gln Ala Leu Pro Pro
450 455 460

Arg
465

<210> 56
<211> 462
<212> PRT
<213> Artificial Sequence

<220>
<223> Description of Artificial Sequence: artificially synthesized sequence

<400> 56

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Ser Asn
20 25 30

Ile Gly Trp Leu Gln Gln Lys Pro Gly Lys Ala Phe Lys Gly Leu Ile

```
                35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Val Gln Tyr Ala Gln Phe Pro Tyr
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Ser Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Ile Tyr Ile Trp
        275                 280                 285
```

Ala Pro Leu Ala Gly Thr Cys Gly Val Leu Leu Leu Ser Leu Val Ile
    290                 295                 300

Thr Leu Tyr Cys Lys Arg Gly Arg Lys Lys Leu Leu Tyr Ile Phe Lys
305                 310                 315                 320

Gln Pro Phe Met Arg Pro Val Gln Thr Thr Gln Glu Glu Asp Gly Cys
                325                 330                 335

Ser Cys Arg Phe Pro Glu Glu Glu Glu Gly Gly Cys Glu Leu Arg Val
            340                 345                 350

Lys Phe Ser Arg Ser Ala Asp Ala Pro Ala Tyr Lys Gln Gly Gln Asn
        355                 360                 365

Gln Leu Tyr Asn Glu Leu Asn Leu Gly Arg Arg Glu Glu Tyr Asp Val
    370                 375                 380

Leu Asp Lys Arg Arg Gly Arg Asp Pro Glu Met Gly Gly Lys Pro Arg
385                 390                 395                 400

Arg Lys Asn Pro Gln Glu Gly Leu Tyr Asn Glu Leu Gln Lys Asp Lys
                405                 410                 415

Met Ala Glu Ala Tyr Ser Glu Ile Gly Met Lys Gly Glu Arg Arg Arg
            420                 425                 430

Gly Lys Gly His Asp Gly Leu Tyr Gln Gly Leu Ser Thr Ala Thr Lys
        435                 440                 445

Asp Thr Tyr Asp Ala Leu His Met Gln Ala Leu Pro Pro Arg
    450                 455                 460

<210> 57
<211> 507
<212> PRT
<213> Artificial Sequence

<220>
<223> Description of Artificial Sequence: artificially synthesized sequence

<400> 57

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Ser Asn
            20                  25                  30

```
Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Val Gln Tyr Ala Gln Phe Pro Tyr
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Ser Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285
```

Val Val Val Gly Gly Val Leu Ala Cys Tyr Ser Leu Leu Val Thr Val
290 295 300

Ala Phe Ile Ile Phe Trp Val Arg Ser Lys Arg Ser Arg Leu Leu His
305 310 315 320

Ser Asp Tyr Met Asn Met Thr Pro Arg Arg Pro Gly Pro Thr Arg Lys
325 330 335

His Tyr Gln Pro Tyr Ala Pro Pro Arg Asp Phe Ala Ala Tyr Arg Ser
340 345 350

Lys Arg Gly Arg Lys Lys Leu Leu Tyr Ile Phe Lys Gln Pro Phe Met
355 360 365

Arg Pro Val Gln Thr Thr Gln Glu Glu Asp Gly Cys Ser Cys Arg Phe
370 375 380

Pro Glu Glu Glu Glu Gly Gly Cys Glu Leu Arg Val Lys Phe Ser Arg
385 390 395 400

Ser Ala Asp Ala Pro Ala Tyr Gln Gln Gly Gln Asn Gln Leu Tyr Asn
405 410 415

Glu Leu Asn Leu Gly Arg Arg Glu Glu Tyr Asp Val Leu Asp Lys Arg
420 425 430

Arg Gly Arg Asp Pro Glu Met Gly Gly Lys Pro Gln Arg Arg Lys Asn
435 440 445

Pro Gln Glu Gly Leu Tyr Asn Glu Leu Gln Lys Asp Lys Met Ala Glu
450 455 460

Ala Tyr Ser Glu Ile Gly Met Lys Gly Glu Arg Arg Arg Gly Lys Gly
465 470 475 480

His Asp Gly Leu Tyr Gln Gly Leu Ser Thr Ala Thr Lys Asp Thr Tyr
485 490 495

Asp Ala Leu His Met Gln Ala Leu Pro Pro Arg
500 505

<210> 58
<211> 465
<212> PRT
<213> Artificial Sequence

<220>

<223> Description of Artificial Sequence: artificially synthesized sequence

<400> 58

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Ser Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Asn Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Phe Trp Val Leu
275 280 285

Val Val Val Gly Gly Val Leu Ala Cys Tyr Ser Leu Leu Val Thr Val
290 295 300

Ala Phe Ile Ile Phe Trp Val Arg Ser Lys Arg Ser Arg Leu Leu His
305 310 315 320

Ser Asp Tyr Met Asn Met Thr Pro Arg Arg Pro Gly Pro Thr Arg Lys
325 330 335

His Tyr Gln Pro Tyr Ala Pro Pro Arg Asp Phe Ala Ala Tyr Arg Ser
340 345 350

Arg Val Lys Phe Ser Arg Ser Ala Asp Ala Pro Ala Tyr Gln Gln Gly
355 360 365

Gln Asn Gln Leu Tyr Asn Glu Leu Asn Leu Gly Arg Arg Glu Glu Tyr
370 375 380

Asp Val Leu Asp Lys Arg Arg Gly Arg Asp Pro Glu Met Gly Gly Lys
385 390 395 400

Pro Gln Arg Arg Lys Asn Pro Gln Glu Gly Leu Tyr Asn Glu Leu Gln
405 410 415

Lys Asp Lys Met Ala Glu Ala Tyr Ser Glu Ile Gly Met Lys Gly Glu
420 425 430

Arg Arg Arg Gly Lys Gly His Asp Gly Leu Tyr Gln Gly Leu Ser Thr
435 440 445

Ala Thr Lys Asp Thr Tyr Asp Ala Leu His Met Gln Ala Leu Pro Pro
450 455 460

Arg
465

<210> 59

<211> 462
<212> PRT
<213> Artificial Sequence

<220>
<223> Description of Artificial Sequence: artificially synthesized sequence

<400> 59

```
Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Ser Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Ile
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Asn Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
```

```
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Ile Tyr Ile Trp
        275                 280                 285

Ala Pro Leu Ala Gly Thr Cys Gly Val Leu Leu Leu Ser Leu Val Ile
    290                 295                 300

Thr Leu Tyr Cys Lys Arg Gly Arg Lys Lys Leu Leu Tyr Ile Phe Lys
305                 310                 315                 320

Gln Pro Phe Met Arg Pro Val Gln Thr Thr Gln Glu Glu Asp Gly Cys
                325                 330                 335

Ser Cys Arg Phe Pro Glu Glu Glu Glu Gly Gly Cys Glu Leu Arg Val
            340                 345                 350

Lys Phe Ser Arg Ser Ala Asp Ala Pro Ala Tyr Lys Gln Gly Gln Asn
        355                 360                 365

Gln Leu Tyr Asn Glu Leu Asn Leu Gly Arg Arg Glu Glu Tyr Asp Val
    370                 375                 380

Leu Asp Lys Arg Arg Gly Arg Asp Pro Glu Met Gly Gly Lys Pro Arg
385                 390                 395                 400

Arg Lys Asn Pro Gln Glu Gly Leu Tyr Asn Glu Leu Gln Lys Asp Lys
                405                 410                 415

Met Ala Glu Ala Tyr Ser Glu Ile Gly Met Lys Gly Glu Arg Arg Arg
            420                 425                 430

Gly Lys Gly His Asp Gly Leu Tyr Gln Gly Leu Ser Thr Ala Thr Lys
        435                 440                 445

Asp Thr Tyr Asp Ala Leu His Met Gln Ala Leu Pro Pro Arg
    450                 455                 460
```

<210> 60
<211> 507
<212> PRT
<213> Artificial Sequence

<220>
<223> Description of Artificial Sequence: artificially synthesized sequence

<400> 60

```
Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Ser Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Ile
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Asn Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205
```

```
Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Lys Arg Gly Arg Lys Lys Leu Leu Tyr Ile Phe Lys Gln Pro Phe Met
        355                 360                 365

Arg Pro Val Gln Thr Thr Gln Glu Glu Asp Gly Cys Ser Cys Arg Phe
    370                 375                 380

Pro Glu Glu Glu Glu Gly Gly Cys Glu Leu Arg Val Lys Phe Ser Arg
385                 390                 395                 400

Ser Ala Asp Ala Pro Ala Tyr Gln Gln Gly Gln Asn Gln Leu Tyr Asn
                405                 410                 415

Glu Leu Asn Leu Gly Arg Arg Glu Glu Tyr Asp Val Leu Asp Lys Arg
            420                 425                 430

Arg Gly Arg Asp Pro Glu Met Gly Gly Lys Pro Gln Arg Arg Lys Asn
        435                 440                 445

Pro Gln Glu Gly Leu Tyr Asn Glu Leu Gln Lys Asp Lys Met Ala Glu
    450                 455                 460
```

```
Ala Tyr Ser Glu Ile Gly Met Lys Gly Glu Arg Arg Arg Gly Lys Gly
465                 470                 475                 480

His Asp Gly Leu Tyr Gln Gly Leu Ser Thr Ala Thr Lys Asp Thr Tyr
                485                 490                 495

Asp Ala Leu His Met Gln Ala Leu Pro Pro Arg
            500                 505


<210>  61
<211>  465
<212>  PRT
<213>  Artificial Sequence

<220>
<223>  Description of Artificial Sequence: artificially synthesized
       sequence

<400>  61

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Ile
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160
```

```
Ile Arg Gln Ala Pro Gly Lys Gly Leu Glu Trp Leu Gly Tyr Ile Ser
                165                 170                 175

Tyr Arg Gly Arg Thr Ser Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Arg Val Lys Phe Ser Arg Ser Ala Asp Ala Pro Ala Tyr Gln Gln Gly
        355                 360                 365

Gln Asn Gln Leu Tyr Asn Glu Leu Asn Leu Gly Arg Arg Glu Glu Tyr
    370                 375                 380

Asp Val Leu Asp Lys Arg Arg Gly Arg Asp Pro Glu Met Gly Gly Lys
385                 390                 395                 400

Pro Gln Arg Arg Lys Asn Pro Gln Glu Gly Leu Tyr Asn Glu Leu Gln
                405                 410                 415
```

Lys Asp Lys Met Ala Glu Ala Tyr Ser Glu Ile Gly Met Lys Gly Glu
420 425 430

Arg Arg Arg Gly Lys Gly His Asp Gly Leu Tyr Gln Gly Leu Ser Thr
435 440 445

Ala Thr Lys Asp Thr Tyr Asp Ala Leu His Met Gln Ala Leu Pro Pro
450 455 460

Arg
465

<210> 62
<211> 462
<212> PRT
<213> Artificial Sequence

<220>
<223> Description of Artificial Sequence: artificially synthesized sequence

<400> 62

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Ser Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Ile Tyr Ile Trp
275 280 285

Ala Pro Leu Ala Gly Thr Cys Gly Val Leu Leu Leu Ser Leu Val Ile
290 295 300

Thr Leu Tyr Cys Lys Arg Gly Arg Lys Lys Leu Leu Tyr Ile Phe Lys
305 310 315 320

Gln Pro Phe Met Arg Pro Val Gln Thr Thr Gln Glu Glu Asp Gly Cys
325 330 335

Ser Cys Arg Phe Pro Glu Glu Glu Glu Gly Gly Cys Glu Leu Arg Val
340 345 350

Lys Phe Ser Arg Ser Ala Asp Ala Pro Ala Tyr Lys Gln Gly Gln Asn
355 360 365

Gln Leu Tyr Asn Glu Leu Asn Leu Gly Arg Arg Glu Glu Tyr Asp Val
370 375 380

Leu Asp Lys Arg Arg Gly Arg Asp Pro Glu Met Gly Gly Lys Pro Arg

385 390 395 400

Arg Lys Asn Pro Gln Glu Gly Leu Tyr Asn Glu Leu Gln Lys Asp Lys
405 410 415

Met Ala Glu Ala Tyr Ser Glu Ile Gly Met Lys Gly Glu Arg Arg Arg
420 425 430

Gly Lys Gly His Asp Gly Leu Tyr Gln Gly Leu Ser Thr Ala Thr Lys
435 440 445

Asp Thr Tyr Asp Ala Leu His Met Gln Ala Leu Pro Pro Arg
450 455 460

<210> 63
<211> 507
<212> PRT
<213> Artificial Sequence

<220>
<223> Description of Artificial Sequence: artificially synthesized sequence

<400> 63

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser

130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Ser Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Phe Trp Val Leu
275 280 285

Val Val Val Gly Gly Val Leu Ala Cys Tyr Ser Leu Leu Val Thr Val
290 295 300

Ala Phe Ile Ile Phe Trp Val Arg Ser Lys Arg Ser Arg Leu Leu His
305 310 315 320

Ser Asp Tyr Met Asn Met Thr Pro Arg Arg Pro Gly Pro Thr Arg Lys
325 330 335

His Tyr Gln Pro Tyr Ala Pro Pro Arg Asp Phe Ala Ala Tyr Arg Ser
340 345 350

Lys Arg Gly Arg Lys Lys Leu Leu Tyr Ile Phe Lys Gln Pro Phe Met
355 360 365

Arg Pro Val Gln Thr Thr Gln Glu Glu Asp Gly Cys Ser Cys Arg Phe
370 375 380

Pro Glu Glu Glu Glu Gly Gly Cys Glu Leu Arg Val Lys Phe Ser Arg
385 390 395 400

Ser Ala Asp Ala Pro Ala Tyr Gln Gln Gly Gln Asn Gln Leu Tyr Asn
405 410 415

Glu Leu Asn Leu Gly Arg Arg Glu Glu Tyr Asp Val Leu Asp Lys Arg
420 425 430

Arg Gly Arg Asp Pro Glu Met Gly Gly Lys Pro Gln Arg Arg Lys Asn
435 440 445

Pro Gln Glu Gly Leu Tyr Asn Glu Leu Gln Lys Asp Lys Met Ala Glu
450 455 460

Ala Tyr Ser Glu Ile Gly Met Lys Gly Glu Arg Arg Arg Gly Lys Gly
465 470 475 480

His Asp Gly Leu Tyr Gln Gly Leu Ser Thr Ala Thr Lys Asp Thr Tyr
485 490 495

Asp Ala Leu His Met Gln Ala Leu Pro Pro Arg
500 505

<210> 64
<211> 465
<212> PRT
<213> Artificial Sequence

<220>
<223> Description of Artificial Sequence: artificially synthesized sequence

<400> 64

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

```
Glu Asp Phe Ala Thr Tyr Tyr Cys Asn Gln Tyr Glu Asn Ile Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Arg Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335
```

```
His Tyr Gln Pro Tyr Ala Pro Pro Arg Asp Phe Ala Ala Tyr Arg Ser
            340                 345                 350

Arg Val Lys Phe Ser Arg Ser Ala Asp Ala Pro Ala Tyr Gln Gln Gly
        355                 360                 365

Gln Asn Gln Leu Tyr Asn Glu Leu Asn Leu Gly Arg Arg Glu Glu Tyr
    370                 375                 380

Asp Val Leu Asp Lys Arg Arg Gly Arg Asp Pro Glu Met Gly Gly Lys
385                 390                 395                 400

Pro Gln Arg Arg Lys Asn Pro Gln Glu Gly Leu Tyr Asn Glu Leu Gln
                405                 410                 415

Lys Asp Lys Met Ala Glu Ala Tyr Ser Glu Ile Gly Met Lys Gly Glu
            420                 425                 430

Arg Arg Arg Gly Lys Gly His Asp Gly Leu Tyr Gln Gly Leu Ser Thr
        435                 440                 445

Ala Thr Lys Asp Thr Tyr Asp Ala Leu His Met Gln Ala Leu Pro Pro
    450                 455                 460

Arg
465


<210>  65
<211>  462
<212>  PRT
<213>  Artificial Sequence

<220>
<223>  Description of Artificial Sequence: artificially synthesized
       sequence

<400>  65

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60
```

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Ile Pro Leu
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Arg Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Ile Tyr Ile Trp
275 280 285

Ala Pro Leu Ala Gly Thr Cys Gly Val Leu Leu Leu Ser Leu Val Ile
290 295 300

Thr Leu Tyr Cys Lys Arg Gly Arg Lys Lys Leu Leu Tyr Ile Phe Lys
305 310 315 320

Gln Pro Phe Met Arg Pro Val Gln Thr Thr Gln Glu Glu Asp Gly Cys
325 330 335

Ser Cys Arg Phe Pro Glu Glu Glu Glu Gly Gly Cys Glu Leu Arg Val
340 345 350

Lys Phe Ser Arg Ser Ala Asp Ala Pro Ala Tyr Lys Gln Gly Gln Asn
355 360 365

Gln Leu Tyr Asn Glu Leu Asn Leu Gly Arg Arg Glu Glu Tyr Asp Val
370 375 380

Leu Asp Lys Arg Arg Gly Arg Asp Pro Glu Met Gly Gly Lys Pro Arg
385 390 395 400

Arg Lys Asn Pro Gln Glu Gly Leu Tyr Asn Glu Leu Gln Lys Asp Lys
405 410 415

Met Ala Glu Ala Tyr Ser Glu Ile Gly Met Lys Gly Glu Arg Arg Arg
420 425 430

Gly Lys Gly His Asp Gly Leu Tyr Gln Gly Leu Ser Thr Ala Thr Lys
435 440 445

Asp Thr Tyr Asp Ala Leu His Met Gln Ala Leu Pro Pro Arg
450 455 460

<210> 66
<211> 507
<212> PRT
<213> Artificial Sequence

<220>
<223> Description of Artificial Sequence: artificially synthesized sequence

<400> 66

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Ile Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Arg Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His

305 310 315 320

Ser Asp Tyr Met Asn Met Thr Pro Arg Arg Pro Gly Pro Thr Arg Lys
325 330 335

His Tyr Gln Pro Tyr Ala Pro Pro Arg Asp Phe Ala Ala Tyr Arg Ser
340 345 350

Lys Arg Gly Arg Lys Lys Leu Leu Tyr Ile Phe Lys Gln Pro Phe Met
355 360 365

Arg Pro Val Gln Thr Thr Gln Glu Glu Asp Gly Cys Ser Cys Arg Phe
370 375 380

Pro Glu Glu Glu Glu Gly Gly Cys Glu Leu Arg Val Lys Phe Ser Arg
385 390 395 400

Ser Ala Asp Ala Pro Ala Tyr Gln Gln Gly Gln Asn Gln Leu Tyr Asn
405 410 415

Glu Leu Asn Leu Gly Arg Arg Glu Glu Tyr Asp Val Leu Asp Lys Arg
420 425 430

Arg Gly Arg Asp Pro Glu Met Gly Gly Lys Pro Gln Arg Arg Lys Asn
435 440 445

Pro Gln Glu Gly Leu Tyr Asn Glu Leu Gln Lys Asp Lys Met Ala Glu
450 455 460

Ala Tyr Ser Glu Ile Gly Met Lys Gly Glu Arg Arg Arg Gly Lys Gly
465 470 475 480

His Asp Gly Leu Tyr Gln Gly Leu Ser Thr Ala Thr Lys Asp Thr Tyr
485 490 495

Asp Ala Leu His Met Gln Ala Leu Pro Pro Arg
500 505

<210> 67
<211> 465
<212> PRT
<213> Artificial Sequence

<220>
<223> Description of Artificial Sequence: artificially synthesized sequence

<400> 67

Asp Ile Gln Met Thr Gln Ser Pro Ser Ser Leu Ser Ala Ser Val Gly

1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Ile Pro Leu
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Gln Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

```
Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Arg Val Lys Phe Ser Arg Ser Ala Asp Ala Pro Ala Tyr Gln Gln Gly
        355                 360                 365

Gln Asn Gln Leu Tyr Asn Glu Leu Asn Leu Gly Arg Arg Glu Glu Tyr
    370                 375                 380

Asp Val Leu Asp Lys Arg Arg Gly Arg Asp Pro Glu Met Gly Gly Lys
385                 390                 395                 400

Pro Gln Arg Arg Lys Asn Pro Gln Glu Gly Leu Tyr Asn Glu Leu Gln
                405                 410                 415

Lys Asp Lys Met Ala Glu Ala Tyr Ser Glu Ile Gly Met Lys Gly Glu
            420                 425                 430

Arg Arg Arg Gly Lys Gly His Asp Gly Leu Tyr Gln Gly Leu Ser Thr
        435                 440                 445

Ala Thr Lys Asp Thr Tyr Asp Ala Leu His Met Gln Ala Leu Pro Pro
    450                 455                 460

Arg
465
```

<210>  68
<211>  462
<212>  PRT
<213>  Artificial Sequence

<220>
<223>  Description of Artificial Sequence: artificially synthesized

sequence

<400> 68

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Ile Pro Leu
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Gln Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Ile Tyr Ile Trp
275 280 285

Ala Pro Leu Ala Gly Thr Cys Gly Val Leu Leu Leu Ser Leu Val Ile
290 295 300

Thr Leu Tyr Cys Lys Arg Gly Arg Lys Lys Leu Leu Tyr Ile Phe Lys
305 310 315 320

Gln Pro Phe Met Arg Pro Val Gln Thr Thr Gln Glu Glu Asp Gly Cys
325 330 335

Ser Cys Arg Phe Pro Glu Glu Glu Glu Gly Gly Cys Glu Leu Arg Val
340 345 350

Lys Phe Ser Arg Ser Ala Asp Ala Pro Ala Tyr Lys Gln Gly Gln Asn
355 360 365

Gln Leu Tyr Asn Glu Leu Asn Leu Gly Arg Arg Glu Glu Tyr Asp Val
370 375 380

Leu Asp Lys Arg Arg Gly Arg Asp Pro Glu Met Gly Gly Lys Pro Arg
385 390 395 400

Arg Lys Asn Pro Gln Glu Gly Leu Tyr Asn Glu Leu Gln Lys Asp Lys
405 410 415

Met Ala Glu Ala Tyr Ser Glu Ile Gly Met Lys Gly Glu Arg Arg Arg
420 425 430

Gly Lys Gly His Asp Gly Leu Tyr Gln Gly Leu Ser Thr Ala Thr Lys
435 440 445

Asp Thr Tyr Asp Ala Leu His Met Gln Ala Leu Pro Pro Arg
450 455 460

<210> 69
<211> 507
<212> PRT
<213> Artificial Sequence

<220>

<223> Description of Artificial Sequence: artificially synthesized sequence

<400> 69

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Ile Pro Leu
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Gln Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Phe Trp Val Leu
275 280 285

Val Val Val Gly Gly Val Leu Ala Cys Tyr Ser Leu Leu Val Thr Val
290 295 300

Ala Phe Ile Ile Phe Trp Val Arg Ser Lys Arg Ser Arg Leu Leu His
305 310 315 320

Ser Asp Tyr Met Asn Met Thr Pro Arg Arg Pro Gly Pro Thr Arg Lys
325 330 335

His Tyr Gln Pro Tyr Ala Pro Pro Arg Asp Phe Ala Ala Tyr Arg Ser
340 345 350

Lys Arg Gly Arg Lys Lys Leu Leu Tyr Ile Phe Lys Gln Pro Phe Met
355 360 365

Arg Pro Val Gln Thr Thr Gln Glu Glu Asp Gly Cys Ser Cys Arg Phe
370 375 380

Pro Glu Glu Glu Glu Gly Gly Cys Glu Leu Arg Val Lys Phe Ser Arg
385 390 395 400

Ser Ala Asp Ala Pro Ala Tyr Gln Gln Gly Gln Asn Gln Leu Tyr Asn
405 410 415

Glu Leu Asn Leu Gly Arg Arg Glu Glu Tyr Asp Val Leu Asp Lys Arg
420 425 430

Arg Gly Arg Asp Pro Glu Met Gly Gly Lys Pro Gln Arg Arg Lys Asn
435 440 445

Pro Gln Glu Gly Leu Tyr Asn Glu Leu Gln Lys Asp Lys Met Ala Glu
450 455 460

Ala Tyr Ser Glu Ile Gly Met Lys Gly Glu Arg Arg Arg Gly Lys Gly
465 470 475 480

His Asp Gly Leu Tyr Gln Gly Leu Ser Thr Ala Thr Lys Asp Thr Tyr

485 490 495

Asp Ala Leu His Met Gln Ala Leu Pro Pro Arg
500 505

<210> 70
<211> 465
<212> PRT
<213> Artificial Sequence

<220>
<223> Description of Artificial Sequence: artificially synthesized sequence

<400> 70

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Thr Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Ser Tyr Asn Pro Ser Leu Lys Ser Arg Ile Ser

```
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Arg Val Lys Phe Ser Arg Ser Ala Asp Ala Pro Ala Tyr Gln Gln Gly
        355                 360                 365

Gln Asn Gln Leu Tyr Asn Glu Leu Asn Leu Gly Arg Arg Glu Glu Tyr
    370                 375                 380

Asp Val Leu Asp Lys Arg Arg Gly Arg Asp Pro Glu Met Gly Gly Lys
385                 390                 395                 400

Pro Gln Arg Arg Lys Asn Pro Gln Glu Gly Leu Tyr Asn Glu Leu Gln
                405                 410                 415

Lys Asp Lys Met Ala Glu Ala Tyr Ser Glu Ile Gly Met Lys Gly Glu
            420                 425                 430
```

Arg Arg Arg Gly Lys Gly His Asp Gly Leu Tyr Gln Gly Leu Ser Thr
435 440 445

Ala Thr Lys Asp Thr Tyr Asp Ala Leu His Met Gln Ala Leu Pro Pro
450 455 460

Arg
465

<210> 71
<211> 462
<212> PRT
<213> Artificial Sequence

<220>
<223> Description of Artificial Sequence: artificially synthesized sequence

<400> 71

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Thr Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Ser Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Ile Tyr Ile Trp
275 280 285

Ala Pro Leu Ala Gly Thr Cys Gly Val Leu Leu Leu Ser Leu Val Ile
290 295 300

Thr Leu Tyr Cys Lys Arg Gly Arg Lys Lys Leu Leu Tyr Ile Phe Lys
305 310 315 320

Gln Pro Phe Met Arg Pro Val Gln Thr Thr Gln Glu Glu Asp Gly Cys
325 330 335

Ser Cys Arg Phe Pro Glu Glu Glu Glu Gly Gly Cys Glu Leu Arg Val
340 345 350

Lys Phe Ser Arg Ser Ala Asp Ala Pro Ala Tyr Lys Gln Gly Gln Asn
355 360 365

Gln Leu Tyr Asn Glu Leu Asn Leu Gly Arg Arg Glu Glu Tyr Asp Val
370 375 380

Leu Asp Lys Arg Arg Gly Arg Asp Pro Glu Met Gly Gly Lys Pro Arg
385 390 395 400

Arg Lys Asn Pro Gln Glu Gly Leu Tyr Asn Glu Leu Gln Lys Asp Lys
405 410 415

Met Ala Glu Ala Tyr Ser Glu Ile Gly Met Lys Gly Glu Arg Arg Arg
420 425 430

Gly Lys Gly His Asp Gly Leu Tyr Gln Gly Leu Ser Thr Ala Thr Lys
435 440 445

Asp Thr Tyr Asp Ala Leu His Met Gln Ala Leu Pro Pro Arg
450 455 460

<210> 72
<211> 507
<212> PRT
<213> Artificial Sequence

<220>
<223> Description of Artificial Sequence: artificially synthesized sequence

<400> 72

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Thr Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Ser Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Phe Trp Val Leu
275 280 285

Val Val Val Gly Gly Val Leu Ala Cys Tyr Ser Leu Leu Val Thr Val
290 295 300

Ala Phe Ile Ile Phe Trp Val Arg Ser Lys Arg Ser Arg Leu Leu His
305 310 315 320

Ser Asp Tyr Met Asn Met Thr Pro Arg Arg Pro Gly Pro Thr Arg Lys
325 330 335

His Tyr Gln Pro Tyr Ala Pro Pro Arg Asp Phe Ala Ala Tyr Arg Ser
340 345 350

Lys Arg Gly Arg Lys Lys Leu Leu Tyr Ile Phe Lys Gln Pro Phe Met
355 360 365

Arg Pro Val Gln Thr Thr Gln Glu Glu Asp Gly Cys Ser Cys Arg Phe
370 375 380

Pro Glu Glu Glu Glu Gly Gly Cys Glu Leu Arg Val Lys Phe Ser Arg
385 390 395 400

Ser Ala Asp Ala Pro Ala Tyr Gln Gln Gly Gln Asn Gln Leu Tyr Asn
405 410 415

```
Glu Leu Asn Leu Gly Arg Arg Glu Glu Tyr Asp Val Leu Asp Lys Arg
            420                 425                 430

Arg Gly Arg Asp Pro Glu Met Gly Gly Lys Pro Gln Arg Arg Lys Asn
        435                 440                 445

Pro Gln Glu Gly Leu Tyr Asn Glu Leu Gln Lys Asp Lys Met Ala Glu
    450                 455                 460

Ala Tyr Ser Glu Ile Gly Met Lys Gly Glu Arg Arg Arg Gly Lys Gly
465                 470                 475                 480

His Asp Gly Leu Tyr Gln Gly Leu Ser Thr Ala Thr Lys Asp Thr Tyr
                485                 490                 495

Asp Ala Leu His Met Gln Ala Leu Pro Pro Arg
            500                 505


<210>  73
<211>  465
<212>  PRT
<213>  Artificial Sequence

<220>
<223>  Description of Artificial Sequence: artificially synthesized
       sequence

<400>  73

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ser Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Ile
                85                  90                  95

Thr Phe Gly Gln Gly Thr Lys Val Glu Ile Lys Arg Gly Gly Gly Gly
            100                 105                 110
```

```
Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Ser Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Arg Val Lys Phe Ser Arg Ser Ala Asp Ala Pro Ala Tyr Gln Gln Gly
```

355 360 365

Gln Asn Gln Leu Tyr Asn Glu Leu Asn Leu Gly Arg Arg Glu Glu Tyr
370 375 380

Asp Val Leu Asp Lys Arg Arg Gly Arg Asp Pro Glu Met Gly Gly Lys
385 390 395 400

Pro Gln Arg Arg Lys Asn Pro Gln Glu Gly Leu Tyr Asn Glu Leu Gln
405 410 415

Lys Asp Lys Met Ala Glu Ala Tyr Ser Glu Ile Gly Met Lys Gly Glu
420 425 430

Arg Arg Arg Gly Lys Gly His Asp Gly Leu Tyr Gln Gly Leu Ser Thr
435 440 445

Ala Thr Lys Asp Thr Tyr Asp Ala Leu His Met Gln Ala Leu Pro Pro
450 455 460

Arg
465

<210> 74
<211> 462
<212> PRT
<213> Artificial Sequence

<220>
<223> Description of Artificial Sequence: artificially synthesized sequence

<400> 74

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ser Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Ile

85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Ser Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Ile Tyr Ile Trp
275 280 285

Ala Pro Leu Ala Gly Thr Cys Gly Val Leu Leu Leu Ser Leu Val Ile
290 295 300

Thr Leu Tyr Cys Lys Arg Gly Arg Lys Lys Leu Leu Tyr Ile Phe Lys
305 310 315 320

Gln Pro Phe Met Arg Pro Val Gln Thr Thr Gln Glu Glu Asp Gly Cys
325 330 335

Ser Cys Arg Phe Pro Glu Glu Glu Glu Gly Gly Cys Glu Leu Arg Val
340 345 350

Lys Phe Ser Arg Ser Ala Asp Ala Pro Ala Tyr Lys Gln Gly Gln Asn
355 360 365

Gln Leu Tyr Asn Glu Leu Asn Leu Gly Arg Arg Glu Glu Tyr Asp Val
370 375 380

Leu Asp Lys Arg Arg Gly Arg Asp Pro Glu Met Gly Gly Lys Pro Arg
385 390 395 400

Arg Lys Asn Pro Gln Glu Gly Leu Tyr Asn Glu Leu Gln Lys Asp Lys
405 410 415

Met Ala Glu Ala Tyr Ser Glu Ile Gly Met Lys Gly Glu Arg Arg Arg
420 425 430

Gly Lys Gly His Asp Gly Leu Tyr Gln Gly Leu Ser Thr Ala Thr Lys
435 440 445

Asp Thr Tyr Asp Ala Leu His Met Gln Ala Leu Pro Pro Arg
450 455 460

<210> 75
<211> 507
<212> PRT
<213> Artificial Sequence

<220>
<223> Description of Artificial Sequence: artificially synthesized sequence

<400> 75

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ser Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

```
Glu Asp Phe Ala Thr Tyr Tyr Cys Asn Gln Tyr Glu Asn Asn Pro Ile
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Ser Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335
```

His Tyr Gln Pro Tyr Ala Pro Pro Arg Asp Phe Ala Ala Tyr Arg Ser
340 345 350

Lys Arg Gly Arg Lys Lys Leu Leu Tyr Ile Phe Lys Gln Pro Phe Met
355 360 365

Arg Pro Val Gln Thr Thr Gln Glu Glu Asp Gly Cys Ser Cys Arg Phe
370 375 380

Pro Glu Glu Glu Glu Gly Gly Cys Glu Leu Arg Val Lys Phe Ser Arg
385 390 395 400

Ser Ala Asp Ala Pro Ala Tyr Gln Gln Gly Gln Asn Gln Leu Tyr Asn
405 410 415

Glu Leu Asn Leu Gly Arg Arg Glu Glu Tyr Asp Val Leu Asp Lys Arg
420 425 430

Arg Gly Arg Asp Pro Glu Met Gly Gly Lys Pro Gln Arg Arg Lys Asn
435 440 445

Pro Gln Glu Gly Leu Tyr Asn Glu Leu Gln Lys Asp Lys Met Ala Glu
450 455 460

Ala Tyr Ser Glu Ile Gly Met Lys Gly Glu Arg Arg Arg Gly Lys Gly
465 470 475 480

His Asp Gly Leu Tyr Gln Gly Leu Ser Thr Ala Thr Lys Asp Thr Tyr
485 490 495

Asp Ala Leu His Met Gln Ala Leu Pro Pro Arg
500 505

<210> 76
<211> 465
<212> PRT
<213> Artificial Sequence

<220>
<223> Description of Artificial Sequence: artificially synthesized sequence

<400> 76

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Thr Asn
20 25 30

```
Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Gln Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285
```

```
Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Arg Val Lys Phe Ser Arg Ser Ala Asp Ala Pro Ala Tyr Gln Gln Gly
        355                 360                 365

Gln Asn Gln Leu Tyr Asn Glu Leu Asn Leu Gly Arg Arg Glu Glu Tyr
    370                 375                 380

Asp Val Leu Asp Lys Arg Arg Gly Arg Asp Pro Glu Met Gly Gly Lys
385                 390                 395                 400

Pro Gln Arg Arg Lys Asn Pro Gln Glu Gly Leu Tyr Asn Glu Leu Gln
                405                 410                 415

Lys Asp Lys Met Ala Glu Ala Tyr Ser Glu Ile Gly Met Lys Gly Glu
            420                 425                 430

Arg Arg Arg Gly Lys Gly His Asp Gly Leu Tyr Gln Gly Leu Ser Thr
        435                 440                 445

Ala Thr Lys Asp Thr Tyr Asp Ala Leu His Met Gln Ala Leu Pro Pro
    450                 455                 460

Arg
465
```

<210> 77
<211> 462
<212> PRT
<213> Artificial Sequence

<220>
<223> Description of Artificial Sequence: artificially synthesized sequence

<400> 77

```
Asp Ile Gln Met Thr Gln Ser Pro Ser Ser Leu Ser Ala Ser Val Gly
1               5                   10                  15
```

```
Asp Arg Val Thr Ile Thr Cys His Ala Ser Gln Asp Ile Asn Thr Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Gln Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
```

260 265 270

Ala Val His Thr Arg Gly Leu Asp Phe Ala Cys Asp Ile Tyr Ile Trp
275 280 285

Ala Pro Leu Ala Gly Thr Cys Gly Val Leu Leu Leu Ser Leu Val Ile
290 295 300

Thr Leu Tyr Cys Lys Arg Gly Arg Lys Lys Leu Leu Tyr Ile Phe Lys
305 310 315 320

Gln Pro Phe Met Arg Pro Val Gln Thr Thr Gln Glu Glu Asp Gly Cys
325 330 335

Ser Cys Arg Phe Pro Glu Glu Glu Glu Gly Gly Cys Glu Leu Arg Val
340 345 350

Lys Phe Ser Arg Ser Ala Asp Ala Pro Ala Tyr Lys Gln Gly Gln Asn
355 360 365

Gln Leu Tyr Asn Glu Leu Asn Leu Gly Arg Arg Glu Glu Tyr Asp Val
370 375 380

Leu Asp Lys Arg Arg Gly Arg Asp Pro Glu Met Gly Gly Lys Pro Arg
385 390 395 400

Arg Lys Asn Pro Gln Glu Gly Leu Tyr Asn Glu Leu Gln Lys Asp Lys
405 410 415

Met Ala Glu Ala Tyr Ser Glu Ile Gly Met Lys Gly Glu Arg Arg Arg
420 425 430

Gly Lys Gly His Asp Gly Leu Tyr Gln Gly Leu Ser Thr Ala Thr Lys
435 440 445

Asp Thr Tyr Asp Ala Leu His Met Gln Ala Leu Pro Pro Arg
450 455 460

<210> 78
<211> 507
<212> PRT
<213> Artificial Sequence

<220>
<223> Description of Artificial Sequence: artificially synthesized sequence

<400> 78

Asp Ile Gln Met Thr Gln Ser Pro Ser Ser Leu Ser Ala Ser Val Gly

```
1               5                   10                  15

Asp Arg Val Thr Ile Thr Cys His Ala Ser Gln Asp Ile Asn Thr Asn
            20                  25                  30

Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Gln Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255
```

```
Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Lys Arg Gly Arg Lys Lys Leu Leu Tyr Ile Phe Lys Gln Pro Phe Met
        355                 360                 365

Arg Pro Val Gln Thr Thr Gln Glu Glu Asp Gly Cys Ser Cys Arg Phe
    370                 375                 380

Pro Glu Glu Glu Glu Gly Gly Cys Glu Leu Arg Val Lys Phe Ser Arg
385                 390                 395                 400

Ser Ala Asp Ala Pro Ala Tyr Gln Gln Gly Gln Asn Gln Leu Tyr Asn
                405                 410                 415

Glu Leu Asn Leu Gly Arg Arg Glu Glu Tyr Asp Val Leu Asp Lys Arg
            420                 425                 430

Arg Gly Arg Asp Pro Glu Met Gly Gly Lys Pro Gln Arg Arg Lys Asn
        435                 440                 445

Pro Gln Glu Gly Leu Tyr Asn Glu Leu Gln Lys Asp Lys Met Ala Glu
    450                 455                 460

Ala Tyr Ser Glu Ile Gly Met Lys Gly Glu Arg Arg Arg Gly Lys Gly
465                 470                 475                 480

His Asp Gly Leu Tyr Gln Gly Leu Ser Thr Ala Thr Lys Asp Thr Tyr
                485                 490                 495

Asp Ala Leu His Met Gln Ala Leu Pro Pro Arg
            500                 505
```

<210> 79
<211> 465
<212> PRT
<213> Artificial Sequence

<220>
<223> Description of Artificial Sequence: artificially synthesized sequence

<400> 79

```
Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
        35                  40                  45

Tyr His Gly Thr Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Arg Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205
```

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Arg Val Lys Phe Ser Arg Ser Ala Asp Ala Pro Ala Tyr Gln Gln Gly
        355                 360                 365

Gln Asn Gln Leu Tyr Asn Glu Leu Asn Leu Gly Arg Arg Glu Glu Tyr
    370                 375                 380

Asp Val Leu Asp Lys Arg Arg Gly Arg Asp Pro Glu Met Gly Gly Lys
385                 390                 395                 400

Pro Gln Arg Arg Lys Asn Pro Gln Glu Gly Leu Tyr Asn Glu Leu Gln
                405                 410                 415

Lys Asp Lys Met Ala Glu Ala Tyr Ser Glu Ile Gly Met Lys Gly Glu
            420                 425                 430

Arg Arg Arg Gly Lys Gly His Asp Gly Leu Tyr Gln Gly Leu Ser Thr
        435                 440                 445

Ala Thr Lys Asp Thr Tyr Asp Ala Leu His Met Gln Ala Leu Pro Pro
    450                 455                 460

Arg
465

<210> 80
<211> 462
<212> PRT
<213> Artificial Sequence

<220>
<223> Description of Artificial Sequence: artificially synthesized sequence

<400> 80

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Thr Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Leu
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Arg Tyr Asn Pro Ser Leu Lys Ser Arg Ile Ser
180 185 190

```
Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Ile Tyr Ile Trp
        275                 280                 285

Ala Pro Leu Ala Gly Thr Cys Gly Val Leu Leu Leu Ser Leu Val Ile
    290                 295                 300

Thr Leu Tyr Cys Lys Arg Gly Arg Lys Lys Leu Leu Tyr Ile Phe Lys
305                 310                 315                 320

Gln Pro Phe Met Arg Pro Val Gln Thr Thr Gln Glu Glu Asp Gly Cys
                325                 330                 335

Ser Cys Arg Phe Pro Glu Glu Glu Glu Gly Gly Cys Glu Leu Arg Val
            340                 345                 350

Lys Phe Ser Arg Ser Ala Asp Ala Pro Ala Tyr Lys Gln Gly Gln Asn
        355                 360                 365

Gln Leu Tyr Asn Glu Leu Asn Leu Gly Arg Arg Glu Glu Tyr Asp Val
    370                 375                 380

Leu Asp Lys Arg Arg Gly Arg Asp Pro Glu Met Gly Gly Lys Pro Arg
385                 390                 395                 400

Arg Lys Asn Pro Gln Glu Gly Leu Tyr Asn Glu Leu Gln Lys Asp Lys
                405                 410                 415

Met Ala Glu Ala Tyr Ser Glu Ile Gly Met Lys Gly Glu Arg Arg Arg
            420                 425                 430

Gly Lys Gly His Asp Gly Leu Tyr Gln Gly Leu Ser Thr Ala Thr Lys
```

435 440 445

Asp Thr Tyr Asp Ala Leu His Met Gln Ala Leu Pro Pro Arg
450 455 460

<210> 81
<211> 507
<212> PRT
<213> Artificial Sequence

<220>
<223> Description of Artificial Sequence: artificially synthesized sequence

<400> 81

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Thr Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Leu
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Arg Tyr Asn Pro Ser Leu Lys Ser Arg Ile Ser

180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Phe Trp Val Leu
275 280 285

Val Val Val Gly Gly Val Leu Ala Cys Tyr Ser Leu Leu Val Thr Val
290 295 300

Ala Phe Ile Ile Phe Trp Val Arg Ser Lys Arg Ser Arg Leu Leu His
305 310 315 320

Ser Asp Tyr Met Asn Met Thr Pro Arg Arg Pro Gly Pro Thr Arg Lys
325 330 335

His Tyr Gln Pro Tyr Ala Pro Pro Arg Asp Phe Ala Ala Tyr Arg Ser
340 345 350

Lys Arg Gly Arg Lys Lys Leu Leu Tyr Ile Phe Lys Gln Pro Phe Met
355 360 365

Arg Pro Val Gln Thr Thr Gln Glu Glu Asp Gly Cys Ser Cys Arg Phe
370 375 380

Pro Glu Glu Glu Glu Gly Gly Cys Glu Leu Arg Val Lys Phe Ser Arg
385 390 395 400

Ser Ala Asp Ala Pro Ala Tyr Gln Gln Gly Gln Asn Gln Leu Tyr Asn
405 410 415

Glu Leu Asn Leu Gly Arg Arg Glu Glu Tyr Asp Val Leu Asp Lys Arg
420 425 430

Arg Gly Arg Asp Pro Glu Met Gly Gly Lys Pro Gln Arg Arg Lys Asn
435 440 445

Pro Gln Glu Gly Leu Tyr Asn Glu Leu Gln Lys Asp Lys Met Ala Glu
450 455 460

Ala Tyr Ser Glu Ile Gly Met Lys Gly Glu Arg Arg Arg Gly Lys Gly
465 470 475 480

His Asp Gly Leu Tyr Gln Gly Leu Ser Thr Ala Thr Lys Asp Thr Tyr
485 490 495

Asp Ala Leu His Met Gln Ala Leu Pro Pro Arg
500 505

<210> 82
<211> 465
<212> PRT
<213> Artificial Sequence

<220>
<223> Description of Artificial Sequence: artificially synthesized sequence

<400> 82

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ser Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Leu
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Arg Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Phe Trp Val Leu
275 280 285

Val Val Val Gly Gly Val Leu Ala Cys Tyr Ser Leu Leu Val Thr Val
290 295 300

Ala Phe Ile Ile Phe Trp Val Arg Ser Lys Arg Ser Arg Leu Leu His
305 310 315 320

Ser Asp Tyr Met Asn Met Thr Pro Arg Arg Pro Gly Pro Thr Arg Lys
325 330 335

His Tyr Gln Pro Tyr Ala Pro Pro Arg Asp Phe Ala Ala Tyr Arg Ser
340 345 350

Arg Val Lys Phe Ser Arg Ser Ala Asp Ala Pro Ala Tyr Gln Gln Gly
355 360 365

Gln Asn Gln Leu Tyr Asn Glu Leu Asn Leu Gly Arg Arg Glu Glu Tyr
370 375 380

Asp Val Leu Asp Lys Arg Arg Gly Arg Asp Pro Glu Met Gly Gly Lys
385 390 395 400

Pro Gln Arg Arg Lys Asn Pro Gln Glu Gly Leu Tyr Asn Glu Leu Gln
405 410 415

Lys Asp Lys Met Ala Glu Ala Tyr Ser Glu Ile Gly Met Lys Gly Glu
420 425 430

Arg Arg Arg Gly Lys Gly His Asp Gly Leu Tyr Gln Gly Leu Ser Thr
435 440 445

Ala Thr Lys Asp Thr Tyr Asp Ala Leu His Met Gln Ala Leu Pro Pro
450 455 460

Arg
465

<210> 83
<211> 462
<212> PRT
<213> Artificial Sequence

<220>
<223> Description of Artificial Sequence: artificially synthesized sequence

<400> 83

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ser Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Asn Pro Leu
85 90 95

Thr Phe Gly Gln Gly Thr Lys Val Glu Ile Lys Arg Gly Gly Gly Gly
100 105 110

```
Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Arg Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Ile Tyr Ile Trp
        275                 280                 285

Ala Pro Leu Ala Gly Thr Cys Gly Val Leu Leu Leu Ser Leu Val Ile
    290                 295                 300

Thr Leu Tyr Cys Lys Arg Gly Arg Lys Lys Leu Leu Tyr Ile Phe Lys
305                 310                 315                 320

Gln Pro Phe Met Arg Pro Val Gln Thr Thr Gln Glu Glu Asp Gly Cys
                325                 330                 335

Ser Cys Arg Phe Pro Glu Glu Glu Glu Gly Gly Cys Glu Leu Arg Val
            340                 345                 350

Lys Phe Ser Arg Ser Ala Asp Ala Pro Ala Tyr Lys Gln Gly Gln Asn
        355                 360                 365
```

Gln Leu Tyr Asn Glu Leu Asn Leu Gly Arg Arg Glu Glu Tyr Asp Val
    370                 375                 380

Leu Asp Lys Arg Arg Gly Arg Asp Pro Glu Met Gly Gly Lys Pro Arg
385                 390                 395                 400

Arg Lys Asn Pro Gln Glu Gly Leu Tyr Asn Glu Leu Gln Lys Asp Lys
                405                 410                 415

Met Ala Glu Ala Tyr Ser Glu Ile Gly Met Lys Gly Glu Arg Arg Arg
            420                 425                 430

Gly Lys Gly His Asp Gly Leu Tyr Gln Gly Leu Ser Thr Ala Thr Lys
        435                 440                 445

Asp Thr Tyr Asp Ala Leu His Met Gln Ala Leu Pro Pro Arg
    450                 455                 460

<210> 84
<211> 507
<212> PRT
<213> Artificial Sequence

<220>
<223> Description of Artificial Sequence: artificially synthesized sequence

<400> 84

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ser Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Asn Pro Leu
                85                  90                  95

Thr Phe Gly Gln Gly Thr Lys Val Glu Ile Lys Arg Gly Gly Gly Gly
            100                 105                 110

```
Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Arg Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Lys Arg Gly Arg Lys Lys Leu Leu Tyr Ile Phe Lys Gln Pro Phe Met
```

355 360 365

Arg Pro Val Gln Thr Thr Gln Glu Glu Asp Gly Cys Ser Cys Arg Phe
370 375 380

Pro Glu Glu Glu Glu Gly Gly Cys Glu Leu Arg Val Lys Phe Ser Arg
385 390 395 400

Ser Ala Asp Ala Pro Ala Tyr Gln Gln Gly Gln Asn Gln Leu Tyr Asn
405 410 415

Glu Leu Asn Leu Gly Arg Arg Glu Glu Tyr Asp Val Leu Asp Lys Arg
420 425 430

Arg Gly Arg Asp Pro Glu Met Gly Gly Lys Pro Gln Arg Arg Lys Asn
435 440 445

Pro Gln Glu Gly Leu Tyr Asn Glu Leu Gln Lys Asp Lys Met Ala Glu
450 455 460

Ala Tyr Ser Glu Ile Gly Met Lys Gly Glu Arg Arg Arg Gly Lys Gly
465 470 475 480

His Asp Gly Leu Tyr Gln Gly Leu Ser Thr Ala Thr Lys Asp Thr Tyr
485 490 495

Asp Ala Leu His Met Gln Ala Leu Pro Pro Arg
500 505

<210> 85
<211> 465
<212> PRT
<213> Artificial Sequence

<220>
<223> Description of Artificial Sequence: artificially synthesized sequence

<400> 85

Asp Ile Gln Met Thr Gln Ser Pro Ser Ser Leu Ser Ala Ser Val Gly
1 5 10 15

Asp Arg Val Thr Ile Thr Cys His Ala Ser Gln Asp Ile Asn Val Asn
20 25 30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly

```
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Phe Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Arg Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Phe Trp Val Leu
        275                 280                 285

Val Val Val Gly Gly Val Leu Ala Cys Tyr Ser Leu Leu Val Thr Val
    290                 295                 300
```

Ala Phe Ile Ile Phe Trp Val Arg Ser Lys Arg Ser Arg Leu Leu His
305                 310                 315                 320

Ser Asp Tyr Met Asn Met Thr Pro Arg Arg Pro Gly Pro Thr Arg Lys
                325                 330                 335

His Tyr Gln Pro Tyr Ala Pro Pro Arg Asp Phe Ala Ala Tyr Arg Ser
            340                 345                 350

Arg Val Lys Phe Ser Arg Ser Ala Asp Ala Pro Ala Tyr Gln Gln Gly
        355                 360                 365

Gln Asn Gln Leu Tyr Asn Glu Leu Asn Leu Gly Arg Arg Glu Glu Tyr
    370                 375                 380

Asp Val Leu Asp Lys Arg Arg Gly Arg Asp Pro Glu Met Gly Gly Lys
385                 390                 395                 400

Pro Gln Arg Arg Lys Asn Pro Gln Glu Gly Leu Tyr Asn Glu Leu Gln
                405                 410                 415

Lys Asp Lys Met Ala Glu Ala Tyr Ser Glu Ile Gly Met Lys Gly Glu
            420                 425                 430

Arg Arg Arg Gly Lys Gly His Asp Gly Leu Tyr Gln Gly Leu Ser Thr
        435                 440                 445

Ala Thr Lys Asp Thr Tyr Asp Ala Leu His Met Gln Ala Leu Pro Pro
    450                 455                 460

Arg
465

<210> 86
<211> 462
<212> PRT
<213> Artificial Sequence

<220>
<223> Description of Artificial Sequence: artificially synthesized sequence

<400> 86

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

```
Ile Gly Trp Leu Gln Gln Lys Pro Gly Lys Ala Phe Lys Gly Leu Ile
        35                  40                  45

Tyr His Gly Lys Asn Leu Glu Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Asn Gln Tyr Glu Asn Phe Pro Leu
                85                  90                  95

Thr Phe Gly Gln Gly Thr Lys Val Glu Ile Lys Arg Gly Gly Gly Gly
            100                 105                 110

Ser Gly Gly Gly Gly Ser Gly Gly Gly Gly Ser Asp Val Gln Leu Val
        115                 120                 125

Glu Ser Gly Gly Gly Leu Val Gln Pro Gly Gly Ser Leu Arg Leu Ser
    130                 135                 140

Cys Ala Val Ser Gly Tyr Ser Ile Thr Ser Asp Tyr Ala Trp Asn Trp
145                 150                 155                 160

Ile Arg Gln Ala Pro Gly Lys Gly Leu Glu Trp Leu Gly Tyr Ile Ser
                165                 170                 175

Tyr Arg Gly Arg Thr Arg Tyr Asn Pro Ser Leu Lys Ser Arg Ile Ser
            180                 185                 190

Ile Thr Arg Asp Asn Ser Lys Asn Thr Phe Phe Leu Gln Leu Asn Ser
        195                 200                 205

Leu Arg Ala Glu Asp Thr Ala Val Tyr Tyr Cys Ala Arg Leu Gly Arg
    210                 215                 220

Gly Phe Arg Tyr Trp Gly Gln Gly Thr Leu Val Thr Val Ser Ser Thr
225                 230                 235                 240

Thr Thr Pro Ala Pro Arg Pro Pro Thr Pro Ala Pro Thr Ile Ala Ser
                245                 250                 255

Gln Pro Leu Ser Leu Arg Pro Glu Ala Cys Arg Pro Ala Ala Gly Gly
            260                 265                 270

Ala Val His Thr Arg Gly Leu Asp Phe Ala Cys Asp Ile Tyr Ile Trp
        275                 280                 285
```

Ala Pro Leu Ala Gly Thr Cys Gly Val Leu Leu Leu Ser Leu Val Ile
    290                 295                 300

Thr Leu Tyr Cys Lys Arg Gly Arg Lys Lys Leu Leu Tyr Ile Phe Lys
305                 310                 315                 320

Gln Pro Phe Met Arg Pro Val Gln Thr Thr Gln Glu Glu Asp Gly Cys
                325                 330                 335

Ser Cys Arg Phe Pro Glu Glu Glu Glu Gly Gly Cys Glu Leu Arg Val
            340                 345                 350

Lys Phe Ser Arg Ser Ala Asp Ala Pro Ala Tyr Lys Gln Gly Gln Asn
        355                 360                 365

Gln Leu Tyr Asn Glu Leu Asn Leu Gly Arg Arg Glu Glu Tyr Asp Val
    370                 375                 380

Leu Asp Lys Arg Arg Gly Arg Asp Pro Glu Met Gly Gly Lys Pro Arg
385                 390                 395                 400

Arg Lys Asn Pro Gln Glu Gly Leu Tyr Asn Glu Leu Gln Lys Asp Lys
                405                 410                 415

Met Ala Glu Ala Tyr Ser Glu Ile Gly Met Lys Gly Glu Arg Arg Arg
            420                 425                 430

Gly Lys Gly His Asp Gly Leu Tyr Gln Gly Leu Ser Thr Ala Thr Lys
        435                 440                 445

Asp Thr Tyr Asp Ala Leu His Met Gln Ala Leu Pro Pro Arg
    450                 455                 460

<210> 87
<211> 507
<212> PRT
<213> Artificial Sequence

<220>
<223> Description of Artificial Sequence: artificially synthesized sequence

<400> 87

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Val Asn
            20                  25                  30

Ile Gly Trp Leu Gln Gln Lys Pro Gly Lys Ala Phe Lys Gly Leu Ile
35 40 45

Tyr His Gly Lys Asn Leu Glu Asp Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Asn Gln Tyr Glu Asn Phe Pro Leu
85 90 95

Thr Phe Gly Gln Gly Thr Lys Val Glu Ile Lys Arg Gly Gly Gly Gly
100 105 110

Ser Gly Gly Gly Gly Ser Gly Gly Gly Gly Ser Asp Val Gln Leu Val
115 120 125

Glu Ser Gly Gly Gly Leu Val Gln Pro Gly Gly Ser Leu Arg Leu Ser
130 135 140

Cys Ala Val Ser Gly Tyr Ser Ile Thr Ser Asp Tyr Ala Trp Asn Trp
145 150 155 160

Ile Arg Gln Ala Pro Gly Lys Gly Leu Glu Trp Leu Gly Tyr Ile Ser
165 170 175

Tyr Arg Gly Arg Thr Arg Tyr Asn Pro Ser Leu Lys Ser Arg Ile Ser
180 185 190

Ile Thr Arg Asp Asn Ser Lys Asn Thr Phe Phe Leu Gln Leu Asn Ser
195 200 205

Leu Arg Ala Glu Asp Thr Ala Val Tyr Tyr Cys Ala Arg Leu Gly Arg
210 215 220

Gly Phe Arg Tyr Trp Gly Gln Gly Thr Leu Val Thr Val Ser Ser Thr
225 230 235 240

Thr Thr Pro Ala Pro Arg Pro Pro Thr Pro Ala Pro Thr Ile Ala Ser
245 250 255

Gln Pro Leu Ser Leu Arg Pro Glu Ala Cys Arg Pro Ala Ala Gly Gly
260 265 270

Ala Val His Thr Arg Gly Leu Asp Phe Ala Cys Asp Phe Trp Val Leu
275 280 285

Val Val Val Gly Gly Val Leu Ala Cys Tyr Ser Leu Leu Val Thr Val
290 295 300

Ala Phe Ile Ile Phe Trp Val Arg Ser Lys Arg Ser Arg Leu Leu His
305 310 315 320

Ser Asp Tyr Met Asn Met Thr Pro Arg Arg Pro Gly Pro Thr Arg Lys
325 330 335

His Tyr Gln Pro Tyr Ala Pro Pro Arg Asp Phe Ala Ala Tyr Arg Ser
340 345 350

Lys Arg Gly Arg Lys Lys Leu Leu Tyr Ile Phe Lys Gln Pro Phe Met
355 360 365

Arg Pro Val Gln Thr Thr Gln Glu Glu Asp Gly Cys Ser Cys Arg Phe
370 375 380

Pro Glu Glu Glu Glu Gly Gly Cys Glu Leu Arg Val Lys Phe Ser Arg
385 390 395 400

Ser Ala Asp Ala Pro Ala Tyr Gln Gln Gly Gln Asn Gln Leu Tyr Asn
405 410 415

Glu Leu Asn Leu Gly Arg Arg Glu Glu Tyr Asp Val Leu Asp Lys Arg
420 425 430

Arg Gly Arg Asp Pro Glu Met Gly Gly Lys Pro Gln Arg Arg Lys Asn
435 440 445

Pro Gln Glu Gly Leu Tyr Asn Glu Leu Gln Lys Asp Lys Met Ala Glu
450 455 460

Ala Tyr Ser Glu Ile Gly Met Lys Gly Glu Arg Arg Arg Gly Lys Gly
465 470 475 480

His Asp Gly Leu Tyr Gln Gly Leu Ser Thr Ala Thr Lys Asp Thr Tyr
485 490 495

Asp Ala Leu His Met Gln Ala Leu Pro Pro Arg
500 505

## Description

### TECHNICAL FIELD

This application belongs to the field of cellular immunotherapy, and particularly relates to a combined anti-tumor immunotherapy of a targeted tumor-specific antigen and a PARP inhibitor.

### BACKGROUND OF THE INVENTION

In recent years, immune cell therapy, especially CAR-T cell therapy, has shown amazing therapeutic effect in the treatment of B-cell tumor. At present, there are more than 200 clinical trials about CAR-T cells for the treatment of hematological tumors (Clinical development of CAR T cells-challenges and opportunities in translating innovative treatment concepts, Jessica Hartmann et al., EMBO Molecule Medicine, Published online, August 1, 2017).

However, due to the complexity of organisms, especially the microenvironment of solid tumors, drug candidates showing excellent effect *in vitro* often fail to show corresponding effects *in vivo.* In other words, the *in vitro* results of a candidate drug cannot reasonably predict the effect *in vivo.* In addition, the same antibody has different effects on tumors in different parts with the same target. For example, trastuzumab has a good therapeutic effect when applied to HER2-positive breast cancer, but it has no effect when applied to HER2-positive gastric cancer (Qiang FU et al., Progress of HER2 Signaling Pathway in Gastric Cancer and Clinical Application of Trastuzumab, YAO PIN PING JIA, 2012, vol. 9(27): 8-12).

Although immune effector cells have attractive prospects in tumor immunotherapy, CAR-T cells face many problems when applied to solid tumors, wherein the key step to take effect in CAR-T cell therapy is to home the cells to the tumor location and activate them for proliferation.

In recent years, PARP inhibitors have made breakthrough progress in the development of anti-cancer drugs. The anti-cancer mechanisms of PARP inhibitors mainly include: (1) blocking DNA damage repair to cause accumulation of DNA damage, and ultimately killing tumor cells; (2) increasing the sensitivity of cells to other endogenous and exogenous DNA damage factors; (3) inhibiting angiogenesis; (4) enhancing the immunity of normal cells to resist the invasion of cancer cells. The researches of many PARP inhibitors have entered clinical trials. Among them, olaparib (AZD2281) is the third-generation PARP inhibitor with the most extensive clinical research so far, and it is a strong and effective PARP-1 inhibitor. However, some patients are still not sensitive to these drugs.

Therefore, this research aims to combine with appropriate small molecule drugs to enhance the immunogenicity of tumor cells, reduce tumor immunosuppressive factors, and promote the homing and continuous activation of CAR-T cells, thereby improving the anti-tumor activity of CAR-T cells themselves.

### CONTENTS OF THE INVENTION

The purpose of this application is to provide a method for treating a tumor, wherein a small molecule drug is combined to use for improving the anti-tumor effect of immune cell therapy, especially CAR-T cell therapy in solid tumor treatment.

In one aspect of the application, provided is a method for treating a tumor, which comprises: administering an immune effector cell and a PARP inhibitor to an individual suffering from a tumor, wherein the immune effector cell expresses a receptor recognizing a tumor antigen.

In one aspect of the application, provided is a method for reducing the growth, survival, or viability, or all the above of cancer cells, which comprises: administering an immune effector cell and a PARP inhibitor to an individual suffering from a tumor, wherein the immune effector cell expresses a receptor recognizing a tumor antigen.

In at least one particular aspect, the PARP inhibitor is any one selected from the group consisting of: talazoparib, niraparib, olaparib, rucaparib, niraparib, pamiparib, fluzoparib, mefuparib, and simmiparib.

In at least one particular aspect, the PARP inhibitor is olaparib.

In at least one particular aspect, the therapeutic effect of the immune effector cell and the PARP inhibitor is greater than that of either the immune effector cell or the PARP inhibitor used alone.

In at least one particular aspect, the above method of the present application increases the CAR-T copy number of tumor tissue.

In at least one particular aspect, the above method of the present application increases the expression level of IFN-$\gamma$ in tumor tissue.

In at least one particular aspect, the above method of the present application increases CD8+ T cell immune cell infiltration in tumor tissue.

In at least one particular aspect, the above method of the present application increases CD45+ immune cell infiltration.

In at least one particular aspect, the above method of the present application reduces CD11b+Ly6G+ MDSCs cell infiltration.

In at least one particular aspect, the above method of the present application described above reduces CD31+ cell infiltration.

In at least one particular aspect, the above method of the present application does not cause a significant decrease in the expression of PD1, LAG3, and/or TIM3.

In at least one particular aspect, in the above method of the present application, the manner of administering an immune effector cell and a PARP inhibitor to an individual suffering from a tumor is any one selected from: (1) firstly administering the PARP inhibitor and then the immune effector cell, (2) simultaneously administering the immune effector cell and the PARP inhibitor, and (3) firstly administering the immune effector cell and then the PARP inhibitor.

In at least one particular aspect, in the above method of the present application, the PARP inhibitor is administered firstly and then the immune effector cell.

In at least one particular aspect, in the above method of the present application, the immune effector cell and PARP inhibitor are administered simultaneously.

In at least one particular aspect, in the above method of the present application, the immune effector cell is administered firstly and then the PARP inhibitor.

In at least one particular aspect, the receptor is selected from: chimeric antigen receptor (CAR), T cell receptor (TCR), T cell fusion protein (TFP), T cell antigen coupler (TAC), or a combination thereof.

In at least one particular aspect, the tumor antigen is selected from EGFR or EGFRvIII.

In at least one particular aspect, the chimeric antigen receptor has: (i) an antibody or a fragment thereof specifically recognizing a tumor antigen, the transmembrane region of CD28 or CD8, the costimulatory signal domain of CD28, and CD3ζ; or (ii) an antibody or a fragment thereof specifically recognizing a tumor antigen, the transmembrane region of CD28 or CD8, the costimulatory signal domain of CD137, and CD3ζ; or (iii) an antibody or a fragment thereof specifically recognizing a tumor antigen, the transmembrane region of CD28 or CD8, the costimulatory signal domain of CD28, the costimulatory signal domain of CD137, and CD3ζ.

In at least one particular aspect, the above antibody specifically recognizing a tumor antigen is an antibody targeting EGFR or EGFRvIII, further preferably the antibody has an amino acid sequence selected from any one of the group consisting of: SEQ ID NOs: 11-29.

In at least one particular aspect, the above chimeric antigen receptor has an amino acid sequence selected from any one of the group consisting of: SEQ ID NOs: 30-51, and SEQ ID NOs: 55-87.

In at least one particular aspect, the above tumor comprises: breast cancer, colon cancer, rectal cancer, renal cell carcinoma, liver cancer, lung cancer, small intestine cancer, esophageal cancer, melanoma, bone cancer, pancreatic cancer, skin cancer, head and neck cancer, uterine cancer, ovarian cancer, rectal cancer, stomach cancer, testicular cancer, fallopian tube cancer, endometrial cancer, cervical cancer, vagina cancer, thyroid cancer, parathyroid cancer, adrenal cancer, soft tissue sarcoma, urethral cancer, penile cancer, bladder cancer, ureter cancer, renal pelvis cancer, spinal tumor, glioma, pituitary adenoma, Kaposi's sarcoma, a combination and the metastatic foci thereof.

In at least one particular aspect, the above immune effector cell is selected from: T cell, B cell, natural killer (NK) cell, natural killer T (NKT) cell, mast cell, or bone marrow-derived phagocytic cell, or a combination thereof; preferably the immune effector cell is selected from autologous T cell, allogeneic T cell, or allogeneic NK cell; more preferably the T cell is autologous T cell.

In at least one particular aspect, the above PARP inhibitor is administered by oral administration, intraperitoneal administration and/or injection.

In at least one particular aspect, in the above method, lymphocyte clearance is not performed on the individual.

In another aspect, the application provides use of an immune effector cell expressing a receptor recognizing a tumor antigen and a PARP inhibitor in the preparation of a medicament, wherein the medicament is used for treating a tumor or reducing the growth, survival or viability of a cancer cell.

In at least one particular aspect, the therapeutic effect of the immune effector cell and the PARP inhibitor is greater than that of either the immune effector cell or the PARP inhibitor used alone.

In another aspect, the application provides use of an immune effector cell expressing a receptor recognizing a tumor antigen in the preparation of a medicament, wherein the medicament comprises the immune effector cell and a PARP inhibitor and is used for treating a tumor or reducing the growth, survival or viability of a cancer cell in a human patient.

In at least one particular aspect, the therapeutic effect of the immune effector cell and the PARP inhibitor is greater than that of either the immune effector cell or the PARP inhibitor used alone.

In at least one particular aspect, the PARP inhibitor comprises: talazoparib, niraparib, olaparib, rucaparib, niraparib, pamiparib, fluzoparib, mefuparib and/or simmiparib.

In at least one particular aspect, the immune effector cell and the PARP inhibitor are administrated in no particular order. The PARP inhibitor may the administrated firstly and then the immune effector cell; or they are the administrated simultaneously; or the immune effector cell may be administrated firstly and then the PARP inhibitor; preferably the PARP inhibitor is olaparib.

In at least one particular aspect, the receptor is selected from: chimeric antigen receptor (CAR), T cell receptor (TCR), T cell fusion protein (TFP), T cell antigen coupler (TAC), or a combination thereof.

In at least one particular aspect, the chimeric antigen receptor has: (i) an antibody specifically recognizing a tumor antigen, the transmembrane region of CD28 or CD8, the costimulatory signal domain of CD28, and CD3ζ; or (ii) an antibody specifically recognizing a tumor antigen, the transmembrane region of CD28 or CD8, the costimulatory signal domain of CD137, and CD3ζ; or (iii) an antibody specifically recognizing a tumor antigen, the transmembrane region of CD28 or CD8, the costimulatory signal domain of CD28, the costimulatory signal domain of CD137, and CD3ζ.

In at least one particular aspect, the above tumor comprises: breast cancer, colon cancer, rectal cancer, renal cell carcinoma, liver cancer, lung cancer, small intestine cancer, esophageal cancer, melanoma, bone cancer, pancreatic cancer, skin cancer, head and neck cancer, uterine cancer, ovarian cancer, rectal cancer, stomach cancer, testicular cancer, fallopian tube cancer, endometrial cancer, cervical cancer, vagina cancer, thyroid cancer, parathyroid cancer, adrenal cancer, soft tissue sarcoma, urethral cancer, penile cancer, bladder cancer, ureter cancer, renal pelvis cancer, spinal tumor, glioma, pituitary adenoma, Kaposi's sarcoma, a combination and the metastatic foci thereof. In at least one particular aspect, the immune effector cell comprises: T cell, B cell, natural killer (NK) cell, natural killer T (NKT) cell, mast cell, or bone marrow-derived phagocytic cell, or a combination thereof; preferably the immune effector cell is selected from autologous T cell, allogeneic T cell, or allogeneic NK cell; more preferably the T cell is autologous T cell.

In at least one particular aspect, when the medicament or kit of the application is used, the PARP inhibitor is administered by oral administration, intraperitoneal administration and/or injection; preferably the PARP inhibitor is olaparib.

In at least one particular aspect, when the medicament or kit of the application is used, lymphocyte clearance is not performed on the individual.

It should be understood that within the scope of the present invention, the above technical features of the present invention and the technical features specifically described in the following (such as the Examples) may be combined with each other to form a new or preferred technical solution. Due to the limitation of space, they will not be repeated herein one by one.

## BRIEF DESCRIPTION OF THE DRAWINGS

Fig. 1A is the plasmid map of the recombinant vector MSCV-806-mCD28z; Fig. 1B is the detection of the positive rate of CAR-T infection.

Fig. 2A shows the detection of EGFRvIII positive cells; Fig. 2B shows the detection of the expression of PARP1 in the cells treated with olaparib; Fig. 2C shows the CCK8 experiment detecting the toxicity of olaparib on breast cancer cells and CAR-T cells.

Fig. 3A shows the cytotoxicity test detecting the killing effect on tumor cells by olaparib pretreatment plus CAR-T treatment or the combination of olaparib and CAR T; Fig. 3B shows the ELISA test detecting the effect of olaparib on the secretion of cytokine TNFα, IL-2 and IFN-γ after activating the CAR-T cells.

Fig. 4A shows the effect on cell proliferation after incubating the CAR-T in the olaparib pretreatment group and the target cells together in Example 4; Fig. 4B shows changes of the expression of exhaustion-related proteins PD1, LAG3 and TIM3 on the surface of CAR-T cells after incubating the CAR-T in the olaparib pretreatment group and the target cells together in Example 4.

Fig. 5 shows the experiments of the treatment of orthotopic breast cancer xenografts in mice by combination of olaparib and CAR-T: Fig. 5A shows the experimental flowchart; Fig. 5B shows the detection results of the tumor volumes; Fig. 5C shows the detection of changes in mouse body weights; Fig. 5D shows the detection of the mouse tumor weights; Fig. 5E shows the detection of CAR copy number in tumor tissues; Fig. 5F shows the immunohistochemical detection of tumor tissue CD4/CD8/CD31; Fig. 5G shows the detection of MDSC cells in tumor tissues.

Fig. 6 shows the experiments of the treatment of orthotopic breast cancer xenografts in mice by combination of olaparib and CAR-T: Fig. 6A shows the experimental flowchart; Fig. 6B shows the detection results of the tumor volumes; Fig. 6C shows the detection of changes in mouse body weights; Fig. 6D shows the detection of the mouse tumor weights; Fig. 6E shows the detection of CAR copy number in tumor tissues; Fig. 6F shows the immunohistochemical detection of tumor tissue CD4/CD8/CD31; Fig. 6G shows the detection of MDSC cells in tumor tissues.

## SPECIFIC EMBODIMENTS

This application relates to the treatment of a tumor by combination of an immune effector cell and a PARP inhibitor. It should be understood that the present invention is not limited to the described methods and experimental conditions. Unless specifically defined, all technical and scientific terms used herein have the same meaning as commonly understood by those skilled in the fields of gene therapy, biochemistry, genetics, molecular biology, and medicinal chemistry.

All methods and materials similar or equivalent to those described herein may be used in the practice or testing of the present invention, wherein suitable methods and materials are described herein. All publications, patent applications, patents and other references mentioned in this disclosure are incorporated herein by reference in their entirety. In case of conflict, the present specification, including definitions, will control. In addition, unless otherwise specified, the materials, methods, and examples are illustrative only and not intended to be limiting.

Unless otherwise specified, the practice of this application will use traditional techniques of cell biology, cell culture, molecular biology, transgenic biology, microbiology, recombinant DNA and immunology, and they all fall within the technical scope of this field. These techniques are fully explained in the references. See, for example, Current Protocols in Molecular Biology (Frederick M. Ausubel, 2000, Wiley and Son Inc, Library of Congress, USA); Molecular Cloning: A Laboratory Manual, Third Edition, (Sambrook et al., 2001, Cold Spring Harbor, New York: Cold Spring Harbor Laboratory Press); Oligonucleotide Synthesis (M. J. Gaited., 1984); Mullis et al. U.S. Pat. No. 4,683,195; Nucleic Acid Hybridization (B. D. Harries & S. J. Higginseds.1984); Transcription And Translation (B. D. Hames & S. J. Higginseds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In Enzymology (J. Abelson and M. Simon, eds.-in-chief, Academic Press, Inc., New York), especially Vols. 154 and 155 (Wu et al. eds.), and Vol.185, "Gene Expression Technology" (D. Goeddel, ed.); Gene Transfer Vectors for Mammalian Cells (J. H. Miller and M. P. Calos eds., 1987, Cold Spring Harbor Laboratory); Immunochemical Methods In Cell and Molecular Biology (Mayer and Walker, eds., Academic Press, London, 1987); Hand book of Experimental Immunology, Vols. I-IV (D. M. Weir and C. C. Blackwell, eds., 1986); and Manipulating the Mouse Embryo (Cold Spring Harbor Laboratory Press, Cold Spring Harbor, N.Y., 1986).

The present disclosure is derived at least in part from the following groundbreaking recognition: one or more cycles and/or doses of combined treatment regimen including consecutive, in any order, or substantially simultaneous administration of low-dose olaparib and immune effector cells may be more effective in the treatment of a cancer in some subjects, and/or it may initiate, achieve, increase, enhance or prolong the activity and/or number of immune cells, or may bring medically beneficial response for tumor treatment.

The term "PARP" is a poly (ADP-ribose) polymerase, which participates in cellular processes including chromosome remodeling, regulation of cell apoptosis, and cell division. PARP also plays a role in the immune response. So far, 17 family members including PARP1, PARP2, PARP3, Vault-PARP and Tankyrase1 have been found, wherein, PARP1 is the most abundant, earliest discovered, and most extensively researched member of the PARP protein family, accounting for more than 80% of the total activity of PARP proteins in the cell. The biological function of PARP1 is that: the activated PARP1 forms a homodimer, cleaves the carbon-hydrogen bonds of NAD+, generates nicotinamide and adenosine diphosphate ribose, and further transfers and adds the latter to specific amino acid residues of specific nuclear receptor proteins (including itself), and they are continuously added to form a straight or branched PARP long chain. PARP1 plays an important role in the physiological processes including DNA damage repair, gene transcription and expression, maintenance of genome stability, regulation of cell cycle, and cell apoptosis. At present, three PARP inhibitor drugs have been approved by the U.S. Food and Drug Administration (FDA) for marketing.

Olaparib, as an oral inhibitor of PARP1 and PARP2, received accelerated approval from the FDA in December 2014 to be marketed under the trade name Lynparza, and it is the world's first PARP inhibitor to be marketed. Olaparib is used for monotherapy of BRCA-mutated advanced ovarian cancer that has undergone chemotherapy with 3 or more drugs. Currently, olaparib is used for treating advanced ovarian cancer and BRAC-deficient triple-negative breast cancer that have recurred after platinum chemotherapy and is in phase III clinical trials, and the treatment of gastric cancer by combination of olaparib with paclitaxel is also in phase III clinical trials. In addition, the treatment of prostate cancer with olaparib has entered the phase II clinical stage; however, some patients are still not sensitive to this drug.

In a specific embodiment, the PARP inhibitors used in the present application include but are not limited to: talazoparib, niraparib, olaparib, rucaparib, veliparib, niraparib, pamiparib, fluzoparib, mefuparib, and/or simmiparib, they are used in combination with CAR-T cells for treating breast cancer.

The applicant found that in *in vitro* experiments: olaparib at a concentration higher than 1 μM may inhibit the growth of tumor cells, olaparib at a concentration higher than 2.5 μM may inhibit the growth of CAR-T cells; olaparib may significantly increase the killing effect of CAR-T cells on E0771-EGFRvIII cells and/or 4T1-EGFRvIII cells; especially in the 5μM olaparib pretreatment group, the promotion of killing effect is the most significant and is more effective than the 5μM olaparib combined treatment group, suggesting that higher concentrations of olaparib may inhibit the growth of CAR-T to a certain extent; compared with the CAR-T single treatment group, the levels of IFN-γ in the cell culture supernatant in the olaparib pretreatment group and the olaparib combined treatment group are increased significantly.

In this application, olaparib may be used to significantly improve the killing effect of CAR-T cells on E0771-EGFRvIII and 4T1-EGFRvIII cells, especially in the 5μM olaparib pretreatment group (i.e., firstly olaparib is used, then CAR-T cells is provided for treatment) the promotion of killing effect is the most significant and is better than that of the 5μM olaparib combined treatment group (i.e., firstly olaparib is used, then olaparib and CAR-T cells are used simultaneously for treatment), indicating that higher concentrations of olaparib may inhibit the growth of CAR-T to a certain extent. However, compared with the CAR-T single treatment group, the 5μM olaparib combined treatment group still has a higher synergistic lethal effect.

Compared with CAR-T single treatment group, the levels of IFN-γ in the supernatant of the co-culture of E0771-EGFRvIII cells and CAR-cells in the olaparib pretreatment group and the olaparib combined treatment group are increased significantly, indicating that olaparib may effectively improve the killing ability of CAR-T cells against tumor cells. After CAR-T in the olaparib pretreatment group is incubated with cancer cells, the proliferation of CAR-T cells is not significantly affected; while in the olaparib combined treatment group, the proliferation of CAR-T cells is slightly inhibited due to the presence of olaparib in the co-culture environment, but it was not statistically significant. After CAR-T cells in the olaparib pretreatment group are incubated with cancer cells, the expressions of exhaustion-related proteins PD1, LAG3, and TIM3 on the surface of CAR-T cells do not change significantly; after in the olaparib combined treatment group, the expression of exhaustion-related proteins PD1, LAG3 and TIM3 on the surface of CAR-T cells does not change significantly as well. This suggests that olaparib treatment does not lead to CAR-T depletion, and thus does not affect the killing effect of effector T cells, indicating that olaparib is used in combination with CAR-T cells.

The applicant found in the experiment that, the method or pharmaceutical preparation or kit according to the present application may be used to effectively increase the CAR-T copy number of tumor tissues, increase the expression level of IFN-γ in tumor tissues, increase immune cell infiltration of CD8+ T cells in tumor tissues, and increase immune cell infiltration of CD45+ cells, reduce cell infiltration of CD11b+Gr1+ MDSCs, and reduce cell infiltration of CD31+ cells.

It is found in further in vivo experiments that, the treatment of CAR-T cells in combination with 50mg/kg olaparib may significantly increase the copy number of CAR in tumor tissues in mice, improve the immunosuppressive tumor environment, and does not affect the weight of mice, but may obviously inhibit the tumor volume and tumor weight of mouse breast cancer, indicating that olaparib may significantly enhance the tumor cell growth inhibition effect of CAR-T cells by homing CAR-T cells to a local part of the middle tumor and activating the proliferation. In *in vitro* experiments, another PARP inhibitor, veliparib, may affect the proliferation level of E0771ERVIII cells at a concentration level of 10μM or more, and may significantly inhibit the proliferation of E0771ERVIII cells at a concentration of 20μM or more, but at this concentration it may significantly inhibit the proliferation of mouse CAR-T cells; it is found in the LDH cell killing experiment that, 10μM Veliparib could not improve the killing effect of CART cells. Another small molecule drug JQ1 is a BET bromodomain inhibitor acting on BRD4, it may induce tumor cell apoptosis (i.e., slow proliferation) by targeted inhibition of BRD4, thereby achieving anti-tumor effects. In *in vitro* experiments, after treating E0771ERVIII cells with different concentrations of JQ1 for 48h, it is found that JQ1 could not inhibit the proliferation of E0771ERVIII cells, but it significantly inhibit the proliferation of mouse T cells and CAR-T cells in a dose-dependent manner.

The applicant also found that, this application may not only improve the anti-cancer effect of refractory cancers, but also does not require lymphocyte clearance when using CAR-T cells, thereby greatly reducing the low anti-cancer treatment effect caused by lymphocyte clearance, and reducing the toxic side effects caused by damage to normal tissues, especially severe suppression of bone marrow.

In particular embodiments, the PARP inhibitor itself may be safety administered by oral or non-oral administration, or the PARP inhibitor may be safety administered in a composition formed with a pharmaceutically acceptable carrier, excipient, or other additive (such as a tablet, sustained-release preparation, capsule, injection, solution) by oral or non-oral administration. When administered orally, the composition may be formulated into a tablet, dragee or capsule. To prepare an oral composition, lactose or starch may be used as a carrier, and gelatin, sodium carboxymethyl cellulose, methyl cellulose polyvinylpyrrolidone, etc. are suitable binding agents or granulating agents. Starch or microcrystalline cellulose may be used as the disintegrant, and talc, colloidal silica, glyceryl stearate, calcium or magnesium stearate, etc. are often used as suitable anti-adhesive agents and lubricants. For example, a tablet may be prepared by compressing wet granules. The active ingredient, the carrier, and optionally a part of the degrading additive are used to form a mixture, and the mixture and the aqueous solution, alcoholic or aqueous alcoholic solution of a binder are granulated in a suitable equipment; the dried granules are then mixed with other degrading agent, lubricant and anti-sticking agent, and the mixture is squashed into tablets. In order to increase the solubility, the heterocyclic derivatives may be freed and made into pharmaceutically acceptable organic acids, preferably methanesulfonic acid, fumaric acid, etc., to facilitate administration by injection, although the dosage will change depends on the subject to be treated, the manner of administration, symptoms and other factors.

In a specific embodiment, the PARP inhibitor is an oral chemotherapeutic drug olaparib (AZD2281) for treating advanced breast cancer, ovarian cancer, and the like.

In certain embodiments, the daily oral dose of olaparib for an individual with a tumor is: about 700, 650, 600, 550, 500, 450, 400, 350, 300, 290, 280, 270, 260, 250, 240, 230, 220, 210, 200, 190, 180, 170, 160, 150, 140, 130, 120, 110, 100, 90, 80, 70, 60, 50, 40, 30, 20, 10, 9, 8, 7, 6, 5, 4, 3, 2, or 1 mg.

In this application, the administration of an immune effector cell and a PARP inhibitor has no particular order; the PARP inhibitor may be firstly administrated, and then the immune effector cell; they may also be administered simultaneously; and the immune effector cell may also be firstly administrated and then the PARP inhibitor. In certain embodiments, the treatment of the immune effector cell is 1 hour, 2 hours, 3 hours, 4 hours, 5 hours, 6 hours, 7 hours, 8 hours, 9 hours, 10 hours, 11 hours, 12 hours, 1 day, 2 days, 3 days, 4 days, 5 days, 6 days, 7 days, 8 days, 9 days, 10 days, 11 days, 12 days, 13 days, 14 days, 15 days, 16 days , 17 days, 18 days, 19 days, 20 days, 21 days, 22 days, 23 days, 24 days, 25 days, 26 days, 27 days, 28 days, 29 days, 1 month or any combination thereof before administration of the PARP inhibitor. In certain embodiments, the treatment of the immune effector cell is 1 hour, 2 hours, 3 hours, 4 hours, 5 hours, 6 hours, 7 hours, 8 hours, 9 hours, 10 hours, 11 hours, 12 hours, 1 day, 2 days, 3 days, 4 days, 5 days, 6 days, 7 days, 8 days, 9 days, 10 days, 11 days, 12 days, 13 days, 14 days, 15 days, 16 days , 17 days, 18 days, 19 days, 20 days, 21 days, 22 days, 23 days, 24 days, 25 days, 26 days, 27 days, 28 days, 29 days, 1 month or any combination thereof after administration of the PARP inhibitor.

The term "an immune effector cell" refers to a cell participating in immune response and producing immune effect, such as T cell, B cell, natural killer (NK) cell, natural killer T (NKT) cell, mast cell, and bone marrow-derived phagocyte; preferably the immune effector cell is a T cell, NK cell, and NKT cell.

In a specific embodiment, the T cell is an autologous T cell, a heterologous T cell, or an allogeneic T cell.

In a specific embodiment, the natural killer cell is an allogeneic NK cell.

The term "immune effector cell activity" refers to the ability of an immune effector cell to respond to antigen stimulation, proliferate to form immune effector substances, and carry out a characteristic immune response, for example, promote the killing of target cells or inhibit their growth or proliferation.

The terms "therapeutically effective amount" and "effective amount" are used interchangeably herein, and refer to the amount of a compound, preparation, substance, or composition effective to achieve a specific biological result, for example, but not limited to the amount or dose sufficient to promote T cell response. When referring to "immunologically effective amount", "anti-tumor effective amount", "tumor-suppressing effective amount" or "therapeutically effective amount", the precise administration amount of the immune effector cell or therapeutic agent according to the application may be determined by a physician under the consideration of the individual's age, weight, tumor size, degree of metastasis, and the condition of the patient (subject). An effective amount of immune effector cells refers to, but is not limited to, the number of immune effector cells capable of increasing, enhancing or prolonging the anti-tumor activity of immune effector cells; increasing the number of anti-tumor immune effector cells or activated immune effector cells; promoting IFN-γ secretion, tumor regression, tumor shrinkage, and tumor necrosis.

The terms "therapeutically effective amount" and "effective amount" are used interchangeably herein, and refer to the amount of a compound, preparation, substance, or composition effective to achieve a specific biological result, for example, but not limited to the amount or dose sufficient to promote T cell response. When referring to "immunologically effective amount", "anti-tumor effective amount", "tumor-suppressing effective amount" or "therapeutically effective amount", the precise administration amount of the immune effector cell or therapeutic agent according to the application may be determined by a physician under the consideration of the individual's age, weight, tumor size, degree of metastasis, and the condition of the patient (subject). An effective amount of immune effector cells refers to, but is not limited to, the number of immune effector cells capable of increasing, enhancing or prolonging the anti-tumor activity of immune effector cells; increasing the number of anti-tumor immune effector cells or activated immune effector cells; promoting IFN-γ secretion, tumor regression, tumor shrinkage, and tumor necrosis.

The term "no lymphocyte clearance" or "without performing lymphocyte clearance" means that the lymphocytes in the subject are not cleared, including but not limited to: without administrating lymphodepleting agent, whole body radiation therapy or their combination, or other means to cause lymphocyte clearance; however, after administratinglymphodepleting agent, whole body radiation therapy or their combination, or other means to cause lymphocyte clearance, when the lymphocyte clearance rate in the subject is less than 60%, we believe that although the treatment of lymphocyte clearance is performed, the effect of clearing lymphocytes has not been achieved, and it will equivalently fall into the scope of "no lymphocyte clearance" in the application.

As used herein, "chimeric receptor" refers to a fusion molecule formed by linking DNA fragments from different sources or cDNAs corresponding to proteins by gene recombination technology, including extracellular domain, transmembrane domain and intracellular domain. A chimeric receptor includes but is not limited to: chimeric antigen receptor (CAR), modified T cell (antigen) receptor (TCR), T cell fusion protein (TFP), and T cell antigen coupler (TAC).

As used herein, "chimeric antigen receptor" or "CAR" refers to a set of polypeptides, when they are in an immune effector cell, said cell is provided with specificity against a target cell (usually a cancer cell) and will generate intracellular signal. CAR usually comprises at least one extracellular antigen binding domain, transmembrane domain and cytoplasmic signaling domain (also referred to herein as "intracellular signaling domain"), and it comprises the functional signaling domain of stimulatory molecules and/or costimulatory molecules derived from the following definitions. In certain aspects, groups of polypeptides are adjacent to each other. The group of polypeptides comprises a dimerization switch that may couple polypeptides to each other in the presence of a dimerization molecule, for example, it may couple an antigen binding domain to an intracellular signaling domain. In one aspect, the stimulatory molecule is the ζ chain binding to the T cell receptor complex. In one aspect, the cytoplasmic signaling domain further comprises one or more functional signaling domains derived from at least one costimulatory molecule as defined below. In one aspect, the costimulatory molecule is selected from the costimulatory molecules described herein, such as 4-1BB (i.e., CD137), CD27, and/or CD28. In one aspect, the CAR comprises a chimeric fusion protein, which comprises an extracellular antigen binding domain, a transmembrane domain, and an intracellular signaling domain comprising a functional signaling domain derived from a stimulatory molecule. In one aspect, the CAR comprises a chimeric fusion protein, which comprises an extracellular antigen binding domain, a transmembrane domain, and intracellular signaling domains of a functional signaling domain derived from a costimulatory molecule and a functional signaling domain derived from a stimulatory molecule. In one aspect, the CAR comprises a chimeric fusion protein, which comprises an extracellular antigen binding domain, a transmembrane domain, and two functional signaling domains derived from one or more costimulatory molecules.

As used herein, "transmembrane domain" refers to a region in a protein sequence that spans the cell membrane, and may comprise one or more additional amino acids adjacent to the transmembrane region, for example, one or more amino acids associated with the extracellular region of the protein from which the transmembrane domain is derived (for example, 1, 2, 3, 4, 5, 6, 7, 8, 9, 10 up to 15 amino acids in the extracellular region) and/or one or more additional amino acids associated with the extracellular region of the protein from which the transmembrane domain is derived (e.g., 1, 2, 3, 4, 5, 6, 7, 8, 9, 10 up to 15 amino acids in the intracellular region). In one aspect, the transmembrane domain is a domain related to one of the other domains of the chimeric receptor, for example, in one embodiment, the transmembrane domain may be derived from the same protein from which a signaling domain, a costimulatory domain or a hinge domain is derived. In certain cases, a transmembrane domain may be selected or modified by amino acid substitution to prevent such a domain from binding to a transmembrane domain of the same or different surface membrane protein, for example, to minimize the interaction with other member of the receptor complex. In one aspect, the transmembrane domain is capable of homo-dimerizing with another chimeric receptor on the surface of the cell expressing the chimeric receptor. The transmembrane domain may be derived from natural or recombinant sources. When the source is natural, the domain may be derived from any membrane-bound protein or transmembrane protein. In one aspect, the transmembrane domain is capable of transmitting signals to the intracellular domain whenever the chimeric receptor binds to the target. The transmembrane domain specifically used in this application may comprise at least the following transmembrane domains: for example, α, β or ζ chains of a T-cell receptor, CD28, CD27, CD3ε, CD45, CD4, CD5, CD8, CD9, CD16, CD22, CD33, CD37, CD64, CD80, CD86, CD134, CD137, CD154. In certain embodiments, the transmembrane domain may include at least the following transmembrane regions: for example, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2Rβ, IL2Rγ, IL7Rα, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKG2D, NKG2C.

In certain cases, the transmembrane domain may be connected to the extracellular region of the CAR (for example, the antigen binding domain of the CAR) via a hinge (for example, a hinge from a human protein). Optionally, a short oligopeptide or polypeptide linker with 2-10 amino acids in length may form a bond between the transmembrane domain and the cytoplasmic region of the CAR. The glycine-serine dimer provides a particularly suitable linker.

As used herein, "intracellular domain" and "cytoplasmic domain" have the same meaning, including intracellular signaling domain. The intracellular signaling domain is generally responsible for the activation of at least one of the normal immune effector functions of immune cells into which a chimeric receptor has been introduced. The term "effector function" refers to a specialized function of a cell. The immune effector function of a T immune cell may be, for example, cytolytic activity or auxiliary activity, including secretion of cytokines. Therefore, the term "intracellular signaling domain" refers to a part of a protein that transduces an immune effector function signal and guides a cell to perform a specific function. Although the entire intracellular signaling domain may usually be used, in many cases it is not necessary to use the entire chain. In the case of using a truncated part of the intracellular signaling domain, such a truncated part may be used instead of the complete chain, as long as it transduces the immune effector function signal. Therefore, the term intracellular signaling domain is meant to include a truncated part of the intracellular signaling domain sufficient to transduce an immune effector function signal.

Specifically, the chimeric antigen receptor used in this application has: (i) an antibody or a fragment thereof specifically recognizing a tumor antigen, the transmembrane region of CD28 or CD8, the costimulatory signal domain of CD28, and CD3ζ; or (ii) an antibody or a fragment thereof specifically recognizing a tumor antigen, the transmembrane region of CD28 or CD8, the costimulatory signal domain of CD137, and CD3ζ; or (iii) an antibody or a fragment thereof specifically recognizing a tumor antigen, the transmembrane region of CD28 or CD8, the costimulatory signal domain of CD28, the costimulatory signal domain of CD137, and CD3ζ.

In specific examples, mouse CD8α signal peptide, mouse CD8α hinge region and transmembrane region, mouse CD28 intracellular domain, and mouse CD3ζ intracellular domain are used.

In specific examples, human CD8α signal peptide, human CD8α hinge region and transmembrane region, human CD28 transmembrane domain, human CD28 intracellular domain, and human CD3ζ intracellular domain may be used.

It is well known that, the signal generated by TCR alone is not sufficient to fully activate a T cell; and secondary and/or costimulatory signals are also required. Therefore, T cell activation may be said to be mediated by two different kinds of cytoplasmic signaling sequences: those that trigger antigen-dependent primary activation by TCR (primary intracellular signaling domains) and those that act in an antigen-independent manner to provide secondary or costimulatory signals (secondary cytoplasmic domains, such as costimulatory domains).

The term "stimulatory molecule" refers to a molecule expressed by an immune cell (for example, T cell, NK cell, B cell) for providing a cytoplasmic signaling sequence that modulates activation of the immune cell in at least some aspects of the signaling pathways for immune cells in a stimulating manner. In one aspect, the signal is a primary signal initiated by for example the binding of the TCR/CD3 complex and the MHC-antigen peptide complex, and it leads to mediation of T cell responses, including but not limited to: proliferation, activation, differentiation, and the like. The primary cytoplasmic signaling sequence (also referred to as "primary signaling domain") acting in a stimulating manner may contain a signaling motif called as an immune-receptor tyrosine-based activation motif or ITAM. Examples of ITAM-containing cytoplasmic signaling sequences specifically used in this application include, but are not limited to, those derived from: CD3ζ, common FcRy (FCER1G), FcyRIIa, FcRβ (FcEpsilon R1b), CD3γ, CD3δ, CD3ε, CD79a, CD79b, DAP10 and DAP12. The intracellular signaling domain in any one of CAR of the application comprises intracellular signaling sequences, such as the primary signaling sequence of CD3-ζ. In the specific CAR of the application, the primary signaling sequence of CD3-ζ is the equivalent residues from human or non-human species such as mouse, rodent, monkey, ape and the like.

The term "stimulatory molecule" refers to a molecule expressed by an immune cell (for example, T cell, NK cell, B cell) for providing a cytoplasmic signaling sequence that modulates activation of the immune cell in at least some aspects of the signaling pathways for immune cells in a stimulating manner. In one aspect, the signal is a primary signal initiated by for example the binding of the TCR/CD3 complex and the MHC-antigen peptide complex, and it leads to mediation of T cell responses, including but not limited to: proliferation, activation, differentiation, and the like. The primary cytoplasmic signaling sequence (also referred to as "primary signaling domain") acting in a stimulating manner may contain a signaling motif called as an immune-receptor tyrosine-based activation motif or ITAM. Examples of ITAM-containing cytoplasmic signaling sequences specifically used in this application include, but are not limited to, those derived from: CD3ζ, common FcRγ (FCER1G), FcγRIIa, FcRβ (FcEpsilon R1b), CD3γ, CD3δ, CD3ε , CD79a, CD79b, DAP10 and DAP12. The intracellular signaling domain in any one of CAR of the application comprises intracellular signaling sequences, such as the primary signaling sequence of CD3-ζ. In the specific CAR of the application, the primary signaling sequence of CD3-ζ is the equivalent residues from human or non-human species such as mouse, rodent, monkey, ape and the like.

The term "costimulatory molecule" refers to a homologous binding partner on T cells, which specifically binds to a costimulatory ligand, thereby mediating the costimulatory response of T cells, such as but not limited to proliferation. A co-stimulatory molecule is a cell surface molecule other than an antigen receptor or the ligand thereof, which promotes an effective immune response. The co-stimulatory molecules include but are not limited to: MHC class I molecules, BTLA and Toll ligand receptors, as well as OX40, CD27, CD28, CDS, ICAM-1, LFA-1 (CD11a/CD18), ICOS (CD278) and 4- 1BB (CD137). Further examples of such costimulatory molecules include: CDS, ICAM-1, GITR, BAFFR, HVEM (LIGHTR), SLAMF7, NKp80 (KLRF1), NKp44, NKp30, NKp46, CD160, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1 CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, and a ligand specifically binding to CD83.

The costimulatory intracellular signaling domain may be the intracellular part of a costimulatory molecule. Costimulatory molecules may be represented by the following protein families: TNF receptor protein, immunoglobulin-like protein, cytokine receptor, integrin, signaling lymphocyte activation molecule (SLAM protein), and NK cell receptor. Examples of such molecules include: CD27, CD28, 4-1BB (CD137), OX40, GITR, CD30, CD40, ICOS, BAFFR, HVEM, ICAM-1, antigen-1 (LFA-1) associated with lymphocyte function, CD2, CDS, CD7, CD287, LIGHT, NKG2C, NKG2D, SLAMF7, NKp80, NKp30, NKp44, NKp46, CD160, B7-H3, and a ligand specifically binding to CD83, etc.

The intracellular signaling domain may include all the intracellular part or all the natural intracellular signaling domain of the molecule, or a functional fragment or derivative thereof.

The term "4-1BB" refers to a member of the TNFR superfamily with the amino acid sequence provided in GenBank Accession No.AAA62478.2, or equivalent residues from non-human species such as mouse, rodent, monkey, ape and the like; and "4-1BB costimulatory domain" is defined as the amino acid residues 214-255 of GenBank Accession No.AAA62478.2, or equivalent residues from non-human species such as mouse, rodent, monkey, ape and the like. In one aspect, the "4-1BB costimulatory domain" is equivalent residues from human or from non-human species such as mouse, rodent, monkey, ape and the like.

The term "scFv" refers to a fusion protein comprising at least one antibody fragment comprising a light chain variable region and at least one antibody fragment comprising a heavy chain variable region, wherein the variable regions of the light chain and the heavy chain are contiguous (for example, via a synthetic linker such as a short flexible polypeptide linker), and may be expressedas a single-chain polypeptide, and wherein the scFv retains the specificity of the intact antibody from which it is derived. Unless specified, as used herein, the scFv may have the VL and VH variable regions in any order (for example, relative to the N-terminus and C-terminus of the polypeptide), and the scFv may include VL-linker-VH, or may include VH-linker-VL.

The term "antibody" refers to a protein or polypeptide sequence derived from an immunoglobulin molecule specifically binding to an antigen. An antibody may be polyclonal or monoclonal, multi-chain or single-chain, or a whole immunoglobulin, and may be derived from a natural source or recombinant source. The antibody may be a tetramer of immunoglobulin molecules.

The term "antibody fragment" refers to at least a portion of an antibody that retains the ability to specifically interact with an epitope of an antigen (eg., through binding, steric hindrance, stabilization/destabilization, spatial distribution). Examples of antibody fragments include, but are not limited to: Fab, Fab', F(ab')2, Fv fragment, scFv, disulfide-linked Fvs (sdFv), Fd fragment composed of VH and CH1 domains, linear antibody, single domain antibody (such as sdAb), camelid VHH domain, multispecific antibody formed by antibody fragments (e.g., a bivalent fragment comprising two Fab fragments connected by disulfide bonds in the hinge region), and isolated CDRs of an antibody or other epitope binding fragments. The term "scFv" refers to a fusion protein comprising at least one antibody fragment including a variable region of a light chain and at least one antibody fragment including a variable region of a heavy chain, wherein the light chain and the variable region of the heavy chain are contiguous (for example, via a synthetic linker such as a short flexible polypeptide linker), and may be expressed as a single-chain polypeptide, and wherein the scFv retains the specificity of the intact antibody from which it is derived. Unless specified, as used herein, the scFv may have the VL and VH variable regions in any order (for example, relative to the N-terminus and C-terminus of the polypeptide), and the scFv may include VL-linker-VH, or May include VH-linker-VL.

The term "antibody heavy chain" refers to the larger of the two polypeptide chains which is present in an antibody molecule in its naturally occurring configuration and usually determines the type of antibody to which it belongs.

The term "antibody light chain" refers to the smaller of the two polypeptide chains which is present in an antibody molecule in its naturally occurring configuration. Kappa (k) and lambda (1) light chains refer to isotypes of the two main antibody light chains.

The term "recombinant antibody" refers to an antibody produced by recombinant DNA technology, such as, for example, an antibody expressed by a phage or yeast expression system. The term should also be interpreted as referring to an antibody that has been produced by synthesizing a DNA molecule encoding the antibody (and wherein the DNA molecule expresses the antibody protein) or the amino acid sequence of the specified antibody, wherein the DNA or amino acid sequence has been obtained by recombinant DNA technology or an amino acid sequence technology available and well-known in the art.

The term "antigen" or "Ag" refers to a molecule that causes an immune response. The immune response may involve the production of antibodies or the activation of cells with specific immunity or both. Those skilled in the art should understand that, any macromolecule including virtually all proteins or peptides may serve as an antigen. In addition, an antigen may be derived from recombinant or genomic DNA. When the term is used herein, those skilled in the art should understand that, any DNA comprising a nucleotide sequence or part of a nucleotide sequence that encodes a protein causing an immune response may encode an "antigen." In addition, those skilled in the art should understand that, an antigen need not be encoded only by the full-length nucleotide sequence of a gene. It is obvious that this application includes, but is not limited to, utilizing partial nucleotide sequences of more than one gene, and these nucleotide sequences are arranged in different combinations to encode polypeptides that elicit a desired immune response. Those skilled in the art should understand that, an antigen does not need to be encoded by a "gene" at all. It is obvious that an antigen may be produced synthetically, or it may be derived from a biological sample, or it may be a macromolecule other than a polypeptide. Such biological samples may include, but are not limited to: tissue samples, tumor samples, cells or fluids having other biological components.

The term "recombinant antibody" refers to an antibody produced by recombinant DNA technology, such as, for example, an antibody expressed by a phage or yeast expression system. The term should also be interpreted as referring to an antibody that has been produced by synthesizing a DNA molecule encoding the antibody (and wherein the DNA molecule expresses the antibody protein) or the amino acid sequence of the specified antibody, wherein the DNA or amino acid sequence has been obtained by recombinant DNA technology or an amino acid sequence technology available and well-known in the art.

The term "antigen" or "Ag" refers to a molecule that causes an immune response. The immune response may involve the production of antibodies or the activation of cells with specific immunity or both. Those skilled in the art should understand that, any macromolecule including virtually all proteins or peptides may serve as an antigen. In addition, an antigen may be derived from recombinant or genomic DNA. When the term is used herein, those skilled in the art should understand that, any DNA comprising a nucleotide sequence or part of a nucleotide sequence that encodes a protein causing an immune response may encode an "antigen." In addition, those skilled in the art should understand that, an antigen need not be encoded only by the full-length nucleotide sequence of a gene. It is obvious that this application includes, but is not limited to, utilizing partial nucleotide sequences of more than one gene, and these nucleotide sequences are arranged in different combinations to encode polypeptides that elicit a desired immune response. Those skilled in the art should understand that, an antigen does not need to be encoded by a "gene" at all. It is obvious that an antigen may be produced synthetically, or it may be derived from a biological sample, or it may be a macromolecule other than a polypeptide. Such biological samples may include, but are not limited to: tissue samples, tumor samples, cells or fluids having other biological components.

"Tumor antigen" refers to an antigen that is newly emerged or overexpressed during the occurrence and development of hyperproliferative diseases. In certain aspects, the hyperproliferative disorder of the application refers to tumors. The tumor antigen of the application is EGFR or EGFRvIII. EGFR is overexpressed or mutated in many tumors. So far, antibodies against the EGFR287-302 epitope are considered to be able to recognize the over-expression of EGFR, EGFRvIII, and de4EGFR on the tumor surface.

In at least one specific embodiment of the application, an antibody specifically recognizing a tumor antigen is an antibody targeting EGFR or EGFRvIII. In at least one specific embodiment of the application, the amino acid sequence of an antibody specifically recognizing a tumor antigen is shown in SEQ ID NO: 11, or the amino acid sequence of an antibody specifically recognizing a tumor antigen is shown in SEQ ID NO: 12, or the amino acid sequence of an antibody specifically recognizing a tumor antigen is shown in SEQ ID NO: 13, or the amino acid sequence of an antibody specifically recognizing a tumor antigen is shown in SEQ ID NO: 14, or the amino acid sequence of an antibody specifically recognizing a tumor antigen is shown in SEQ ID NO: 15, or the amino acid sequence of an antibody specifically recognizing a tumor antigen is shown in SEQ ID NO: 16, or the amino acid sequence of an antibody specifically recognizing a tumor antigen is shown in SEQ ID NO: 16, or the amino acid sequence of an antibody specifically recognizing a tumor antigen is shown in SEQ ID NO: 17, or the amino acid sequence of an antibody specifically recognizing a tumor antigen is shown in SEQ ID NO: 18, or the amino acid sequence of an antibody specifically recognizing a tumor antigen is shown in SEQ ID NO: 19, or the amino acid sequence of an antibody specifically recognizing a tumor antigen is shown in SEQ ID NO: 20, or the amino acid sequence of an antibody specifically recognizing a tumor antigen is shown in SEQ ID NO: 21, or the amino acid sequence of an antibody specifically recognizing a tumor antigen is shown in SEQ ID NO: 22, or the amino acid sequence of an antibody specifically recognizing a tumor antigen is shown in SEQ ID NO: 23, or the amino acid sequence of an antibody specifically recognizing a tumor antigen is shown in SEQ ID NO: 24, or the amino acid sequence of an antibody specifically recognizing a tumor antigen is shown in SEQ ID NO: 25, or the amino acid sequence of an antibody specifically recognizing a tumor antigen is shown in SEQ ID NO: 26, or the amino acid sequence of an antibody specifically recognizing a tumor antigen is shown in SEQ ID NO: 27, or the amino acid sequence of an antibody specifically recognizing a tumor antigen is shown in SEQ ID NO: 28, or the amino acid sequence of an antibody specifically recognizing a tumor antigen is shown in SEQ ID NO: 29.

In at least one specific embodiment of the application, the amino acid sequence of a chimeric antigen receptor according to the present application is shown in SEQ ID NO: 30, or shown in SEQ ID NO: 31, or shown in SEQ ID NO: 32, or shown in SEQ ID NO: 33, or shown in SEQ ID NO: 34, or shown in SEQ ID NO: 35, or shown in SEQ ID NO: 36, or shown in SEQ ID NO: 37, or shown in SEQ ID NO: 38, or shown in SEQ ID NO: 39, or shown in SEQ ID NO: 40, or shown in SEQ ID NO: 41, or shown in SEQ ID NO: 42, or shown in SEQ ID NO: 43, or shown in SEQ ID NO: 44, or shown in SEQ ID NO: 45, or shown in SEQ ID NO: 46, or shown in SEQ ID NO: 47, or shown in SEQ ID NO: 48, or shown in SEQ ID NO: 49, or shown in SEQ ID NO: 50, or shown in SEQ ID NO: 51, or shown in SEQ ID NO: 55, or shown in SEQ ID NO: 56, or shown in SEQ ID NO: 57, or shown in SEQ ID NO: 58, or shown in SEQ ID NO: 59, or shown in SEQ ID NO: 60, or shown in SEQ ID NO: 61, or shown in SEQ ID NO: 62, or shown in SEQ ID NO: 63, or shown in SEQ ID NO: 64, or shown in SEQ ID NO: 65, or shown in SEQ ID NO: 66, or shown in SEQ ID NO: 67, or shown in SEQ ID NO: 68, or shown in SEQ ID NO: 69, or shown in SEQ ID NO: 70, or shown in SEQ ID NO: 71, or shown in SEQ ID NO: 72, or shown in SEQ ID NO: 73, or shown in SEQ ID NO: 74, or shown in SEQ ID NO: 75, or shown in SEQ ID NO: 76, or shown in SEQ ID NO: 77, or shown in SEQ ID NO: 78, or shown in SEQ ID NO: 79, or shown in SEQ ID NO: 80, or shown in SEQ ID NO: 81, or shown in SEQ ID NO: 82, or shown in SEQ ID NO: 83, or shown in SEQ ID NO: 84, or shown in SEQ ID NO: 85, or shown in SEQ ID NO: 86, or as shown in SEQ ID NO: 87.

The term "cancer tumor" refers to a broad category of disorders characterized by hyperproliferative cell growth *in vitro* (e.g., transformed cells) or *in vivo.* Conditions that may be treated or prevented by the method according to the present invention include, for example, various neoplasms including benign or malignant tumors, various hyperplasias, and the like. The method according to the application may achieve the inhibition and/or reversal of the undesirable hyperproliferative cell growth involved in such conditions. Specific examples of cancer include, but are not limited to: breast cancer, prostate cancer, leukemia, lymphoma, nasopharyngeal cancer, blood cancer, colon cancer, rectal cancer, renal cell carcinoma, liver cancer, non-small cell carcinoma of the lung, small intestine cancer, esophageal cancer, melanoma, bone cancer, pancreatic cancer, skin cancer, head and neck cancer, skin or intraocular malignant melanoma, uterine cancer, ovarian cancer, rectal cancer, anal cancer, stomach cancer, testicular cancer, uterine cancer, fallopian tube cancer, endometrial cancer, cervical cancer, vaginal cancer, vulva cancer, Hodgkin's disease, non-Hodgkin's lymphoma, endocrine system cancer, thyroid cancer, parathyroid cancer, adrenal cancer, soft tissue sarcoma, urethral cancer, penis cancer, childhood solid tumor, bladder cancer, renal or ureteral cancer, renal pelvis cancer, central nervous system (CNS) tumors, primary CNS lymphoma, tumor angiogenesis, vascular carcinoma, spine tumor, brainstem glioma, pituitary gland tumor, Kaposi's sarcoma, epidermoid carcinoma, squamous cell carcinoma, T-cell lymphoma, environmentally-induced cancer, a combination and the metastatic foci thereof.

The term "transfected", or "transformed", or "transduced" refers to the process by which exogenous nucleic acid is transferred or introduced into a host cell. A "transfected", or "transformed", or "transduced" cell is a cell being transfected, transformed, or transduced with exogenous nucleic acid. The cells include primary subject cells and their progeny.

The term "specifically binds" refers to an antibody or ligand that recognizes and binds a protein of a binding partner (such as a tumor antigen) present in a sample, but the antibody or ligand does not substantially recognize or bind to other molecules in the sample.

The term "refractory" refers to a disease, such as a tumor, which does not respond to treatment. In an embodiment, a refractory tumor may be resistant to the treatment before or at the beginning of the treatment. In other embodiments, a refractory tumor may become resistant during treatment. Refractory tumors are also called resistant tumors. In this application, refractory tumors include, but are not limited to, tumors that are not sensitive to radiotherapy, relapse after radiotherapy, are not sensitive to chemotherapy, relapse after chemotherapy, are not sensitive to CAR-T treatment, or relapse after the treatment. Refractory or recurrent malignant tumors may be treated by the treatment regimens described herein.

As used herein, "relapsed" refers to the return of the signs and symptoms of a disease (e.g. cancer) or a disease such as cancer during a period of improvement, for example, after a therapy, such as a previous treatment of cancer therapy.

The terms "individual" and "subject" have the same meaning herein, and may be humans and animals from other species.

The term "enhancement" refers to allowing a subject or tumor cell to improve its ability to respond to the treatment disclosed herein. For example, an enhanced response may include 5%, 10%, 15%, 20%, 25%, 30%, 35%, 40%, 45%, 50%, 55%, 60%, 65%, 70%, 75%, 80%, 85%, 90%, 95% or 98% or more enhancement of the responsiveness. As used herein, "enhancement" may also refer to increasing the number of subjects responding to treatment, such as immune effector cell therapy. For example, an enhanced response may refer to the total percentage of subjects responding to treatment, wherein the percentages are 5%, 10%, 15%, 20%, 25%, 30%, 35%, 40%, 45%, 50%, 55 %, 60%, 65%, 70%, 75%, 80%, 85%, 90%, 95%, or 98%, or more.

In one aspect, treatment is determined by the followings: clinical outcome; increase, enhancement or extension of the anti-tumor activity of T cells; promotion of the secretion of IFN-$\gamma$, TNFa, or a combination thereof by the increase in the number of anti-tumor T cells or activated T cells as compared with the number before treatment. In another aspect, the clinical outcome is tumor regression; tumor shrinkage; tumor necrosis; anti-tumor response through the immune system; tumor enlargement, recurrence or spread, or a combination thereof. In an additional aspect, the therapeutic effect is predicted by the presence of T cells, the presence of genetic markers indicative of T cell inflammation, promotion of IFN-$\gamma$, TNFa secretion, or a combination thereof.

The immune effector cells as disclosed herein may be administered to an individual by various routes, including, for example, orally or parenterally, such as intravenous, intramuscular, subcutaneous, intraorbital, intrasaccular, intraperitoneal, intrarectal, intracisternal, intratumoral, intranasally, intradermally, or passive or promoted absorption through the skin by respectively using, for example, skin patches or transdermal iontophoresis.

The total amount of agent to be administered in practicing the method according to the present application may be administered to a subject as a single dose as a bolus or by infusion over a relatively short period of time; or may be administered using a graded treatment regimen, wherein multiple doses are administered over an extended period of time. Those skilled in the art will know that, the amount of the composition for treating a pathological condition in a subject depends on many factors, including the age and general health of the subject, as well as the route of administration and the number of treatments to be administered. In consideration of these factors, the technician will adjust the specific dosage as needed. Generally speaking, the phase I and phase II clinical trials are initially used to determine the formulation of the composition and the route and frequency of administration.

Scope: throughout this disclosure, various aspects of this application may be presented in a range format. It should be understood that, the description of range format is only for convenience and brevity, and should not be regarded as an unchangeable limitation on the scope of the application. Therefore, the description of a range should be considered as specifically disclosing all possible subranges and individual values within that range. For example, a description of a range such as 1-6 should be considered to specifically disclose the subranges such as 1-3, 1-4, 1-5, 2-4, 2-6, 3-6, etc., and the individual values within the range, for example, 2, 2.7, 3, 4, 5, 5.3, and 6. As another example, a range such as 95-99% identity includes a range with 95%, 96%, 97%, 98%, or 99% identity, and includes sub-ranges such as 96-99%, 96-98%, 96-97%, 97-99%, 97-98% and 98-99% identity. This applies regardless of the width of the range.

Based on the present disclosure, those skilled in the art should understand that, many changes or modifications may be made in the disclosed specific embodiments and still obtain the same or similar results without departing from the spirit and scope of the present invention. The scope of the present invention is not limited to the specific embodiments described herein (which are only intended to be an illustration of various aspects of the present invention), and the functionally equivalent methods and components are within the scope of the present invention.

The present invention will be further explained below in conjunction with particular examples. It should be understood that, these examples are only used to illustrate the present invention and not to limit the scope of the present invention. The following examples do not specify the experimental methods of specific conditions, usually conventional conditions such as those described in J. Edited by Sambrook et al., "Molecular Cloning Experiment Guide (Third Edition)" (Science Press, 2002), or those recommended by the manufacturer are adopted.

Exemplary antigen receptors of the present application, including CAR, and methods for engineering and introducing receptors into cells, may refer to, those described in, for example, Chinese patent application publication Nos. CN107058354A, CN107460201A, CN105194661A, CN105315375A, CN105713881A, CN106146666A, CN106519037A, CN106554414A, CN105331585A, CN106397593A, CN106467573A, and CN104140974A; and International patent application publications Nos. WO2017186121A1, WO2018006882A1, WO2015172339A8, and WO2018018958A1.

**Example 1: Construction of CAR-T Cells**

This example uses a second-generation CAR targeting EGFRvIII. For the needs of later animal experiments, the transmembrane domain and intracellular domain of the CAR are constructed by using mouse gene sequences.

The coding sequence of mouse CD8$\alpha$ signal peptide (SEQ ID NO: 1), the coding sequence of anti-EGFRvIII monoclonal antibody (SEQ ID NO: 2), the coding sequence of mouse CD8$\alpha$ hinge region and transmembrane region (SEQ ID NO: 3), the coding sequence of the mouse CD28 intracellular domain (SEQ ID NO: 4), and the coding sequence of the mouse CD3$\zeta$ intracellular domain (SEQ ID NO: 5) are connected successively; the 806-mCD28z gene fragment is obtained by *in vitro* gene synthesis; and the IRES-GFP fragment in the retroviral vector MSCV-IRES-GFP (purchased from Addgene) is replaced with Mlu I and Sal I double restriction sites to obtain the recombinant vector MSCV-806-mCD28z (Fig. 1A). In this Example 1, the amino acid sequence of the antibody encoded by SEQ ID NO: 2 is shown in SEQ ID NO: 12, and the amino acid sequence of the chimeric antigen receptor of the CAR cell constructed in Example 1 is shown in SEQ ID NO: 30.

The recombinant vector MSCV-806-mCD28z is used to infect 293T cells (purchased from ATCC) to obtain the packaged retrovirus. The infection method is a conventional infection method in the preparation process of T cells expressing chimeric antigen receptors in the art.

The spleen of C57BL/6 mice is ground to obtain lymphocytes, after treating with CD3+ mouse T-cell negative screening kit, the resulting mouse CD3+ T lymphocytes are added to Dynabeads Mouse T-activator CD3/CD28 magnetic beads at a volume ratio of 1:1 for activation and stimulation, and put into the cell incubator, wherein the medium is RPMI1640 complete medium (10% FBS + 50$\mu$M $\beta$-mercaptoethanol + 100U/mL IL-2 + 1ng/mL IL-7).

CD3+ T lymphocytes from mouse spleen that have been activated for 24 hours are inoculated into a 24-well plate coated with Retronectin (5 $\mu$g/mL), after infecting with retrovirus for 24 hours, changing for fresh medium to obtain mouse 806-mCD28z-CAR-T cells. The positive rate of CAR-T cell infection is detected by flow cytometry, and the positive rate of 806-mCD28z-CAR-T cell infection is 70.7% (Fig. 1B).

**Example 2: Detecting the Toxicity of Olaparib to Breast Cancer Cells and CAR-T Cells by CCK8 Assay**

Since the EGFR287-302 epitope is only exposed in EGFRvIII or tumors overexpressing EGFR, this epitope is hidden in normal tissues (Gan HK et al., Targeting of a conformationally exposed, tumor-specific epitope of EGFR as a strategy for cancer therapy. Cancer Res, 2012, 72(12):2924-2930), conventional methods in molecular biology are used to establish E0771, 4T1 mouse breast cancer cell models (E0771-EGFRvIII, 4T1-EGFRvIII) that overexpress mouse EGFR inserted with amino acid epitope at positions 287-302 of human EGFR. Subsequent research is performed to screen EGFRvIII target-positive cells by cell flow cytometry (Fig. 2A). E0771, 4T1 cells are purchased from the American Type Culture Collection (ATCC).

As an effective inhibitor of PARP1, olaparib may inhibit PARP1 to participate in DNA damage repair process.

In mouse breast cancer cell lines and mouse T cells and CART cells, different levels of PARP1 expression are detected (Fig. 2B). Therefore, CCK8 proliferation assay is required to detect the effect of olaparib on proliferation of tumor cells and CART cells.

806-m28Z CAR-T cells in Example 1 are taken to spread in a 96-well plate with $1\times10^4$ cells per well and 100 μl medium. Breast cancer cells E0771-EGFRvIII and 4T1-EGFRvIII are taken to spread in a 96-well plate with $1\times10^4$ cells per well and 100 μl medium. Different concentrations of olaparib are taken to add to the cells to make 10 concentration gradients (i.e., the treatment concentrations of olaparib are respectively 50μM, 20μM, 10μM, 5μM, 2.5μM, 1μM, 0.1μM, 0.01μM, 0.001μM, 0μg/ml). After 48 hours of drug treatment for cells of different groups, 10μl of CCK8 substrate color reagent (Dojindo) is added to each well; after incubating for 1 hour at 37°C, the absorbance at 450nm is measured by a microplate reader, calculating the cell viability respectively.

The formula for calculating the cell viability is as follows:

$$\text{Cell viability (\%)} = [\text{A (adding drug)-A (blank)}]/[\text{A (without adding drug)-A (blank)}]$$

The results are shown in Fig. 2C. After adding different concentrations of olaparib to treat 806-mCD28z-CAR-T cells, 4T1-EGFRvIII cells and E0771-ERVIII cells, the growth of 806-mCD28z-CAR-T cells and E0771-EGFRVIII cells is inhibited in a certain degree, wherein the proliferation of 806-mCD28z-CAR-T cells is inhibited to a certain extent under the treatment of 2.5μM and higher concentration of olaparib, with an inhibition rate of nearly 80%; and the proliferation of E0771-EGFRVIII cell line is inhibited under the treatment of 1μM and higher concentration of olaparib, with an inhibition rate of 65%-90%; the proliferation of 4T1-EGFRvIII cell line is inhibited under the treatment of 1μM and higher concentration of olaparib, with an inhibition rate of 30-40% (Fig. 2C). It shows that the effective concentrations of olaparib on the growth of 806-mCD28z-CAR-T cells and E0771-EGFRVIII, 4T1-EGFRvIII cells are different, and it also suggests that the appropriate drug concentration of olaparib should be selected for subsequent research. Under the treatment of 1μM olaparib, although the proliferation of E0771-EGFRVIII and 4T1-EGFRvIII cells is inhibited, the proliferation of 806-mCD28z-CAR-T cells is not affected; however, under the treatment of 5μM olaparib, the proliferation of E0771-EGFRVIII, 4T1-EGFRvIII cells and 806-mCD28z-CAR-T cells are inhibited. Accordingly, two concentrations of olaparib are selected to demonstrate the effect of a combined treatment of breast cancer by olaparib and CAR-T at different concentrations and under different cell response states. Therefore, in subsequent experiments, 1μM and 5μM olaparib are selected for research.

**Example 3: Detecting the Killing Effect of Olaparib Pretreatment plus CAR-T Treatment, or Combination Administration of Olaparib and CAR-T on Tumors by Cytotoxicity Assay; and Detecting the Effect of Olaparib on the Secretion of Cytokine Granzyme B, IL-2, IFN-γ by ELISA Assay**

The experimental groups for incubating with cells that are negative for the antigen target comprises the following two groups: UTD group, and 806-mCD28z-CAR-T group (or denoted as 806-28Z). The untreated mouse T cells and 806-mCD28z-CAR T cells in Example 1 are respectively taken to incubate with E0771 or 4T1 cells at a ratio of 1:3, 1:1, and 3:1; 16 hours later, the secretion of LDH in the supernatant is detected by Cytox 96 Non-Radioactive Cytotoxicity Assay, then calculating the killing toxicity of 806-mCD28z CAR T cells to tumor cells. As shown in Fig. 3A, 806-mCD28z-CAR T cells have no killing effect on antigen-negative target cells.

The experimental groups for incubating with cells that are positive for the antigen target comprises the following 4 groups: UTD group, 806-mCD28z-CAR-T group (or denoted as 806-28Z), olaparib pretreatment group, and olaparib combined treatment group;

wherein the UTD group is untreated mouse T cells;
the olaparib pretreatment group comprises: UTD+1μM olaparib pretreatment group (or denoted as UTD+Olaparib 1μM pretreatment), UTD+5μM olaparib pretreatment group (or denoted as UTD+Olaparib 5μM pretreatment), 806-mCD28z-CAR-T+1μM olaparib pretreatment group (or denoted as 806-28Z+Olaparib 1μM pretreatment), 806-mCD28z-CAR-T+5μM olaparib pretreatment group (or denoted as 806-28Z+Olaparib 5μM pretreatment);
the olaparib combined treatment group comprises: UTD+1μM olaparib combined treatment group (or denoted as UTD+Olaparib 1μM), UTD+5μM olaparib combined treatment group (or denoted as UTD+Olaparib 5μM), 806-mCD28z-CAR-T+1μM olaparib combined treatment group (or denoted as 806-28Z+Olaparib 1μM), 806-mCD28z-CAR-T+5μM olaparib combined treatment group (or denoted as 806-28Z+Olaparib 5μM).

Olaparib pretreatment group: different concentrations of olaparib (1μM, 5μM) are added to the medium for culturing E0771-EGFRVIII or 4T1-EGFRvIII tumor cells respectively, culturing at 37°C for 24h, and then the cells are treated with trypsin and resuspended in fresh medium. The untreated mouse T cells and 806-mCD28z-CAR T cells in Example 1 are respectively taken to incubate with E0771-EGFRvIII or 4T1-EGFRvIII cells pretreated with different concentrations of olaparib at a ratio of 1:3, 1:1, and 3:1; 16 hours later, the secretion of LDH in the supernatant is detected by Cytox 96 Non-Radioactive Cytotoxicity Assay, then calculating the killing toxicity of 806-mCD28z CAR T cells to tumor cells pretreated with different concentrations of olaparib. For specific detection steps and calculation methods, see the instructions of Promaga Cytox 96 Non-Radioactive Cytotoxicity Assay (Promaga, REF: G1782); at the same time, the secretions of cytokines Granzyme B, IFN-γ, and IL-2 in the supernatant are detected by ELISA kit (Lianke Biotech Co. Ltd., CAT: 1822), for specific experimental procedures, referring to instructions of MouseGranzyme B, IFN-γ, IL-2 ELISA kit from Lianke Biotech Co. Ltd.

Olaparib combined treatment group: different concentrations of olaparib (1μM, 5μM) are added to the medium for culturing E0771-EGFRVIII or 4T1-EGFRvIII tumor cells respectively, culturing at 37°C for 24h, and then the cells are treated with trypsin and resuspended in fresh medium. The untreated mouse T cells and 806-mCD28z-CAR T cells in Example 1 are respectively taken to incubate with E0771-EGFRvIII or 4T1-EGFRvIII cells treated with different concentrations of olaparib at a ratio of 1:3, 1:1, and 3:1; after corresponding concentrations of olaparib (1μM, 5μM) are added to treat the cells continuously for 16 hours, the secretion of LDH in the supernatant is detected by Cytox 96 Non-Radioactive Cytotoxicity Assay, then calculating the killing toxicity of 806-mCD28z CAR T cells to tumor cells pretreated with different concentrations of olaparib. For specific detection steps and calculation methods, see the instructions of Promaga Cytox 96 Non-Radioactive Cytotoxicity Assay (Promaga, REF: G1782); at the same time, the secretions of cytokines Granzyme B, IFN-γ, and IL-2 in the supernatant are detected by ELISA kit (Lianke Biotech Co. Ltd., CAT: 1822), for specific experimental procedures, referring to instructions of MouseGranzyme B, IFN-γ, IL-2 ELISA kit from Lianke Biotech Co. Ltd.

As shown in Fig. 3A, the killing effect of CAR-T cells on E0771-EGFRvIII and 4T1-EGFRvIII cells may be significantly improved by using olaparib, especially in the 5μM olaparib pretreatment group, the promotion of the killing effect is the most significant and is better than that of the 5μM olaparib combined treatment group, suggesting that higher concentrations of olaparib may inhibit the growth of CAR-T to a certain extent. Compared with the CAR-T single treatment group, the 5μM olaparib combined treatment group still has a higher synergistic lethal effect.

As shown in Fig. 3B, compared with the CAR-T single treatment group, in the olaparib pretreatment group and the olaparib combined treatment group, the levels of IFN-γ in the supernatant of co-cultured E0771-EGFRvIII cells and CART cells are significantly increased (Unpaired test, * means $p<0.05$, ** means $p<0.01$, ** means $p<0.001$, ns means no statistical difference); the levels of Granzyme B and IL-2 do not increase significantly. This experiment further confirms that olaparib may significantly improve the killing ability of CAR-T cells. The level of cytokine in the supernatant of the co-cultured 4T1-EGFRvIII cells and CAR-T cells is not increased, indicating that the enhanced killing effect comes from the combined effect of olaparib and CAR-T cells.

**Example 4: The Effect of Olaparib on the Proliferation and the Expression of Exhaustion-related Proteins of CAR-T Cells Co-cultured with Mouse Breast Cancer Cells**

The experimental groups are as follows: 806-mCD28z-CAR-T group (or denoted as 806-28Z), olaparib pretreatment group, and olaparib combined treatment group;

The olaparib pretreatment group comprises: 806-mCD28z-CAR-T+1μM olaparib pretreatment group (or denoted as 806-28Z+Olaparib 1μM pretreatment), 806-mCD28z-CAR-T+5μM olaparib pretreatment group (or denoted as 806-28Z+Olaparib 5μM pretreatment);

Olaparib combined treatment group comprises: 806-mCD28z-CAR-T+1μM olaparib combined treatment group (or denoted as 806-28Z+Olaparib 1μM), 806-mCD28z-CAR-T+5μM olaparib combined treatment group (or denoted as 806-28Z+Olaparib 5μM).

Before the co-incubation experiment, 806-mCD28z-CAR-T cells need to be stained with CellTrace dye. The specific operation process is as follows: 1μL of CellTrace dye is added to 1mL of 1640 medium per $10^6$ cells, treating them in the dark at 37°C for 20 minutes; and 20 minutes later, complete medium is added to treat them for 5 minutes, then centrifuging and discarding the supernatant.

Olaparib pretreatment group: different concentrations of olaparib (1μM, 5μM) are respectively added to a medium for culturing E0771-EGFRvIII or 4T1-EGFRvIII tumor cells, after culturing at 37°C for 24 hours, the cells are treated with trypsin and resuspended in fresh medium. 806-mCD28z-CAR T cells in Example 1 are taken to co-incubate with E0771-EGFRvIII or 4T1-EGFRvIII cells pretreated with different concentrations of olaparib at a ratio of 1:1; 16 hours later, the proliferation of CAR-T cells and the expression levels of the T cell exhaustion-related proteins PD1, LAG3 and TIM3 on the surface of CAR-T cells are detected by flow cytometry.

Olaparib combined treatment group: different concentrations of olaparib (1μM, 5μM) are respectively added to a medium for culturing E0771-EGFRvIII or 4T1-EGFRvIII tumor cells, after culturing at 37°C for 24 hours, the cells are treated with trypsin and resuspended in fresh medium. 806-mCD28z-CAR T cells in Example 1 are taken to co-incubate with E0771-EGFRvIII or 4T1-EGFRvIII cells pretreated with different concentrations of olaparib at a ratio of 1:1; after corresponding concentrations of olaparib (1μM, 5μM) are respectively added to the medium for 16 hours, the proliferation of CAR-T cells and the expression levels of the T cell exhaustion-related proteins PD1, LAG3 and TIM3 on the surface of CAR-T cells are detected by flow cytometry.

As shown in Fig. 4A, after the CART cells of the olaparib pretreatment group are incubated with target cells, the proliferation of CART cells is not significantly affected; while the proliferation of CART cells in the olaparib combined treatment group is slightly inhibited due to the presence of olaparib in the co-culture environment, but it is not statistically significant.

As shown in Fig. 4B, after the CAR-T cells of the olaparib pretreatment group are incubated with target cells, the expression levels of the exhaustion-related proteins PD1, LAG3 and TIM3 on the surface of CAR-T cells do not change significantly; in olaparib combined treatment group, the expression levels of the exhaustion-related proteins PD1, LAG3 and TIM3 on the surface of CAR-T cells do not change significantly, suggesting that olaparib treatment does not lead to exhaustion of CAR-T cells and thus does not affect the killing effect of effector T cells.

**Example 5: Combined Treatment of Mouse E0771-EGFRvIII Orthotopic Breast Cancer Xenografts by Olaparib and CAR-T**

(1) Establishment of mouse orthotopic breast cancer model and respective treatment in groups:
E0771-EGFRvIII cells in the logarithmic growth phase and growing well are collected, inoculating $5\times10^5$ target cells at right side position of the third pair of accessory breasts of C57BL/6 mice (mice with normal immune system), and the inoculating day is recorded as the first day (i.e., Day1).

On the 10th day after tumor inoculation (i.e. Day 10), mouse T cells are taken and 806-mCD28z-CAR-T cell line is constructed as described in step 1 of this example.

On the 14th day after tumor inoculation (i.e., Day 14), the volume of the tumor reaches about 150mm$^3$, and the mice are divided in groups:
The formula of olaparib stock solution: olaparib is added with 4% DMSO + 30% PEG300 + 66% ddH$_2$O in sequence; a solution of 5mg/mL is used as the stock solution.

Olaparib is intraperitoneally administrated, the injection amount of each mouse is 50mg/kg, and the weight of the mouse is about 20g, so the injection for each mouse is 200μL; the control group is given 200μL solvent treatment (4% DMSO + 30% PEG 300 + 66% ddH$_2$O).

The mice are divided into 6 groups, 6 mice for each group. The specific *in vivo* experimental procedure is shown in Fig. 5A:
UTD group: 200 μL solvent/day is administrated by intraperitoneal infusion on Day 14, once a day for 5 consecutive days; after 2 days of interval, 200 μL solvent/day is administrated again by intraperitoneal infusion, once a day for 5 consecutive days; $5\times10^6$ mouse T cells without infected by virus are administrated by tail vein infusion on Day 21.

Olaparib (50mg/kg) group: 50mg/kg olaparib/day is administrated by intraperitoneal infusion on Day 14, once a day for 5 consecutive days; after 2 days of interval, 50mg/kg olaparib/day is administrated again by intraperitoneal infusion, once a day for 5 consecutive days; $5\times10^6$ mouse T cells without infected by virus are administrated by tail vein infusion on Day 21.

CAR-T ($2\times10^6$) group: 200 μL solvent/day is administrated by intraperitoneal infusion on Day 14, once a day for 5 consecutive days; after 2 days of interval, 200 μL solvent/day is administrated again by intraperitoneal infusion, once a day for 5 consecutive days; $2\times10^6$ 806-mCD28z-CAR-T cells are administrated by tail vein infusion on Day 21.

Olaparib (50mg/kg)+CAR-T ($2\times10^6$) group: 50mg/kg olaparib/day is administrated by intraperitoneal infusion on Day 14, once a day for 5 consecutive days; after 2 days of interval, 50mg/kg olaparib/day is administrated again by intraperitoneal infusion, once a day for 5 consecutive days; $2\times10^6$ 806-mCD28z-CAR-T cells are administrated by tail vein infusion on Day 21.

CAR-T ($5\times10^6$) group: 200 μL solvent/day is administrated by intraperitoneal infusion on Day 14, once a day for 5 consecutive days; after 2 days of interval, 200 μL solvent/day is administrated again by intraperitoneal infusion, once a day for 5 consecutive days; $5\times10^6$ 806-mCD28z-CAR-T cells are administrated by tail vein infusion on Day 21.

Olaparib (50mg/kg)+CAR-T($5\times10^6$) group: 50mg/kg olaparib/day is administrated by intraperitoneal infusion on Day 14, once a day for 5 consecutive days; after 2 days of interval, 50mg/kg olaparib/day is administrated again by intraperitoneal infusion, once a day for 5 consecutive days; $5\times10^6$ 806-mCD28z-CAR-T cells are administrated by tail vein infusion on Day 21.

(2) Detection of tumor volume. The tumor volume changes of mice are observed and measured continuously, and recorded three times a week. The calculation formula of tumor volume is: tumor volume = (tumor length $\times$ tumor width$^2$)/2.

The detection results of the tumor volumes of mice are shown in Fig. 5B. The results show that: on the 35th day after tumor inoculation, compared with CAR-T ($2\times10^6$) group, in the Olaparib (50mg/kg) + CAR-T ($2\times10^6$) group the tumor volumes of mice are significantly reduced; compared with the CAR-T ($5\times10^6$) group, in the Olaparib (50mg/kg)+CAR-T (5×10$^6$) group the tumor volumes of the mice are significantly reduced. The above results suggest that, CAR-T cells combined with olaparib may effectively inhibit the growth of mouse breast cancer (Two-way ANOVA with Bonferroni post-tests, * means p<0.05, ** means p<0.01).

At the same time, it is detected that the body weights of the mice in each group do not change significantly (as shown in Fig. 5C), suggesting that the combination of olaparib do not cause significant toxicity to the combination therapy with CAR-T cells.

(3) Measurement of tumor inhibition rate and tumor weight. On the 35th day after tumor inoculation, the mice are euthanized, and the tumor in situ of the mouse breast is stripped and weighed. The specific statistical results are shown in Fig. 5D. Compared with the CAR-T (2×10$^6$) group, in the Olaparib (50mg/kg)+CAR-T (2×10$^6$) group the tumor suppression efficiency and tumor weight of the mice are significantly reduced (*** means p<0.001, Unpaired test); Compared with CAR-T (5×10$^6$) group, in the Olaparib (50mg/kg)+CAR-T (5×10$^6$) group the tumor suppression efficiency and tumor weight of the mice are significantly reduced (*** means p<0.001, Unpaired t test).

This suggests that the combination of olaparib may improve the inhibition effect of CAR-T cells on tumor growth, and has a better anti-tumor effect on mouse breast cancer.

(4) Detection of CAR-T copy number in tumor tissues. Tumor tissues of each group of mice are ground to extract DNA, and the sequence of retroviral vector fragment is detected by Taqman probe method to determine the DNA copy number of 806-mCD28z-CAR.

The primer sequences of Taq probe method are as follows:

Forward primer: 5'-GACGTTGGGTTACCTTCTGC-3' (SEQ ID No.52)
Reverse primer: 5'-TTCCCAGGTCACGATGTAGG-3' (SEQ ID No.53)

The primer sequence of probe: 5'-(FAM)-ATGGCCGCGAGACGGCACCT-(BHQ1)-3'(SEQ ID No.54)

The results of the experiment are shown in Fig. 5E. Compared with the CAR-T (2×10$^6$) group, in the Olaparib (50mg/kg)+CAR-T (2×10$^6$) group the copy number of CAR in the tumor of mice is significantly increased (Unpaired t test, * means p<0.05); Compared with the CAR-T (5×10$^6$) group, in the Olaparib (50mg/kg)+CAR-T (5×10$^6$) group the copy number of CAR in the tumor of mice is significantly increased (Unpaired test, ** means p<0.01). The above results indicate that, the combination of olaparib may improve the survival and expansion of CAR-T cells in tumor tissues, and play a role in improving anti-tumor effect.

(5) CD4/CD8/CD31 immunohistochemical detection of tumor tissues. Immunohistochemical staining is performed on paraffin sections of tumor tissues of mice in each group. CD4 and CD8 may reflect the infiltration of T cells in tissues, and CD31 may reflect the level of angiogenesis in tumor tissues. As shown in Fig. 5F, there is no significant difference in CD4$^+$T cell infiltration in the tumor tissue of mice in each group, and there is no statistical difference; but compared with the CAR-T (5×10$^6$) group, in the Olaparib (50mg/kg)+CAR-T (5×10$^6$) group the CD8$^+$T infiltration in the tumor of mice is significantly increased (Unpaired test, * means p<0.05). In addition, compared with the CAR-T (5×10$^6$) group, in the Olaparib (50mg/kg)+CAR-T (5×10$^6$) group CD31$^+$ cells in the tumor tissue of mice is also fewer, suggesting that the neovascularization is also inhibited. The above results indicate that, the combination of olaparib may increase the infiltration of CD8$^+$T cells with killing ability in tumor tissues, thereby exerting an anti-tumor effect.

(6) Detection of MDSCs (myeloid-derived suppressor cells) in tumor tissue. The tumor tissues of mice in each group are ground, and after enzyme digestion, the changes in the proportion of MDSCs are analyzed by flow cytometry. As shown in Fig. 5G, compared with the CAR-T (2×10$^6$) group, in the Olaparib (50mg/kg)+CAR-T (2×10$^6$) group CD45$^+$ immune cell infiltration in the tumor tissue of mice is significantly increased, but MDSCs cell infiltration of CD11b$^+$Gr1$^+$ is significantly reduced; compared with CAR-T (5×10$^6$) group, in the Olaparib (50mg/kg)+CAR-T (5×10$^6$) group CD45+ immune cell infiltration in tumor tissues of mice is significantly increased, but MDSCs cell infiltration of CD11b$^+$Gr1$^+$ is significantly reduced. MDSCs may produce tumor immunosuppressive microenvironment, which is not conducive to the role of CART in killing tumors. Therefore, the above results indicate that, the combination of olaparib may improve the tumor immunosuppressive environment, thereby promoting the anti-tumor effect of CAR-T.

**Example 6: Combined Treatment of Mouse 4T1-EGFRvIII Orthotopic Breast Cancer Xenografts by Olaparib and CAR-T**

(1) Establishment of mouse orthotopic breast cancer model and respective treatment in groups:
4T1-EGFRvIII cells in the logarithmic growth phase and growing well are collected, inoculating 5×10$^5$ target cells at right side position of the third pair of accessory breasts of BABL/C mice (mice with normal immune system), and the inoculating day is recorded as the first day (i.e., Day1).

On the 10th day after tumor inoculation (i.e. Day 10), mouse T cells are taken and 806-mCD28z-CAR-T cell line is constructed as described in step 1 of this example.

On the 14th day after tumor inoculation (i.e., Day 14), the volume of the tumor reaches about 150mm$^3$, and

the mice are divided in groups:
The formula of olaparib stock solution: olaparib is added with 4% DMSO + 30% PEG300 + 66% $ddH_2O$ in sequence; a solution of 5mg/mL is used as the stock solution.

Olaparib is intraperitoneally administrated, the injection amount of each mouse is 50mg/kg, and the weight of the mouse is about 20g, so the injection for each mouse is 200μL; the control group is given 200μL solvent treatment (4% DMSO + 30% PEG 300 + 66% $ddH_2O$).

The mice are divided into 4 groups, 6 mice for each group. The specific *in vivo* experimental procedure is shown in Fig. 6A:
UTD group: 200 μL solvent/day is administrated by intraperitoneal infusion on Day 14, once a day for 5 consecutive days; after 2 days of interval, 200 μL solvent/day is administrated again by intraperitoneal infusion, once a day for 5 consecutive days; $5\times10^6$ mouse T cells without infected by virus are administrated by tail vein infusion on Day 21.

Olaparib (50mg/kg) group: 50mg/kg olaparib/day is administrated by intraperitoneal infusion on Day 14, once a day for 5 consecutive days; after 2 days of interval, 50mg/kg olaparib/day is administrated again by intraperitoneal infusion, once a day for 5 consecutive days; $5\times10^6$ mouse T cells without infected by virus are administrated by tail vein infusion on Day 21.

CAR-T ($5\times10^6$) group: 200 μL solvent/day is administrated by intraperitoneal infusion on Day 14, once a day for 5 consecutive days; after 2 days of interval, 200 μL solvent/day is administrated again by intraperitoneal infusion, once a day for 5 consecutive days; $5\times10^6$ 806-mCD28z-CAR-T cells are administrated by tail vein infusion on Day 21.

Olaparib (50mg/kg)+CAR-T ($5\times10^6$) group: 50mg/kg olaparib/day is administrated by intraperitoneal infusion on Day 14, once a day for 5 consecutive days; after 2 days of interval, 50mg/kg olaparib/day is administrated again by intraperitoneal infusion, once a day for 5 consecutive days; $5\times10^6$ 806-mCD28z-CAR-T cells are administrated by tail vein infusion on Day 21.

(2) Detection of tumor volume. The tumor volume changes of mice are observed and measured continuously, and recorded three times a week. The calculation formula of tumor volume is: tumor volume = (tumor length × tumor width$^2$)/2.

The detection results of the tumor volumes of mice are shown in Fig. 6B. The results show that: on the 35th day after tumor inoculation, compared with CAR-T ($5\times10^6$) group, in the Olaparib (50mg/kg) + CAR-T ($5\times10^6$) group the tumor volumes of mice are significantly reduced. The above results suggest that, CAR-T cells combined with olaparib may effectively inhibit the growth of mouse breast cancer (Two-way ANOVA with Bonferroni post-tests, * means $p<0.05$).

At the same time, it is detected that the body weights of the mice in each group do not change significantly (as shown in Fig. 6C), suggesting that the combination of olaparib do not cause significant toxicity to the combination therapy with CAR-T cells.

(3) Measurement of tumor inhibition rate and tumor weight. On the 35th day after tumor inoculation, the mice are euthanized, and the tumor in situ of the mouse breast is stripped and weighed. The specific statistical results are shown in Fig. 6D. Compared with the CAR-T ($5\times10^6$) group, in the Olaparib (50mg/kg)+CAR-T ($5\times10^6$) group the tumor suppression efficiency and tumor weight of the mice are significantly reduced (* means $p<0.05$, Unpaired test).

This suggests that the combination of olaparib may improve the inhibition effect of CAR-T cells on tumor growth, and has a better anti-tumor effect on mouse breast cancer.

(4) Detection of CAR-T copy number in tumor tissues. Tumor tissues of each group of mice are ground to extract DNA, and the sequence of retroviral vector fragment is detected by Taqman probe method to determine the DNA copy number of 806-mCD28z-CAR.

The primer sequences of Taq probe method are as follows:

Forward primer: 5'-GACGTTGGGTTACCTTCTGC-3' (SEQ ID No.52)
Reverse primer: 5'-TTCCCAGGTCACGATGTAGG-3' (SEQ ID No.53)

The primer sequence of probe: 5'-(FAM)-ATGGCCGCGAGACGGCACCT-(BHQ1)-3'(SEQ ID No.54)

The results of the experiment are shown in Fig. 6E. Compared with the CAR-T ($5\times10^6$) group, in the Olaparib (50mg/kg)+CAR-T ($5\times10^6$) group the copy number of CAR in the tumor of mice is significantly increased (Unpaired t test, ** means $p<0.01$). The above results indicate that, the combination of olaparib may improve the survival and expansion of CAR-T cells in tumor tissues, and play a role in improving anti-tumor effect.

(5) CD4/CD8/CD31 immunohistochemical detection of tumor tissues. Immunohistochemical staining is performed on paraffin sections of tumor tissues of mice in each group. CD4 and CD8 may reflect the infiltration of T cells in tissues, and CD31 may reflect the level of angiogenesis in tumor tissues. As shown in Fig. 6F, there is no significant difference in $CD4^+$T cell infiltration in the tumor tissue of mice in each group, and there is no statistical difference; but compared with the CAR-T ($5\times10^6$) group, in the Olaparib (50mg/kg)+CAR-T ($5\times10^6$) group the $CD8^+$T infiltration in the tumor of mice is significantly increased (Unpaired test, * means $p<0.05$). In addition, compared with the CAR-T ($5\times10^6$) group, in the Olaparib (50mg/kg)+CAR-T ($5\times10^6$) group $CD31^+$ cells in the tumor tissue of mice is also fewer, suggesting that the neovascularization is also inhibited. The above results indicate that, the combination of olaparib may increase the infiltration of $CD8^+$T cells with killing ability in tumor tissues, thereby exerting an anti-tumor effect.

(6) Detection of MDSC cells in tumor tissue. The tumor tissues of mice in each group are ground, and after enzyme digestion, the changes in the proportion of MDSC cells are analyzed by flow cytometry. As shown in Fig. 6G, compared with the CAR-T ($5\times10^6$) group, in the Olaparib (50mg/kg)+CAR-T ($5\times10^6$) group $CD45^+$ immune cell infiltration in the tumor tissue of mice is significantly increased, but MDSCs cell infiltration of $CD11b^+Gr1^+$ is significantly reduced. MDSCs cells may produce tumor immunosuppressive microenvironment, which is not conducive to the role of CART in killing tumors. Therefore, the above results indicate that, the combination of olaparib may improve the tumor immunosuppressive environment, thereby promoting the anti-tumor effect of CAR-T.

In the above examples, only as an example, CAR-T prepared with anti-EGFRvIII mouse antibody (SEQ ID NO: 11), mouse transmembrane domain and intracellular domain, etc., When applied to human therapy, usually the preparation may be conducted by selecting the following sequences: the coding sequence of human CD8$\alpha$ signal peptide (SEQ ID NO: 6), the coding sequence of human CD8$\alpha$ hinge region and transmembrane region (SEQ ID NO: 7), the coding sequence of human CD28 transmembrane domain (SEQ ID NO: 10), the coding sequence of human CD28 intracellular domain (SEQ ID NO: 8), and the coding sequence of human CD3$\zeta$ intracellular domain (SEQ ID NO: 9). Exemplarily, the amino acid sequence of the adopted chimeric antigen receptor targeting EFGRvIII is shown in any one of SEQ ID NOs: 30-51 or SEQ ID NOs: 55-87.

All the documents mentioned in the present invention are cited as references in this application, as if each document is individually cited as a reference. In addition, it should be understood that after reading the above teaching content of the present invention, those skilled in the art may make various changes or modifications to the present invention, and these equivalent forms also fall within the scope defined by the appended claims of the application.

| Name | SEQ ID NO: | Sequences |
|---|---|---|
| Mouse CD8α signal peptide | 1 | atggcctcaccgttgacccgctttctgtcgctgaacctgctgctgctgggtgagtcgattatcctggggagtggagaagct |
| Anti-EGFRvIII monoclonal antibody | 2 | gacatcctgatgacccaatctccatcctccatgtctgtatctctgggagacacagtcagcatcacttgccattcaagtcaggacattaacagtaata tagggtggttgcagcagagaccagggaaatcatttaagggcctgatctatcatggaaccaacttggacgatgaagttccatcaaggttcagtgg cagtggatctggagccgattattctctcaccatcagcagcctggaatctgaagattttgcagactattactgtgtacagtatgctcagtttccgtgga cgttcggtggaggcaccaagctggaaatcaaacgtggtggaggcggttcaggcggaggtggctctggcggtggcggatcggccgatgtgc agcttcaggagtcgggacctagcctggtgaaaccttctcagtctctgtccctcacctgcactgtcactggctactcaatcaccagtgattttgcctg gaactggatccggcagtttccaggaaacaagctggagtggatgggctacataagttatagtggtaacactaggtacaacccatctctcaaaagt cgaatctctatcactcgagacacatccaagaaccaattcttcctgcagttgaattctgtgactattgaggacacagccacatattactgtgtaacgg cgggacgcgggtttccttattggggccaagggactctggtcactgtctctgca |
| Mouse CD8α hinge region and transmembrane region | 3 | actactaccaagccagtgctgcgaactccctcacctgtgcaccctaccgggacatctcagccccagagaccagaagattgtcggccccgtgg ctcagtgaaggggaccggattggacttcgcctgtgatatttacatctgggcacccttggccggaatctgcgtggcccttctgctgtccttgatcat cactctcatctgctaccacaggagccga |
| Mouse CD28 intracellular domain | 4 | aatagtagaaggaacagactccttcaaagtgactacatgaacatgactccccggaggcctgggctcactcgaaagccttaccagccctacgcc cctgccagagactttgcagcgtaccgcccc |
| Mouse CD3ζ intracellular domain | 5 | agcaggagtgcagagactgctgccaacctgcaggaccccaaccagctctacaatgagctcaatctagggcgaagagaggaatatgacgtctt ggagaagaagcgggctcgggatccagagatgggaggcaaacagcagaggaggaggaacccccaggaaggcgtatacaatgcactgcag aaagacaagatggcagaagcctacagtgagatcggcacaaaaggcgagaggcggagaggcaaggggcacgatggcctttaccagggtct cagcactgccaccaaggacacctatgatgccctgcatatgcagaccctggcc |
| Human CD8α signal peptid | 6 | atggccttaccagtgaccgccttgctcctgccgctggccttgctgctccacgccgccaggccg |
| Human CD8α hinge region and transmembrane region | 7 | accacgacgccagcgccgcgaccaccaacaccggcgcccaccatcgcgtcgcagcccctgtccctgcgcccagaggcgtgccggccag cggcgggggggcgcagtgcacacgagggggctggacttcgcctgtgatatctacatctgggcgcccttggccgggacttgtggggtccttctc ctgtcactggttatcacc |
| Human CD28 intracellular domain | 8 | aggagtaagaggagcaggctcctgcacagtgactacatgaacatgactccccgccgccccgggccaacccgcaagcattaccagccctatg ccccaccacgcgacttcgcagcctatcgctcc |

(continued)

| Name | SEQ ID NO: | Sequences |
|---|---|---|
| Human CD3ζ intracellular domain | 9 | agagtgaagttcagcaggagcgcagacgcccccgcgtaccagcagggccagaaccagctctataacgagctcaatctaggacgaagagag<br>gagtacgatgttttggacaagagacgtggccgggaccctgagatgggggggaaagccgcagagaaggaagaaccctcaggaaggcctgta<br>caatgaactgcagaaagataagatggcggaggcctacagtgagattgggatgaaaggcgagcgccggaggggcaaggggcacgatggc<br>ctttaccagggtctcagtacagccaccaaggacacctacgacgcccttcacatgcaggccctgccccctcgc |
| Human CD28 transmembrane domain | 10 | ttttgggtgctggtggtggttggtggagtcctggcttgctatagcttgctagtaacagtggcctttattattttctgggtg |
| Anti-EGFRvIII monoclonal<br><br>antibody (amino acid sequence) | 11 | DILMTQSPSSMSVSLGDTVSITCHSSQDINSNIGWLQQRPGKSFKGLIYHGTNLDDEVPSRF<br>SGSGSGADYSLTISSLESEDFADYYCVQYAQFPWTFGGGTKLEIKRGGGGSGGGGSGGGGS<br>ADVQLQESGPSLVKPSQSLSLTCTVTGYSITSDFAWNWIRQFPGNKLEWMGYISYSGNTRY<br>NPSLKSRISITRDTSKNQFFLQLNSVTIEDTATYYCVTAGRGFPYWGQGTLVTVSA |
| Antibody 7B3 (amino acid sequence) | 12 | DIQMTQSPSSLSASVGDRVTITCHASQDINSNIGWLQQKPGKAFKGLIYHGKNLEDGVPSR<br>FSGSGSGTDFTLTISSLQPEDFATYYCVQYAQFPYTFGQGTKVEIKRGGGGSGGGGSGGGG<br>SDVQLVESGGGLVQPGGSLRLSCAVSGYSITSDYAWNWIRQAPGKGLEWLGYISYRGRTSY<br>NPSLKSRISITRDNSKNTFFLQLNSLRAEDTAVYYCARLGRGFRYWGQGTLVTVSS |
| Antibody Y022 (amino acid sequence) | 13 | DIQMTQSPSSLSASVGDRVTITCHASQDINVNIGWLQQKPGKAFKGLIYHGKNLEDGVPSR<br>FSGSGSGTDFTLTISSLQPEDFATYYCNQYENIPLTFGQGTKVEIKRGGGGSGGGGSGGGGS<br>DVQLVESGGGLVQPGGSLRLSCAVSGYSITSDYAWNWIRQAPGKGLEWLGYISYRGRTQY<br>NPSLKSRISITRDNSKNTFFLQLNSLRAEDTAVYYCARMGKNWDYWGQGTLVTVSS |
| Antibody S4 (amino acid sequence) | 14 | DIQMTQSPSSLSASVGDRVTITCHASQDIKVNIGWLQQKPGKAFKGLIYHGKNLEDGVPSR<br>FSGSGSGTDFTLTISSLQPEDFATYYCNQYENNPITFGQGTKVEIKRGGGGSGGGGSGGGGS<br>DVQLVESGGGLVQPGGSLRLSCAVSGYSITSDYAWNWIRQAPGKGLEWLGYISYRGRTSY<br>NPSLKSRISITRDNSKNTFFLQLNSLRAEDTAVYYCARLGRGFRYWGQGTLVTVSS |
| Antibody S9 (amino acid sequence) | 15 | DIQMTQSPSSLSASVGDRVTITCHASQDINVNIGWLQQKPGKAPKGLIYHGKNLEDGVPSR<br>FSGSGSGTDFTLTISSLQPEDFATYYCNQYENNPITFGQGTKVEIKRGGGGSGGGGSGGGGS<br>DVQLVESGGGLVQPGGSLRLSCAVSGYSITSDYAWNWIRQAPGKGLEWLGYISYRGRTSY<br>NPSLKSRISITRDNSKNTFFLQLNSLRAEDTAVYYCARLGRGFRYWGQGTLVTVSS |

(continued)

| Name | SEQ ID NO: | Sequences |
|---|---|---|
| Antibody S11 (amino acid sequence) | 16 | DIQMTQSPSSLSASVGDRVTITCHASQDINVNIGWLQQKPGKAFKGLIYHGKKLEDGVPSR FSGSGSGTDFTLTISSLQPEDFATYYCNQYENNPITFGQGTKVEIKRGGGGSGGGGSGGGGS DVQLVESGGGLVQPGGSLRLSCAVSGYSITSDYAWNWIRQAPGKGLEWLGYISYRGRTSY NPSLKSRISITRDNSKNTFFLQLNSLRAEDTAVYYCARLGRGFRYWGQGTLVTVSS |
| Antibody S12 (amino acid sequence) | 17 | DIQMTQSPSSLSASVGDRVTITCHASQDINVNIGWLQQKPGKAFKGLIYHGKYLEDGVPSR FSGSGSGTDFTLTISSLQPEDFATYYCNQYENNPITFGQGTKVEIKRGGGGSGGGGSGGGGS DVQLVESGGGLVQPGGSLRLSCAVSGYSITSDYAWNWIRQAPGKGLEWLGYISYRGRTSY NPSLKSRISITRDNSKNTFFLQLNSLRAEDTAVYYCARLGRGFRYWGQGTLVTVSS |
| Antibody S15 (amino acid sequence) | 18 | DIQMTQSPSSLSASVGDRVTITCHASQDINVNIGWLQQKPGKAFKGLIYHGKNLEDGVPSR FSGSGSGTDFTLTISSLQPEDFATYYCNQYENNPITFGQGTKVEIKRGGGGSGGGGSGGGGS DVQLVESGGGLVQPGGSLRLSCAVSGYSITSDYAWNWIRQAPGKGLEWLGYISYSGNTSY NPSLKSRISITRDNSKNTFFLQLNSLRAEDTAVYYCARLGRGFRYWGQGTLVTVSS |
| Antibody S21 (amino acid sequence) | 19 | DIQMTQSPSSLSASVGDRVTITCHASQDIEVNIGWLQQKPGKAFKGLIYHGKNLEDGVPSR FSGSGSGTDFTLTISSLQPEDFATYYCNQYENNPLTFGQGTKVEIKRGGGGSGGGGSGGGG SDVQLVESGGGLVQPGGSLRLSCAVSGYSITSDYAWNWIRQAPGKGLEWLGYISYRGRTRY NPSLKSRISITRDNSKNTFFLQLNSLRAEDTAVYYCARLGRGFRYWGQGTLVTVSS |
| Antibody M14 (amino acid sequence) | 20 | DIQMTQSPSSLSASVGDRVTITCHASQDINSNIGWLQQKPGKAFKGLIYHGKNLEDGVPSR FSGSGSGTDFTLTISSLQPEDFATYYCNQYENNPITFGQGTKVEIKRGGGGSGGGGSGGGGS DVQLVESGGGLVQPGGSLRLSCAVSGYSITSDYAWNWIRQAPGKGLEWLGYISYRGRTNY NPSLKSRISITRDNSKNTFFLQLNSLRAEDTAVYYCARLGRGFRYWGQGTLVTVSS |
| Antibody M15 (amino acid sequence) | 21 | DIQMTQSPSSLSASVGDRVTITCHASQDINVNIGWLQQKPGKAFKGLIYHGKNLEDGVPSR FSGSGSGTDFTLTISSLQPEDFATYYCNQYENNPITFGQGTKVEIKRGGGGSGGGGSGGGGS DVQLVESGGGLVQPGGSLRLSCAVSGYSITSDYAWNWIRQAPGKGLEWLGYISYRGRTSY NPSLKSRISITRDNSKNTFFLQLNSLRAEDTAVYYCARLGRGFRYWGQGTLVTVSS |
| Antibody M25 | 22 | DIQMTQSPSSLSASVGDRVTITCHASQDINVNIGWLQQKPGKAFKGLIYHGKNLEDGVPSR |

(continued)

| Name | SEQ ID NO: | Sequences |
|---|---|---|
| (amino acid sequence) | | FSGSGSGTDFTLTISSLQPEDFATYYCNQYENIPLTFGQGTKVEIKRGGGGSGGGGSGGGGS DVQLVESGGGLVQPGGSLRLSCAVSGYSITSDYAWNWIRQAPGKGLEWLGYISYRGRTRY NPSLKSRISITRDNSKNTFFLQLNSLRAEDTAVYYCARLGRGFRYWGQGTLVTVSS |
| Antibody M26 (amino acid sequence) | 23 | DIQMTQSPSSLSASVGDRVTITCHASQDINVNIGWLQQKPGKAFKGLIYHGKNLEDGVPSR FSGSGSGTDFTLTISSLQPEDFATYYCNQYENIPLTFGQGTKVEIKRGGGGSGGGGSGGGGS DVQLVESGGGLVQPGGSLRLSCAVSGYSITSDYAWNWIRQAPGKGLEWLGYISYRGRTQY NPSLKSRISITRDNSKNTFFLQLNSLRAEDTAVYYCARLGRGFRYWGQGTLVTVSS |
| Antibody S7 (amino acid sequence) | 24 | DIQMTQSPSSLSASVGDRVTITCHASQDINVNIGWLQQKPGKAFKGLIYHGTNLEDGVPSR FSGSGSGTDFTLTISSLQPEDFATYYCNQYENNPITFGQGTKVEIKRGGGGSGGGGSGGGGS DVQLVESGGGLVQPGGSLRLSCAVSGYSITSDYAWNWIRQAPGKGLEWLGYISYRGRTSY NPSLKSRISITRDNSKNTFFLQLNSLRAEDTAVYYCARLGRGFRYWGQGTLVTVSS |
| Antibody S8 (amino acid sequence) | 25 | DIQMTQSPSSLSASVGDRVTITCHASQDINVNIGWLQQKPGKSFKGLIYHGKNLEDGVPSR FSGSGSGTDFTLTISSLQPEDFATYYCNQYENNPITFGQGTKVEIKRGGGGSGGGGSGGGGS DVQLVESGGGLVQPGGSLRLSCAVSGYSITSDYAWNWIRQAPGKGLEWLGYISYRGRTSY NPSLKSRISITRDNSKNTFFLQLNSLRAEDTAVYYCARLGRGFRYWGQGTLVTVSS |
| Antibody S17 (amino acid sequence) | 26 | DIQMTQSPSSLSASVGDRVTITCHASQDINTNIGWLQQKPGKAFKGLIYHGKNLEDGVPSR FSGSGSGTDFTLTISSLQPEDFATYYCNQYENNPLTFGQGTKVEIKRGGGGSGGGGSGGGG SDVQLVESGGGLVQPGGSLRLSCAVSGYSITSDYAWNWIRQAPGKGLEWLGYISYRGRTQ YNPSLKSRISITRDNSKNTFFLQLNSLRAEDTAVYYCARLGRGFRYWGQGTLVTVSS |
| Antibody S22 (amino acid sequence) | 27 | DIQMTQSPSSLSASVGDRVTITCHASQDINVNIGWLQQKPGKAFKGLIYHGTNLEDGVPSR FSGSGSGTDFTLTISSLQPEDFATYYCNQYENNPLTFGQGTKVEIKRGGGGSGGGGSGGGG SDVQLVESGGGLVQPGGSLRLSCAVSGYSITSDYAWNWIRQAPGKGLEWLGYISYRGRTRY NPSLKSRISITRDNSKNTFFLQLNSLRAEDTAVYYCARLGRGFRYWGQGTLVTVSS |

(continued)

| Name | SEQ ID NO: | Sequences |
|---|---|---|
| Antibody S23 (amino acid sequence) | 28 | DIQMTQSPSSLSASVGDRVTITCHASQDINVNIGWLQQKPGKSFKGLIYHGKNLEDGVPSR<br>FSGSGSGTDFTLTISSLQPEDFATYYCNQYENNPLTFGQGTKVEIKRGGGGSGGGGSGGGG<br>SDVQLVESGGGLVQPGGSLRLSCAVSGYSITSDYAWNWIRQAPGKGLEWLGYISYRGRTRY<br>NPSLKSRISITRDNSKNTFFLQLNSLRAEDTAVYYCARLGRGFRYWGQGTLVTVSS |
| Antibody S29 (amino acid sequence) | 29 | DIQMTQSPSSLSASVGDRVTITCHASQDINVNIGWLQQKPGKAFKGLIYHGKNLEDGVPSR<br>FSGSGSGTDFTLTISSLQPEDFATYYCNQYENFPLTFGQGTKVEIKRGGGGSGGGGSGGGGS<br>DVQLVESGGGLVQPGGSLRLSCAVSGYSITSDYAWNWIRQAPGKGLEWLGYISYRGRTRY<br>NPSLKSRISITRDNSKNTFFLQLNSLRAEDTAVYYCARLGRGFRYWGQGTLVTVSS |
| 806-mCD28Z (amino acid sequence) | 30 | DILMTQSPSSMSVSLGDTVSITCHSSQDINSNIGWLQQRPGKSFKGLIYHGTNLDDEVPSRF<br>SGSGSGADYSLTISSLESEDFADYYCVQYAQFPWTFGGGTKLEIKRGGGGSGGGGSGGGGS<br>ADVQLQESGPSLVKPSQSLSLTCTVTGYSITSDFAWNWIRQFPGNKLEWMGYISYSGNTRY<br>NPSLKSRISITRDTSKNQFFLQLNSVTIEDTATYYCVTAGRGFPYWGQGTLVTVSATTTKPV<br>LRTPSPVHPTGTSQPQRPEDCRPRGSVKGTGLDFACDIYIWAPLAGICVALLLSLIITLICYHR<br>SRNSRRNRLLQSDYMNMTPRRPGLTRKPYQPYAPARDFAAYRPSRSAETAANLQDPNQLY<br>NELNLGRREEYDVLEKKRARDPEMGGKQQRRRNPQEGVYNALQKDKMAEAYSEIGTKG<br>ERRRGKGHDGLYQGLSTATKDTYDALHMQTLA |
| Y022-28Z | 31 | DIQMTQSPSSLSASVGDRVTITCHASQDINVNIGWLQQKPGKAFKGLIYHGKNLEDGVPSR<br>FSGSGSGTDFTLTISSLQPEDFATYYCNQYENIPLTFGQGTKVEIKRGGGGSGGGGSGGGGS<br>DVQLVESGGGLVQPGGSLRLSCAVSGYSITSDYAWNWIRQAPGKGLEWLGYISYRGRTQY<br>NPSLKSRISITRDNSKNTFFLQLNSLRAEDTAVYYCARMGKNWDYWGQGTLVTVSSTTTPA<br>PRPPTPAPTIASQPLSLRPEACRPAAGGAVHTRGLDFACDFWVLVVVGGVLACYSLLVTVA<br>FIIFWVRSKRSRLLHSDYMNMTPRRPGPTRKHYQPYAPPRDFAAYRSRVKFSRSADAPAYQ<br>QGQNQLYNELNLGRREEYDVLDKRRGRDPEMGGKPQRRKNPQEGLYNELQKDKMAEAY<br>SEIGMKGERRRGKGHDGLYQGLSTATKDTYDALHMQALPPR |

(continued)

| Name | SEQ ID NO: | Sequences |
|---|---|---|
| Y022-BBZ | 32 | DIQMTQSPSSLSASVGDRVTITCHASQDINVNIGWLQQKPGKAFKGLIYHGKNLEDGVPSR<br>FSGSGSGTDFTLTISSLQPEDFATYYCNQYENIPLTFGQGTKVEIKRGGGGSGGGGSGGGGS<br>DVQLVESGGGLVQPGGSLRLSCAVSGYSITSDYAWNWIRQAPGKGLEWLGYISYRGRTQY<br>NPSLKSRISITRDNSKNTFFLQLNSLRAEDTAVYYCARMGKNWDYWGQGTLVTVSSTTTPA<br>PRPPTPAPTIASQPLSLRPEACRPAAGGAVHTRGLDFACDIYIWAPLAGTCGVLLLSLVITLY<br>CKRGRKKLLYIFKQPFMRPVQTTQEEDGCSCRFPEEEEGGCELRVKFSRSADAPAYKQGQN<br>QLYNELNLGRREEYDVLDKRRGRDPEMGGKPRRKNPQEGLYNELQKDKMAEAYSEIGMK<br>GERRRGKGHDGLYQGLSTATKDTYDALHMQALPPR |
| Y022-28BBZ | 33 | DIQMTQSPSSLSASVGDRVTITCHASQDINVNIGWLQQKPGKAFKGLIYHGKNLEDGVPSR<br>FSGSGSGTDFTLTISSLQPEDFATYYCNQYENIPLTFGQGTKVEIKRGGGGSGGGGSGGGGS<br>DVQLVESGGGLVQPGGSLRLSCAVSGYSITSDYAWNWIRQAPGKGLEWLGYISYRGRTQY<br>NPSLKSRISITRDNSKNTFFLQLNSLRAEDTAVYYCARMGKNWDYWGQGTLVTVSSTTTPA<br>PRPPTPAPTIASQPLSLRPEACRPAAGGAVHTRGLDFACDFWVLVVVGGVLACYSLLVTVA<br>FIIFWVRSKRSRLLHSDYMNMTPRRPGPTRKHYQPYAPPRDFAAYRSKRGRKKLLYIFKQP<br>FMRPVQTTQEEDGCSCRFPEEEEGGCELRVKFSRSADAPAYQQGQNQLYNELNLGRREEY<br>DVLDKRRGRDPEMGGKPQRRKNPQEGLYNELQKDKMAEAYSEIGMKGERRRGKGHDGL<br>YQGLSTATKDTYDALHMQALPPR |
| S4-28Z | 34 | DIQMTQSPSSLSASVGDRVTITCHASQDIKVNIGWLQQKPGKAFKGLIYHGKNLEDGVPSR<br>FSGSGSGTDFTLTISSLQPEDFATYYCNQYENNPITFGQGTKVEIKRGGGGSGGGGSGGGGS<br>DVQLVESGGGLVQPGGSLRLSCAVSGYSITSDYAWNWIRQAPGKGLEWLGYISYRGRTSY<br>NPSLKSRISITRDNSKNTFFLQLNSLRAEDTAVYYCARLGRGFRYWGQGTLVTVSSTTTPAP<br>RPPTPAPTIASQPLSLRPEACRPAAGGAVHTRGLDFACDFWVLVVVGGVLACYSLLVTVAFI<br>IFWVRSKRSRLLHSDYMNMTPRRPGPTRKHYQPYAPPRDFAAYRSRVKFSRSADAPAYQQ<br>GQNQLYNELNLGRREEYDVLDKRRGRDPEMGGKPQRRKNPQEGLYNELQKDKMAEAYS<br>EIGMKGERRRGKGHDGLYQGLSTATKDTYDALHMQALPPR |

(continued)

| Name | SEQ ID NO: | Sequences |
|---|---|---|
| S4-BBZ | 35 | DIQMTQSPSSLSASVGDRVTITCHASQDIKVNIGWLQQKPGKAFKGLIYHGKNLEDGVPSRFSGSGSGTDFTLTISSLQPEDFATYYCNQYENNPITFGQGTKVEIKRGGGGSGGGGSGGGGSDVQLVESGGGLVQPGGSLRLSCAVSGYSITSDYAWNWIRQAPGKGLEWLGYISYRGRTSYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYKQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| S4-28BBZ | 36 | DIQMTQSPSSLSASVGDRVTITCHASQDIKVNIGWLQQKPGKAFKGLIYHGKNLEDGVPSRFSGSGSGTDFTLTISSLQPEDFATYYCNQYENNPITFGQGTKVEIKRGGGGSGGGGSGGGGSDVQLVESGGGLVQPGGSLRLSCAVSGYSITSDYAWNWIRQAPGKGLEWLGYISYRGRTSYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSKRGRKKLLYIFKQPFMRPVQTTQEEDGCSCRFPEEEEGGCELRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |
| S9-28Z | 37 | DIQMTQSPSSLSASVGDRVTITCHASQDINVNIGWLQQKPGKAPKGLIYHGKNLEDGVPSRFSGSGSGTDFTLTISSLQPEDFATYYCNQYENNPITFGQGTKVEIKRGGGGSGGGGSGGGGSDVQLVESGGGLVQPGGSLRLSCAVSGYSITSDYAWNWIRQAPGKGLEWLGYISYRGRTSYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |

(continued)

| Name | SEQ ID NO: | Sequences |
|---|---|---|
| S9-BBZ | 38 | DIQMTQSPSSLSASVGDRVTITCHASQDINVNIGWLQQKPGKAPKGLIYHGKNLEDGVPSRFSGSGSGTDFTLTISSLQPEDFATYYCNQYENNPITFGQGTKVEIKRGGGGSGGGGSGGGGSDVQLVESGGGLVQPGGSLRLSCAVSGYSITSDYAWNWIRQAPGKGLEWLGYISYRGRTSYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYKQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| S9-28BBZ | 39 | DIQMTQSPSSLSASVGDRVTITCHASQDINVNIGWLQQKPGKAPKGLIYHGKNLEDGVPSRFSGSGSGTDFTLTISSLQPEDFATYYCNQYENNPITFGQGTKVEIKRGGGGSGGGGSGGGGSDVQLVESGGGLVQPGGSLRLSCAVSGYSITSDYAWNWIRQAPGKGLEWLGYISYRGRTSYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSKRGRKKLLYIFKQPFMRPVQTTQEEDGCSCRFPEEEEGGCELRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |
| S11-28Z | 40 | DIQMTQSPSSLSASVGDRVTITCHASQDINVNIGWLQQKPGKAFKGLIYHGKKLEDGVPSRFSGSGSGTDFTLTISSLQPEDFATYYCNQYENNPITFGQGTKVEIKRGGGGSGGGGSGGGGSDVQLVESGGGLVQPGGSLRLSCAVSGYSITSDYAWNWIRQAPGKGLEWLGYISYRGRTSYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |

(continued)

| Name | SEQ ID NO: | Sequences |
|---|---|---|
| S11-BBZ | 41 | DIQMTQSPSSLSASVGDRVTITCHASQDINVNIGWLQQKPGKAFKGLIYHGKKLEDGVPSR FSGSGSGTDFTLTISSLQPEDFATYYCNQYENNPITFGQGTKVEIKRGGGGSGGGGSGGGGS DVQLVESGGGLVQPGGSLRLSCAVSGYSITSDYAWNWIRQAPGKGLEWLGYISYRGRTSY NPSLKSRISITRDNSKNTFFLQLNSLRAEDTAVYYCARLGRGFRYWGQGTLVTVSSTTTPAP RPPTPAPTIASQPLSLRPEACRPAAGGAVHTRGLDFACDIYIWAPLAGTCGVLLLSLVITLYC KRGRKKLLYIFKQPFMRPVQTTQEEDGCSCRFPEEEEGGCELRVKFSRSADAPAYKQGQNQ LYNELNLGRREEYDVLDKRRGRDPEMGGKPRRKNPQEGLYNELQKDKMAEAYSEIGMKG ERRRGKGHDGLYQGLSTATKDTYDALHMQALPPR |
| S11-28BBZ | 42 | DIQMTQSPSSLSASVGDRVTITCHASQDINVNIGWLQQKPGKAFKGLIYHGKKLEDGVPSR FSGSGSGTDFTLTISSLQPEDFATYYCNQYENNPITFGQGTKVEIKRGGGGSGGGGSGGGGS |
| | | DVQLVESGGGLVQPGGSLRLSCAVSGYSITSDYAWNWIRQAPGKGLEWLGYISYRGRTSY NPSLKSRISITRDNSKNTFFLQLNSLRAEDTAVYYCARLGRGFRYWGQGTLVTVSSTTTPAP RPPTPAPTIASQPLSLRPEACRPAAGGAVHTRGLDFACDFWVLVVVGGVLACYSLLVTVAFI IFWVRSKRSRLLHSDYMNMTPRRPGPTRKHYQPYAPPRDFAAYRSKRGRKKLLYIFKQPF MRPVQTTQEEDGCSCRFPEEEEGGCELRVKFSRSADAPAYQQGQNQLYNELNLGRREEYD VLDKRRGRDPEMGGKPQRRKNPQEGLYNELQKDKMAEAYSEIGMKGERRRGKGHDGLY QGLSTATKDTYDALHMQALPPR |
| S12-28Z | 43 | DIQMTQSPSSLSASVGDRVTITCHASQDINVNIGWLQQKPGKAFKGLIYHGKYLEDGVPSR FSGSGSGTDFTLTISSLQPEDFATYYCNQYENNPITFGQGTKVEIKRGGGGSGGGGSGGGGS DVQLVESGGGLVQPGGSLRLSCAVSGYSITSDYAWNWIRQAPGKGLEWLGYISYRGRTSY NPSLKSRISITRDNSKNTFFLQLNSLRAEDTAVYYCARLGRGFRYWGQGTLVTVSSTTTPAP RPPTPAPTIASQPLSLRPEACRPAAGGAVHTRGLDFACDFWVLVVVGGVLACYSLLVTVAFI IFWVRSKRSRLLHSDYMNMTPRRPGPTRKHYQPYAPPRDFAAYRSRVKFSRSADAPAYQQ GQNQLYNELNLGRREEYDVLDKRRGRDPEMGGKPQRRKNPQEGLYNELQKDKMAEAYS EIGMKGERRRGKGHDGLYQGLSTATKDTYDALHMQALPPR |

(continued)

| Name | SEQ ID NO: | Sequences |
|---|---|---|
| S12-BBZ | 44 | DIQMTQSPSSLSASVGDRVTITCHASQDINVNIGWLQQKPGKAFKGLIYHGKYLEDGVPSR FSGSGSGTDFTLTISSLQPEDFATYYCNQYENNPITFGQGTKVEIKRGGGGSGGGGSGGGGS DVQLVESGGGLVQPGGSLRLSCAVSGYSITSDYAWNWIRQAPGKGLEWLGYISYRGRTSY NPSLKSRISITRDNSKNTFFLQLNSLRAEDTAVYYCARLGRGFRYWGQGTLVTVSSTTTPAP RPPTPAPTIASQPLSLRPEACRPAAGGAVHTRGLDFACDIYIWAPLAGTCGVLLLSLVITLYC KRGRKKLLYIFKQPFMRPVQTTQEEDGCSCRFPEEEEGGCELRVKFSRSADAPAYKQGQNQ LYNELNLGRREEYDVLDKRRGRDPEMGGKPRRKNPQEGLYNELQKDKMAEAYSEIGMKG ERRRGKGHDGLYQGLSTATKDTYDALHMQALPPR |
| S12-28BBZ | 45 | DIQMTQSPSSLSASVGDRVTITCHASQDINVNIGWLQQKPGKAFKGLIYHGKYLEDGVPSR FSGSGSGTDFTLTISSLQPEDFATYYCNQYENNPITFGQGTKVEIKRGGGGSGGGGSGGGGS DVQLVESGGGLVQPGGSLRLSCAVSGYSITSDYAWNWIRQAPGKGLEWLGYISYRGRTSY NPSLKSRISITRDNSKNTFFLQLNSLRAEDTAVYYCARLGRGFRYWGQGTLVTVSSTTTPAP RPPTPAPTIASQPLSLRPEACRPAAGGAVHTRGLDFACDFWVLVVVGGVLACYSLLVTVAFI IFWVRSKRSRLLHSDYMNMTPRRPGPTRKHYQPYAPPRDFAAYRSKRGRKKLLYIFKQPF MRPVQTTQEEDGCSCRFPEEEEGGCELRVKFSRSADAPAYQQGQNQLYNELNLGRREEYD VLDKRRGRDPEMGGKPQRRKNPQEGLYNELQKDKMAEAYSEIGMKGERRRGKGHDGLY QGLSTATKDTYDALHMQALPPR |
| S15-28Z | 46 | DIQMTQSPSSLSASVGDRVTITCHASQDINVNIGWLQQKPGKAFKGLIYHGKNLEDGVPSR FSGSGSGTDFTLTISSLQPEDFATYYCNQYENNPITFGQGTKVEIKRGGGGSGGGGSGGGGS DVQLVESGGGLVQPGGSLRLSCAVSGYSITSDYAWNWIRQAPGKGLEWLGYISYSGNTSY NPSLKSRISITRDNSKNTFFLQLNSLRAEDTAVYYCARLGRGFRYWGQGTLVTVSSTTTPAP RPPTPAPTIASQPLSLRPEACRPAAGGAVHTRGLDFACDFWVLVVVGGVLACYSLLVTVAFI IFWVRSKRSRLLHSDYMNMTPRRPGPTRKHYQPYAPPRDFAAYRSRVKFSRSADAPAYQQ GQNQLYNELNLGRREEYDVLDKRRGRDPEMGGKPQRRKNPQEGLYNELQKDKMAEAYS EIGMKGERRRGKGHDGLYQGLSTATKDTYDALHMQALPPR |

(continued)

| Name | SEQ ID NO: | Sequences |
|---|---|---|
| S15-BBZ | 47 | DIQMTQSPSSLSASVGDRVTITCHASQDINVNIGWLQQKPGKAFKGLIYHGKNLEDGVPSR FSGSGSGTDFTLTISSLQPEDFATYYCNQYENNPITFGQGTKVEIKRGGGGSGGGGSGGGGS DVQLVESGGGLVQPGGSLRLSCAVSGYSITSDYAWNWIRQAPGKGLEWLGYISYSGNTSY NPSLKSRISITRDNSKNTFFLQLNSLRAEDTAVYYCARLGRGFRYWGQGTLVTVSSTTTPAP RPPTPAPTIASQPLSLRPEACRPAAGGAVHTRGLDFACDIYIWAPLAGTCGVLLLSLVITLYC KRGRKKLLYIFKQPFMRPVQTTQEEDGCSCRFPEEEEGGCELRVKFSRSADAPAYKQGQNQ LYNELNLGRREEYDVLDKRRGRDPEMGGKPRRKNPQEGLYNELQKDKMAEAYSEIGMKG ERRRGKGHDGLYQGLSTATKDTYDALHMQALPPR |
| S15-28BBZ | 48 | DIQMTQSPSSLSASVGDRVTITCHASQDINVNIGWLQQKPGKAFKGLIYHGKNLEDGVPSR FSGSGSGTDFTLTISSLQPEDFATYYCNQYENNPITFGQGTKVEIKRGGGGSGGGGSGGGGS DVQLVESGGGLVQPGGSLRLSCAVSGYSITSDYAWNWIRQAPGKGLEWLGYISYSGNTSY NPSLKSRISITRDNSKNTFFLQLNSLRAEDTAVYYCARLGRGFRYWGQGTLVTVSSTTTPAP RPPTPAPTIASQPLSLRPEACRPAAGGAVHTRGLDFACDFWVLVVVGGVLACYSLLVTVAFI IFWVRSKRSRLLHSDYMNMTPRRPGPTRKHYQPYAPPRDFAAYRSKRGRKKLLYIFKQPF MRPVQTTQEEDGCSCRFPEEEEGGCELRVKFSRSADAPAYQQGQNQLYNELNLGRREEYD VLDKRRGRDPEMGGKPQRRKNPQEGLYNELQKDKMAEAYSEIGMKGERRRGKGHDGLY QGLSTATKDTYDALHMQALPPR |
| S21-28Z | 49 | DIQMTQSPSSLSASVGDRVTITCHASQDIEVNIGWLQQKPGKAFKGLIYHGKNLEDGVPSR FSGSGSGTDFTLTISSLQPEDFATYYCNQYENNPLTFGQGTKVEIKRGGGGSGGGGSGGGG SDVQLVESGGGLVQPGGSLRLSCAVSGYSITSDYAWNWIRQAPGKGLEWLGYISYRGRTRY NPSLKSRISITRDNSKNTFFLQLNSLRAEDTAVYYCARLGRGFRYWGQGTLVTVSSTTTPAP RPPTPAPTIASQPLSLRPEACRPAAGGAVHTRGLDFACDFWVLVVVGGVLACYSLLVTVAFI IFWVRSKRSRLLHSDYMNMTPRRPGPTRKHYQPYAPPRDFAAYRSRVKFSRSADAPAYQQ GQNQLYNELNLGRREEYDVLDKRRGRDPEMGGKPQRRKNPQEGLYNELQKDKMAEAYS EIGMKGERRRGKGHDGLYQGLSTATKDTYDALHMQALPPR |

(continued)

| Name | SEQ ID NO: | Sequences |
|---|---|---|
| S21-BBZ | 50 | DIQMTQSPSSLSASVGDRVTITCHASQDIEVNIGWLQQKPGKAFKGLIYHGKNLEDGVPSR<br>FSGSGSGTDFTLTISSLQPEDFATYYCNQYENNPLTFGQGTKVEIKRGGGGSGGGGSGGGG<br>SDVQLVESGGGLVQPGGSLRLSCAVSGYSITSDYAWNWIRQAPGKGLEWLGYISYRGRTRY<br>NPSLKSRISITRDNSKNTFFLQLNSLRAEDTAVYYCARLGRGFRYWGQGTLVTVSSTTTPAP<br>RPPTPAPTIASQPLSLRPEACRPAAGGAVHTRGLDFACDIYIWAPLAGTCGVLLLSLVITLYC<br>KRGRKKLLYIFKQPFMRPVQTTQEEDGCSCRFPEEEEGGCELRVKFSRSADAPAYKQGQNQ<br>LYNELNLGRREEYDVLDKRRGRDPEMGGKPRRKNPQEGLYNELQKDKMAEAYSEIGMKG<br>ERRRGKGHDGLYQGLSTATKDTYDALHMQALPPR |
| S21-28BBZ | 51 | DIQMTQSPSSLSASVGDRVTITCHASQDIEVNIGWLQQKPGKAFKGLIYHGKNLEDGVPSR<br>FSGSGSGTDFTLTISSLQPEDFATYYCNQYENNPLTFGQGTKVEIKRGGGGSGGGGSGGGG<br>SDVQLVESGGGLVQPGGSLRLSCAVSGYSITSDYAWNWIRQAPGKGLEWLGYISYRGRTRY<br>NPSLKSRISITRDNSKNTFFLQLNSLRAEDTAVYYCARLGRGFRYWGQGTLVTVSSTTTPAP<br>RPPTPAPTIASQPLSLRPEACRPAAGGAVHTRGLDFACDFWVLVVVGGVLACYSLLVTVAFI<br>IFWVRSKRSRLLHSDYMNMTPRRPGPTRKHYQPYAPPRDFAAYRSKRGRKKLLYIFKQPF<br>MRPVQTTQEEDGCSCRFPEEEEGGCELRVKFSRSADAPAYQQGQNQLYNELNLGRREEYD<br>VLDKRRGRDPEMGGKPQRRKNPQEGLYNELQKDKMAEAYSEIGMKGERRRGKGHDGLY<br>QGLSTATKDTYDALHMQALPPR |
| Forward primer | 52 | gacgttgggttaccttctgc |
| Reverse primer | 53 | ttcccaggtcacgatgtagg |
| The primer sequence of probe | 54 | (FAM)-atggccgcgagacggcacct-(BHQ1) |

(continued)

| Name | SEQ ID NO: | Sequences |
|---|---|---|
| 7B3-28Z | 55 | DIQMTQSPSSLSASVGDRVTITCHASQDINSNIGWLQQKPGKAFKGLIYHGKNLEDGVPSR<br>FSGSGSGTDFTLTISSLQPEDFATYYCVQYAQFPYTFGQGTKVEIKRGGGGSGGGGSGGGG<br>SDVQLVESGGGLVQPGGSLRLSCAVSGYSITSDYAWNWIRQAPGKGLEWLGYISYRGRTSY<br>NPSLKSRISITRDNSKNTFFLQLNSLRAEDTAVYYCARLGRGFRYWGQGTLVTVSSTTTPAP<br>RPPTPAPTIASQPLSLRPEACRPAAGGAVHTRGLDFACDFWVLVVVGGVLACYSLLVTVAFI<br>IFWVRSKRSRLLHSDYMNMTPRRPGPTRKHYQPYAPPRDFAAYRSRVKFSRSADAPAYQQ<br>GQNQLYNELNLGRREEYDVLDKRRGRDPEMGGKPQRRKNPQEGLYNELQKDKMAEAYS<br>EIGMKGERRRGKGHDGLYQGLSTATKDTYDALHMQALPPR |
| 7B3-BBZ | 56 | DIQMTQSPSSLSASVGDRVTITCHASQDINSNIGWLQQKPGKAFKGLIYHGKNLEDGVPSR<br>FSGSGSGTDFTLTISSLQPEDFATYYCVQYAQFPYTFGQGTKVEIKRGGGGSGGGGSGGGG<br>SDVQLVESGGGLVQPGGSLRLSCAVSGYSITSDYAWNWIRQAPGKGLEWLGYISYRGRTSY<br>NPSLKSRISITRDNSKNTFFLQLNSLRAEDTAVYYCARLGRGFRYWGQGTLVTVSSTTTPAP<br>RPPTPAPTIASQPLSLRPEACRPAAGGAVHTRGLDFACDIYIWAPLAGTCGVLLLSLVITLYC<br>KRGRKKLLYIFKQPFMRPVQTTQEEDGCSCRFPEEEEGGCELRVKFSRSADAPAYKQGQNQ<br>LYNELNLGRREEYDVLDKRRGRDPEMGGKPRRKNPQEGLYNELQKDKMAEAYSEIGMKG<br>ERRRGKGHDGLYQGLSTATKDTYDALHMQALPPR |
| 7B3-28BBZ | 57 | DIQMTQSPSSLSASVGDRVTITCHASQDINSNIGWLQQKPGKAFKGLIYHGKNLEDGVPSR<br>FSGSGSGTDFTLTISSLQPEDFATYYCVQYAQFPYTFGQGTKVEIKRGGGGSGGGGSGGGG<br>SDVQLVESGGGLVQPGGSLRLSCAVSGYSITSDYAWNWIRQAPGKGLEWLGYISYRGRTSY<br>NPSLKSRISITRDNSKNTFFLQLNSLRAEDTAVYYCARLGRGFRYWGQGTLVTVSSTTTPAP<br>RPPTPAPTIASQPLSLRPEACRPAAGGAVHTRGLDFACDFWVLVVVGGVLACYSLLVTVAFI<br>IFWVRSKRSRLLHSDYMNMTPRRPGPTRKHYQPYAPPRDFAAYRSKRGRKKLLYIFKQPF<br>MRPVQTTQEEDGCSCRFPEEEEGGCELRVKFSRSADAPAYQQGQNQLYNELNLGRREEYD<br>VLDKRRGRDPEMGGKPQRRKNPQEGLYNELQKDKMAEAYSEIGMKGERRRGKGHDGLY<br>QGLSTATKDTYDALHMQALPPR |

(continued)

| Name | SEQ ID NO: | Sequences |
|---|---|---|
| Antibody M14-28Z | 58 | DIQMTQSPSSLSASVGDRVTITCHASQDINSNIGWLQQKPGKAFKGLIYHGKNLEDGVPSRFSGSGSGTDFTLTISSLQPEDFATYYCNQYENNPITFGQGTKVEIKRGGGGSGGGGSGGGGSDVQLVESGGGLVQPGGSLRLSCAVSGYSITSDYAWNWIRQAPGKGLEWLGYISYRGRTNYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |
| Antibody M14-BBZ | 59 | DIQMTQSPSSLSASVGDRVTITCHASQDINSNIGWLQQKPGKAFKGLIYHGKNLEDGVPSRFSGSGSGTDFTLTISSLQPEDFATYYCNQYENNPITFGQGTKVEIKRGGGGSGGGGSGGGGSDVQLVESGGGLVQPGGSLRLSCAVSGYSITSDYAWNWIRQAPGKGLEWLGYISYRGRTNYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYKQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| Antibody M14-28BBZ | 60 | DIQMTQSPSSLSASVGDRVTITCHASQDINSNIGWLQQKPGKAFKGLIYHGKNLEDGVPSRFSGSGSGTDFTLTISSLQPEDFATYYCNQYENNPITFGQGTKVEIKRGGGGSGGGGSGGGGSDVQLVESGGGLVQPGGSLRLSCAVSGYSITSDYAWNWIRQAPGKGLEWLGYISYRGRTNYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSKRGRKKLLYIFKQPFMRPVQTTQEEDGCSCRFPEEEEGGCELRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |

(continued)

| Name | SEQ ID NO: | Sequences |
|---|---|---|
| Antibody M15-28Z | 61 | DIQMTQSPSSLSASVGDRVTITCHASQDINVNIGWLQQKPGKAFKGLIYHGKNLEDGVPSR FSGSGSGTDFTLTISSLQPEDFATYYCNQYENNPITFGQGTKVEIKRGGGGSGGGGSGGGGS DVQLVESGGGLVQPGGSLRLSCAVSGYSITSDYAWNWIRQAPGKGLEWLGYISYRGRTSY NPSLKSRISITRDNSKNTFFLQLNSLRAEDTAVYYCARLGRGFRYWGQGTLVTVSSTTTPAP RPPTPAPTIASQPLSLRPEACRPAAGGAVHTRGLDFACDFWVLVVVGGVLACYSLLVTVAFI IFWVRSKRSRLLHSDYMNMTPRRPGPTRKHYQPYAPPRDFAAYRSRVKFSRSADAPAYQQ GQNQLYNELNLGRREEYDVLDKRRGRDPEMGGKPQRRKNPQEGLYNELQKDKMAEAYS EIGMKGERRRGKGHDGLYQGLSTATKDTYDALHMQALPPR |
| Antibody M15-BBZ | 62 | DIQMTQSPSSLSASVGDRVTITCHASQDINVNIGWLQQKPGKAFKGLIYHGKNLEDGVPSR FSGSGSGTDFTLTISSLQPEDFATYYCNQYENNPITFGQGTKVEIKRGGGGSGGGGSGGGGS DVQLVESGGGLVQPGGSLRLSCAVSGYSITSDYAWNWIRQAPGKGLEWLGYISYRGRTSY NPSLKSRISITRDNSKNTFFLQLNSLRAEDTAVYYCARLGRGFRYWGQGTLVTVSSTTTPAP RPPTPAPTIASQPLSLRPEACRPAAGGAVHTRGLDFACDIYIWAPLAGTCGVLLLSLVITLYC KRGRKKLLYIFKQPFMRPVQTTQEEDGCSCRFPEEEEGGCELRVKFSRSADAPAYKQGQNQ LYNELNLGRREEYDVLDKRRGRDPEMGGKPRRKNPQEGLYNELQKDKMAEAYSEIGMKG ERRRGKGHDGLYQGLSTATKDTYDALHMQALPPR |
| Antibody M15-28BBZ | 63 | DIQMTQSPSSLSASVGDRVTITCHASQDINVNIGWLQQKPGKAFKGLIYHGKNLEDGVPSR FSGSGSGTDFTLTISSLQPEDFATYYCNQYENNPITFGQGTKVEIKRGGGGSGGGGSGGGGS DVQLVESGGGLVQPGGSLRLSCAVSGYSITSDYAWNWIRQAPGKGLEWLGYISYRGRTSY NPSLKSRISITRDNSKNTFFLQLNSLRAEDTAVYYCARLGRGFRYWGQGTLVTVSSTTTPAP RPPTPAPTIASQPLSLRPEACRPAAGGAVHTRGLDFACDFWVLVVVGGVLACYSLLVTVAFI IFWVRSKRSRLLHSDYMNMTPRRPGPTRKHYQPYAPPRDFAAYRSKRGRKKLLYIFKQPF MRPVQTTQEEDGCSCRFPEEEEGGCELRVKFSRSADAPAYQQGQNQLYNELNLGRREEYD VLDKRRGRDPEMGGKPQRRKNPQEGLYNELQKDKMAEAYSEIGMKGERRRGKGHDGLY QGLSTATKDTYDALHMQALPPR |

(continued)

| Name | SEQ ID NO: | Sequences |
|---|---|---|
| Antibody M25-28Z | 64 | DIQMTQSPSSLSASVGDRVTITCHASQDINVNIGWLQQKPGKAFKGLIYHGKNLEDGVPSRFSGSGSGTDFTLTISSLQPEDFATYYCNQYENIPLTFGQGTKVEIKRGGGGSGGGGSGGGGSDVQLVESGGGLVQPGGSLRLSCAVSGYSITSDYAWNWIRQAPGKGLEWLGYISYRGRTRYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |
| Antibody M25-BBZ | 65 | DIQMTQSPSSLSASVGDRVTITCHASQDINVNIGWLQQKPGKAFKGLIYHGKNLEDGVPSRFSGSGSGTDFTLTISSLQPEDFATYYCNQYENIPLTFGQGTKVEIKRGGGGSGGGGSGGGGSDVQLVESGGGLVQPGGSLRLSCAVSGYSITSDYAWNWIRQAPGKGLEWLGYISYRGRTRYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYKQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| Antibody M25-28BBZ | 66 | DIQMTQSPSSLSASVGDRVTITCHASQDINVNIGWLQQKPGKAFKGLIYHGKNLEDGVPSRFSGSGSGTDFTLTISSLQPEDFATYYCNQYENIPLTFGQGTKVEIKRGGGGSGGGGSGGGGSDVQLVESGGGLVQPGGSLRLSCAVSGYSITSDYAWNWIRQAPGKGLEWLGYISYRGRTRYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSKRGRKKLLYIFKQPFMRPVQTTQEEDGCSCRFPEEEEGGCELRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |

(continued)

| Name | SEQ ID NO: | Sequences |
|---|---|---|
| Antibody M26-28Z | 67 | DIQMTQSPSSLSASVGDRVTITCHASQDINVNIGWLQQKPGKAFKGLIYHGKNLEDGVPSR<br>FSGSGSGTDFTLTISSLQPEDFATYYCNQYENIPLTFGQGTKVEIKRGGGGSGGGGSGGGGS<br>DVQLVESGGGLVQPGGSLRLSCAVSGYSITSDYAWNWIRQAPGKGLEWLGYISYRGRTQY<br>NPSLKSRISITRDNSKNTFFLQLNSLRAEDTAVYYCARLGRGFRYWGQGTLVTVSSTTTPAP<br>RPPTPAPTIASQPLSLRPEACRPAAGGAVHTRGLDFACDFWVLVVVGGVLACYSLLVTVAFI<br>IFWVRSKRSRLLHSDYMNMTPRRPGPTRKHYQPYAPPRDFAAYRSRVKFSRSADAPAYQQ<br>GQNQLYNELNLGRREEYDVLDKRRGRDPEMGGKPQRRKNPQEGLYNELQKDKMAEAYS<br>EIGMKGERRRGKGHDGLYQGLSTATKDTYDALHMQALPPR |
| Antibody M26-BBZ | 68 | DIQMTQSPSSLSASVGDRVTITCHASQDINVNIGWLQQKPGKAFKGLIYHGKNLEDGVPSR<br>FSGSGSGTDFTLTISSLQPEDFATYYCNQYENIPLTFGQGTKVEIKRGGGGSGGGGSGGGGS<br>DVQLVESGGGLVQPGGSLRLSCAVSGYSITSDYAWNWIRQAPGKGLEWLGYISYRGRTQY<br>NPSLKSRISITRDNSKNTFFLQLNSLRAEDTAVYYCARLGRGFRYWGQGTLVTVSSTTTPAP<br>RPPTPAPTIASQPLSLRPEACRPAAGGAVHTRGLDFACDIYIWAPLAGTCGVLLLSLVITLYC<br>KRGRKKLLYIFKQPFMRPVQTTQEEDGCSCRFPEEEEGGCELRVKFSRSADAPAYKQGQNQ<br>LYNELNLGRREEYDVLDKRRGRDPEMGGKPRRKNPQEGLYNELQKDKMAEAYSEIGMKG<br>ERRRGKGHDGLYQGLSTATKDTYDALHMQALPPR |
| Antibody M26-28BBZ | 69 | DIQMTQSPSSLSASVGDRVTITCHASQDINVNIGWLQQKPGKAFKGLIYHGKNLEDGVPSR<br>FSGSGSGTDFTLTISSLQPEDFATYYCNQYENIPLTFGQGTKVEIKRGGGGSGGGGSGGGGS<br>DVQLVESGGGLVQPGGSLRLSCAVSGYSITSDYAWNWIRQAPGKGLEWLGYISYRGRTQY<br>NPSLKSRISITRDNSKNTFFLQLNSLRAEDTAVYYCARLGRGFRYWGQGTLVTVSSTTTPAP<br>RPPTPAPTIASQPLSLRPEACRPAAGGAVHTRGLDFACDFWVLVVVGGVLACYSLLVTVAFI<br>IFWVRSKRSRLLHSDYMNMTPRRPGPTRKHYQPYAPPRDFAAYRSKRGRKKLLYIFKQPF<br>MRPVQTTQEEDGCSCRFPEEEEGGCELRVKFSRSADAPAYQQGQNQLYNELNLGRREEYD<br>VLDKRRGRDPEMGGKPQRRKNPQEGLYNELQKDKMAEAYSEIGMKGERRRGKGHDGLY<br>QGLSTATKDTYDALHMQALPPR |

(continued)

| Name | SEQ ID NO: | Sequences |
|---|---|---|
| Antibody S7-28Z | 70 | DIQMTQSPSSLSASVGDRVTITCHASQDINVNIGWLQQKPGKAFKGLIYHGTNLEDGVPSRFSGSGSGTDFTLTISSLQPEDFATYYCNQYENNPITFGQGTKVEIKRGGGGSGGGGSGGGGSDVQLVESGGGLVQPGGSLRLSCAVSGYSITSDYAWNWIRQAPGKGLEWLGYISYRGRTSYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |
| Antibody S7-BBZ | 71 | DIQMTQSPSSLSASVGDRVTITCHASQDINVNIGWLQQKPGKAFKGLIYHGTNLEDGVPSRFSGSGSGTDFTLTISSLQPEDFATYYCNQYENNPITFGQGTKVEIKRGGGGSGGGGSGGGGSDVQLVESGGGLVQPGGSLRLSCAVSGYSITSDYAWNWIRQAPGKGLEWLGYISYRGRTSYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYKQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| Antibody S7-28BBZ | 72 | DIQMTQSPSSLSASVGDRVTITCHASQDINVNIGWLQQKPGKAFKGLIYHGTNLEDGVPSRFSGSGSGTDFTLTISSLQPEDFATYYCNQYENNPITFGQGTKVEIKRGGGGSGGGGSGGGGSDVQLVESGGGLVQPGGSLRLSCAVSGYSITSDYAWNWIRQAPGKGLEWLGYISYRGRTSYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSKRGRKKLLYIFKQPFMRPVQTTQEEDGCSCRFPEEEEGGCELRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |

(continued)

| Name | SEQ ID NO: | Sequences |
|---|---|---|
| Antibody S8-28Z | 73 | DIQMTQSPSSLSASVGDRVTITCHASQDINVNIGWLQQKPGKSFKGLIYHGKNLEDGVPSRFSGSGSGTDFTLTISSLQPEDFATYYCNQYENNPITFGQGTKVEIKRGGGGSGGGGSGGGGSDVQLVESGGGLVQPGGSLRLSCAVSGYSITSDYAWNWIRQAPGKGLEWLGYISYRGRTSYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |
| Antibody S8-BBZ | 74 | DIQMTQSPSSLSASVGDRVTITCHASQDINVNIGWLQQKPGKSFKGLIYHGKNLEDGVPSRFSGSGSGTDFTLTISSLQPEDFATYYCNQYENNPITFGQGTKVEIKRGGGGSGGGGSGGGGSDVQLVESGGGLVQPGGSLRLSCAVSGYSITSDYAWNWIRQAPGKGLEWLGYISYRGRTSYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYKQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| Antibody S8-28BBZ | 75 | DIQMTQSPSSLSASVGDRVTITCHASQDINVNIGWLQQKPGKSFKGLIYHGKNLEDGVPSRFSGSGSGTDFTLTISSLQPEDFATYYCNQYENNPITFGQGTKVEIKRGGGGSGGGGSGGGGSDVQLVESGGGLVQPGGSLRLSCAVSGYSITSDYAWNWIRQAPGKGLEWLGYISYRGRTSYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSKRGRKKLLYIFKQPFMRPVQTTQEEDGCSCRFPEEEEGGCELRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |
| Antibody | 76 | DIQMTQSPSSLSASVGDRVTITCHASQDINTNIGWLQQKPGKAFKGLIYHGKNLEDGVPSR |

(continued)

| Name | SEQ ID NO: | Sequences |
|---|---|---|
| S17-28Z | | FSGSGSGTDFTLTISSLQPEDFATYYCNQYENNPLTFGQGTKVEIKRGGGGSGGGGSGGGGSDVQLVESGGGLVQPGGSLRLSCAVSGYSITSDYAWNWIRQAPGKGLEWLGYISYRGRTQYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |
| Antibody S17-BBZ | 77 | DIQMTQSPSSLSASVGDRVTITCHASQDINTNIGWLQQKPGKAFKGLIYHGKNLEDGVPSRFSGSGSGTDFTLTISSLQPEDFATYYCNQYENNPLTFGQGTKVEIKRGGGGSGGGGSGGGGSDVQLVESGGGLVQPGGSLRLSCAVSGYSITSDYAWNWIRQAPGKGLEWLGYISYRGRTQYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYKQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| Antibody S17-28BBZ | 78 | DIQMTQSPSSLSASVGDRVTITCHASQDINTNIGWLQQKPGKAFKGLIYHGKNLEDGVPSRFSGSGSGTDFTLTISSLQPEDFATYYCNQYENNPLTFGQGTKVEIKRGGGGSGGGGSGGGGSDVQLVESGGGLVQPGGSLRLSCAVSGYSITSDYAWNWIRQAPGKGLEWLGYISYRGRTQYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSKRGRKKLLYIFKQPFMRPVQTTQEEDGCSCRFPEEEEGGCELRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |

(continued)

| Name | SEQ ID NO: | Sequences |
|---|---|---|
| Antibody S22-28Z | 79 | DIQMTQSPSSLSASVGDRVTITCHASQDINVNIGWLQQKPGKAFKGLIYHGTNLEDGVPSRFSGSGSGTDFTLTISSLQPEDFATYYCNQYENNPLTFGQGTKVEIKRGGGGSGGGGSGGGGSDVQLVESGGGLVQPGGSLRLSCAVSGYSITSDYAWNWIRQAPGKGLEWLGYISYRGRTRYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |
| Antibody S22-BBZ | 80 | DIQMTQSPSSLSASVGDRVTITCHASQDINVNIGWLQQKPGKAFKGLIYHGTNLEDGVPSRFSGSGSGTDFTLTISSLQPEDFATYYCNQYENNPLTFGQGTKVEIKRGGGGSGGGGSGGGGSDVQLVESGGGLVQPGGSLRLSCAVSGYSITSDYAWNWIRQAPGKGLEWLGYISYRGRTRYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYKQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| Antibody S22-28BBZ | 81 | DIQMTQSPSSLSASVGDRVTITCHASQDINVNIGWLQQKPGKAFKGLIYHGTNLEDGVPSRFSGSGSGTDFTLTISSLQPEDFATYYCNQYENNPLTFGQGTKVEIKRGGGGSGGGGSGGGGSDVQLVESGGGLVQPGGSLRLSCAVSGYSITSDYAWNWIRQAPGKGLEWLGYISYRGRTRYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSKRGRKKLLYIFKQPFMRPVQTTQEEDGCSCRFPEEEEGGCELRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |

(continued)

| Name | SEQ ID NO: | Sequences |
|---|---|---|
| Antibody S23-28Z | 82 | DIQMTQSPSSLSASVGDRVTITCHASQDINVNIGWLQQKPGKSFKGLIYHGKNLEDGVPSRFSGSGSGTDFTLTISSLQPEDFATYYCNQYENNPLTFGQGTKVEIKRGGGGSGGGGSGGGGSDVQLVESGGGLVQPGGSLRLSCAVSGYSITSDYAWNWIRQAPGKGLEWLGYISYRGRTRYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |
| Antibody S23-BBZ | 83 | DIQMTQSPSSLSASVGDRVTITCHASQDINVNIGWLQQKPGKSFKGLIYHGKNLEDGVPSRFSGSGSGTDFTLTISSLQPEDFATYYCNQYENNPLTFGQGTKVEIKRGGGGSGGGGSGGGGSDVQLVESGGGLVQPGGSLRLSCAVSGYSITSDYAWNWIRQAPGKGLEWLGYISYRGRTRYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYKQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| Antibody S23-28BBZ | 84 | DIQMTQSPSSLSASVGDRVTITCHASQDINVNIGWLQQKPGKSFKGLIYHGKNLEDGVPSRFSGSGSGTDFTLTISSLQPEDFATYYCNQYENNPLTFGQGTKVEIKRGGGGSGGGGSGGGGSDVQLVESGGGLVQPGGSLRLSCAVSGYSITSDYAWNWIRQAPGKGLEWLGYISYRGRTRYNPSLKSRISITRDNSKNTFFLQLNSLRAEDTAVYYCARLGRGFRYWGQGTLVTVSSTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSKRGRKKLLYIFKQPFMRPVQTTQEEDGCSCRFPEEEEGGCELRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |

(continued)

| Name | SEQ ID NO: | Sequences |
|---|---|---|
| Antibody S29-28Z | 85 | DIQMTQSPSSLSASVGDRVTITCHASQDINVNIGWLQQKPGKAFKGLIYHGKNLEDGVPSR FSGSGSGTDFTLTISSLQPEDFATYYCNQYENFPLTFGQGTKVEIKRGGGGSGGGGSGGGGS DVQLVESGGGLVQPGGSLRLSCAVSGYSITSDYAWNWIRQAPGKGLEWLGYISYRGRTRY NPSLKSRISITRDNSKNTFFLQLNSLRAEDTAVYYCARLGRGFRYWGQGTLVTVSSTTTPAP RPPTPAPTIASQPLSLRPEACRPAAGGAVHTRGLDFACDFWVLVVVGGVLACYSLLVTVAFI IFWVRSKRSRLLHSDYMNMTPRRPGPTRKHYQPYAPPRDFAAYRSRVKFSRSADAPAYQQ GQNQLYNELNLGRREEYDVLDKRRGRDPEMGGKPQRRKNPQEGLYNELQKDKMAEAYS EIGMKGERRRGKGHDGLYQGLSTATKDTYDALHMQALPPR |
| Antibody S29-BBZ | 86 | DIQMTQSPSSLSASVGDRVTITCHASQDINVNIGWLQQKPGKAFKGLIYHGKNLEDGVPSR FSGSGSGTDFTLTISSLQPEDFATYYCNQYENFPLTFGQGTKVEIKRGGGGSGGGGSGGGGS DVQLVESGGGLVQPGGSLRLSCAVSGYSITSDYAWNWIRQAPGKGLEWLGYISYRGRTRY NPSLKSRISITRDNSKNTFFLQLNSLRAEDTAVYYCARLGRGFRYWGQGTLVTVSSTTTPAP RPPTPAPTIASQPLSLRPEACRPAAGGAVHTRGLDFACDIYIWAPLAGTCGVLLLSLVITLYC KRGRKKLLYIFKQPFMRPVQTTQEEDGCSCRFPEEEEGGCELRVKFSRSADAPAYKQGQNQ |
| | | LYNELNLGRREEYDVLDKRRGRDPEMGGKPRRKNPQEGLYNELQKDKMAEAYSEIGMKG ERRRGKGHDGLYQGLSTATKDTYDALHMQALPPR |
| Antibody S29-28BBZ | 87 | DIQMTQSPSSLSASVGDRVTITCHASQDINVNIGWLQQKPGKAFKGLIYHGKNLEDGVPSR FSGSGSGTDFTLTISSLQPEDFATYYCNQYENFPLTFGQGTKVEIKRGGGGSGGGGSGGGGS DVQLVESGGGLVQPGGSLRLSCAVSGYSITSDYAWNWIRQAPGKGLEWLGYISYRGRTRY NPSLKSRISITRDNSKNTFFLQLNSLRAEDTAVYYCARLGRGFRYWGQGTLVTVSSTTTPAP RPPTPAPTIASQPLSLRPEACRPAAGGAVHTRGLDFACDFWVLVVVGGVLACYSLLVTVAFI IFWVRSKRSRLLHSDYMNMTPRRPGPTRKHYQPYAPPRDFAAYRSKRGRKKLLYIFKQPF MRPVQTTQEEDGCSCRFPEEEEGGCELRVKFSRSADAPAYQQGQNQLYNELNLGRREEYD VLDKRRGRDPEMGGKPQRRKNPQEGLYNELQKDKMAEAYSEIGMKGERRRGKGHDGLY QGLSTATKDTYDALHMQALPPR |

## Claims

1. A method for treating a tumor, **characterized by** administering an immune effector cell and a PARP inhibitor to an individual suffering from a tumor, wherein the immune effector cell expresses a receptor recognizing a tumor antigen.

2. A method for reducing the growth, survival, or viability, or all the above of cancer cells, **characterized by** administering an immune effector cell and a PARP inhibitor to an individual suffering from a tumor, wherein the immune effector cell expresses a receptor recognizing a tumor antigen.

3. The method of claim 1 or 2, **characterized in that** the PARP inhibitor is any one selected from the group consisting of: talazoparib, niraparib, olaparib, rucaparib, niraparib, pamiparib, fluzoparib, mefuparib, and simmiparib; preferably the PARP inhibitor is olaparib; and further preferably the therapeutic effect of the immune effector cell and the PARP inhibitor is greater than that of either the immune effector cell or the PARP inhibitor used alone.

4. The method according to any one of claims 1-3, **characterized in that**:

   the method increases the CAR-T copy number of tumor tissue, and/or
   the method increases the expression level of IFN-γ in tumor tissue, and/or
   the method increases CD8+ T cell immune cell infiltration in tumor tissue, and/or
   the method increases CD45+ immune cell infiltration, and/or
   the method reduces CD11b+Ly6G+ MDSCs cell infiltration, and/or
   the method reduces CD31+ cell infiltration.

5. The method according to any one of claims 1-4, **characterized in that** the method does not cause a significant decrease in the expression of PD1, LAG3, and/or TIM3.

6. The method according to any one of claims 1-5, **characterized in that** the manner of administering an immune effector cell and a PARP inhibitor to an individual suffering from a tumor is any one selected from:

   (1) firstly administering the PARP inhibitor and then the immune effector cell,
   (2) simultaneously administering the immune effector cell and the PARP inhibitor, and
   (3) firstly administering the immune effector cell and then the PARP inhibitor.

7. The method according to any one of claims 1-6, **characterized in that** the receptor is selected from: chimeric antigen receptor (CAR), T cell receptor (TCR), T cell fusion protein (TFP), T cell antigen coupler (TAC), or a combination thereof.

8. The method according to any one of claims 1-7, **characterized in that** the tumor antigen is selected from EGFR or EGFRvIII.

9. The method according to claim 7 or 8, **characterized in that** the chimeric antigen receptor has:

   (i) an antibody or a fragment thereof specifically recognizing a tumor antigen, the transmembrane region of CD28 or CD8, the costimulatory signal domain of CD28, and CD3ζ; or
   (ii) an antibody or a fragment thereof specifically recognizing a tumor antigen, the transmembrane region of CD28 or CD8, the costimulatory signal domain of CD137, and CD3ζ; or
   (iii) an antibody or a fragment thereof specifically recognizing a tumor antigen, the transmembrane region of CD28 or CD8, the costimulatory signal domain of CD28, the costimulatory signal domain of CD137, and CD3ζ.

10. The method according to claim 9, **characterized in that**:

    the antibody specifically recognizing a tumor antigen is an antibody targeting EGFR or EGFRvIII,
    further preferably the antibody has an amino acid sequence selected from any one of the group consisting of: SEQ ID NOs: 11-29.

11. The method according to claim 9 or 10, **characterized in that** the chimeric antigen receptor has an amino acid sequence selected from any one of the group consisting of: SEQ ID NOs: 30-51, and SEQ ID NOs: 55-87.

**12.** The method according to any one of claims 1-11, **characterized in that** the tumor comprises:

breast cancer, colon cancer, rectal cancer, renal cell carcinoma, liver cancer, lung cancer, small intestine cancer, esophageal cancer, melanoma, bone cancer, pancreatic cancer, skin cancer, head and neck cancer, uterine cancer, ovarian cancer, rectal cancer, stomach cancer, testicular cancer,
fallopian tube cancer, endometrial cancer, cervical cancer, vagina cancer, thyroid cancer, parathyroid cancer, adrenal cancer, soft tissue sarcoma, urethral cancer, penile cancer, bladder cancer, ureter cancer, renal pelvis cancer, spinal tumor, glioma, pituitary adenoma, Kaposi's sarcoma, a combination and the metastatic foci thereof.

**13.** The method according to any one of claims 1-12, **characterized in that** the immune effector cell is selected from: T cell, B cell, natural killer (NK) cell, natural killer T (NKT) cell, mast cell, or bone marrow-derived phagocytic cell, or a combination thereof; preferably the immune effector cell is selected from autologous T cell, allogeneic T cell, or allogeneic NK cell; more preferably the T cell is autologous T cell.

**14.** The method according to any one of claims 1-13, **characterized in that** the PARP inhibitor is administered by oral administration, intraperitoneal administration and/or injection.

**15.** The method according to any one of claims 1-14, **characterized in that** lymphocyte clearance is not performed on the individual.

**16.** Use of an immune effector cell expressing a receptor recognizing a tumor antigen and a PARP inhibitor in the preparation of a medicament, wherein the medicament is used for treating a tumor or reducing the growth, survival or viability of a cancer cell.

**17.** Use of an immune effector cell expressing a receptor recognizing a tumor antigen in the preparation of a medicament, **characterized in that** the medicament comprises the cell and a PARP inhibitor, and is used for treating a tumor or reducing the growth, survival or viability of a cancer cell in a human patient, wherein the therapeutic effect of the immune effector cell and the PARP inhibitor is greater than that of either the immune effector cell or the PARP inhibitor used alone.

**18.** The use according to claim 16 or 17, **characterized in that** the PARP inhibitor comprises talazoparib, niraparib, olaparib, rucaparib, niraparib, pamiparib, mefuparib and/or simmiparib, and preferably the PARP inhibitor is olaparib.

**19.** The use according to claim 16 or 17, **characterized in that** the immune effector cell is a CAR-T cell, and preferably the CAR-T cell specifically recognizes EGFR or EGFRvIII.

**20.** A kit for treating a tumor, **characterized in that** the kit comprises:

1) an immune effector cell expressing a receptor recognizing a tumor antigen;
2) olaparib;
3) a container for containing the substances described in the above 1) and 2); and
4) administration instructions for using the kit to treat a tumor;

wherein the therapeutic effect of the immune effector cell and olaparib is greater than that of either the immune effector cell or olaparib used alone.

**21.** The kit according to claim 20, **characterized in that** the immune effector cell is a CAR-T cell; preferably the CAR-T cell specifically recognizes EGFR or EGFRvIII.

**22.** The use according to any one of claims 16-19 or the kit according to claim 20 or 21, **characterized in that** the chimeric antigen receptor has:

(i) an antibody or a fragment thereof specifically recognizing a tumor antigen, the transmembrane region of CD28 or CD8, the costimulatory signal domain of CD28, and CD3ζ; or
(ii) an antibody or a fragment thereof specifically recognizing a tumor antigen, the transmembrane region of CD28 or CD8, the costimulatory signal domain of CD137, and CD3ζ; or
(iii) an antibody or a fragment thereof specifically recognizing a tumor antigen, the transmembrane region of CD28 or CD8, the costimulatory signal domain of CD28, the costimulatory signal domain of CD137, and CD3ζ.

**23.** The use according to any one of claims 16-19 or the kit according to claim 20 or 21, **characterized in that**:

the antibody specifically recognizing a tumor antigen is an antibody targeting EGFR or EGFRvIII, preferably the antibody has an amino acid sequence selected from any one of the group consisting of: SEQ ID NOs: 11-29.

**24.** The use according to any one of claims 16-19 or the kit according to claim 20 or 21, **characterized in that** the chimeric antigen receptor has an amino acid sequence selected from any one of the group consisting of: SEQ ID NOs: 30-51, and SEQ ID NOs: 55-87.

**25.** The use according to any one of claims 16-19 or the kit according to claim 20 or 21, **characterized in that** the tumor comprises: breast cancer, colon cancer, rectal cancer, renal cell carcinoma, liver cancer, lung cancer, small intestine cancer, esophageal cancer, melanoma, bone cancer, pancreatic cancer, skin cancer, head and neck cancer, uterine cancer, ovarian cancer, rectal cancer, stomach cancer, testicular cancer, fallopian tube cancer, endometrial cancer, cervical cancer, vagina cancer, thyroid cancer, parathyroid cancer, adrenal cancer, soft tissue sarcoma, urethral cancer, penile cancer, bladder cancer, ureter cancer, renal pelvis cancer, spinal tumor, glioma, pituitary adenoma, Kaposi's sarcoma, a combination and the metastatic foci thereof.

**26.** The use according to any one of claims 16-19 or the kit according to claim 20 or 21, **characterized in that** the immune effector cell is selected from: T cell, B cell, natural killer (NK) cell, natural killer T (NKT) cell, mast cell, or bone marrow-derived phagocytic cell, or a combination thereof; preferably the immune effector cell is selected from autologous T cell, allogeneic T cell, or allogeneic NK cell; more preferably the T cell is autologous T cell.

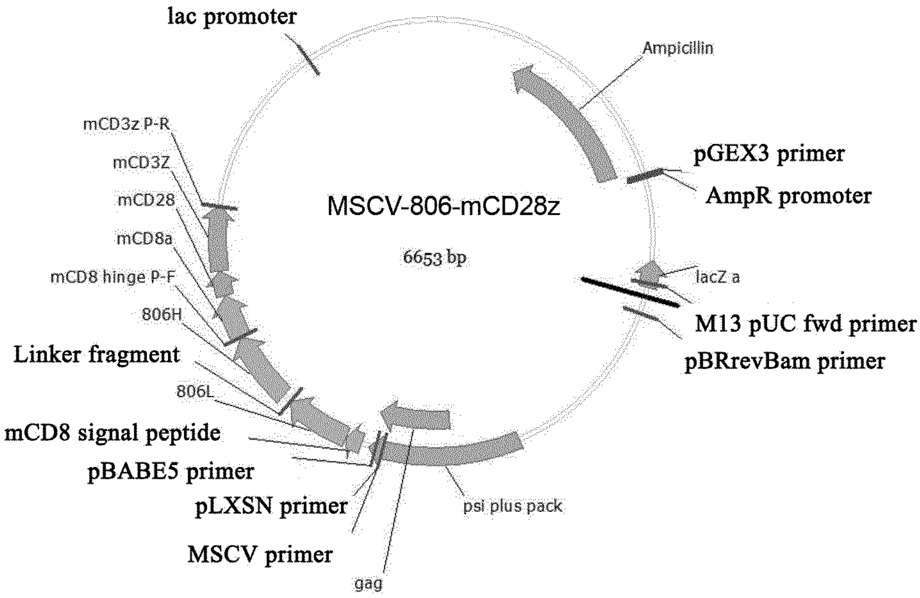
lac promoter
Ampicillin
mCD3z P-R
mCD3Z
mCD28
mCD8a
mCD8 hinge P-F
806H
Linker fragment
806L
mCD8 signal peptide
pBABE5 primer
pLXSN primer
MSCV primer
gag
psi plus pack
MSCV-806-mCD28z
6653 bp
pGEX3 primer
AmpR promoter
lacZ a
M13 pUC fwd primer
pBRrevBam primer

Fig. 1A

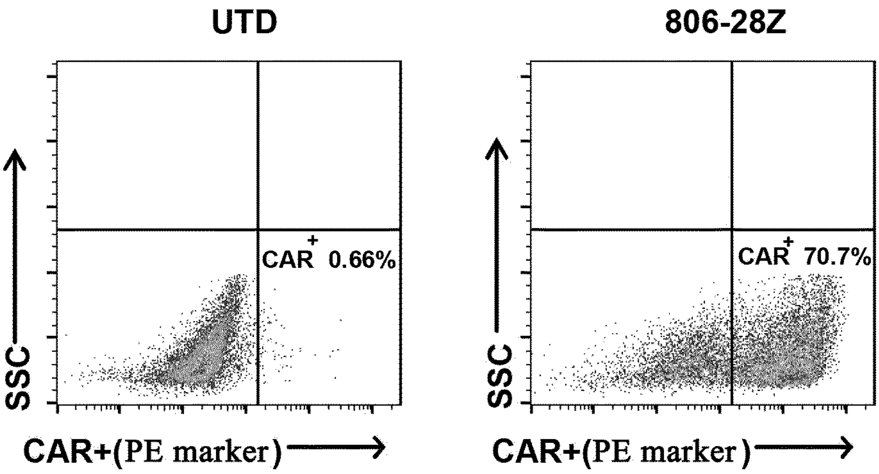
UTD
806-28Z
CAR+ 0.66%
CAR+ 70.7%
SSC
SSC
CAR+(PE marker)
CAR+(PE marker)

Fig. 1B

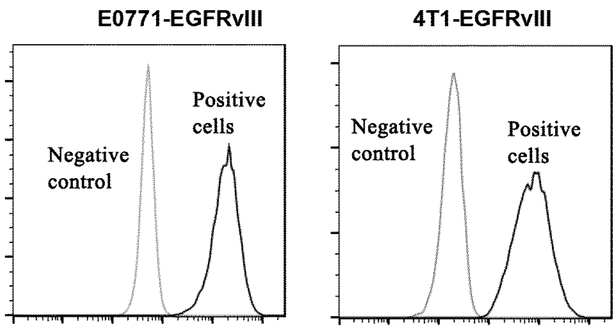
E0771-EGFRvIII
4T1-EGFRvIII
Positive
cells
Negative
control
Negative
control
Positive
cells

Fig. 2A

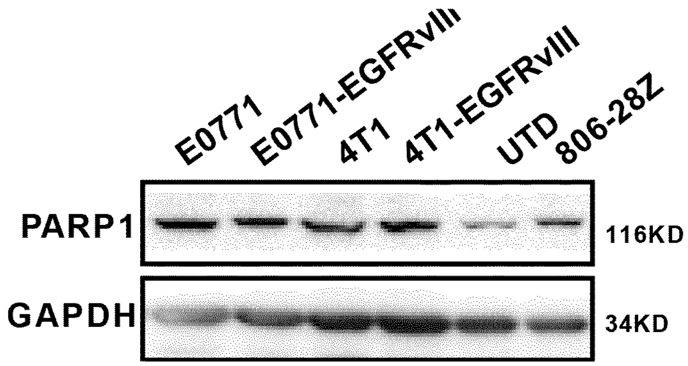
E0771
E0771-EGFRvIII
4T1
4T1-EGFRvIII
UTD
806-28Z
PARP1
116KD
GAPDH
34KD

Fig. 2B

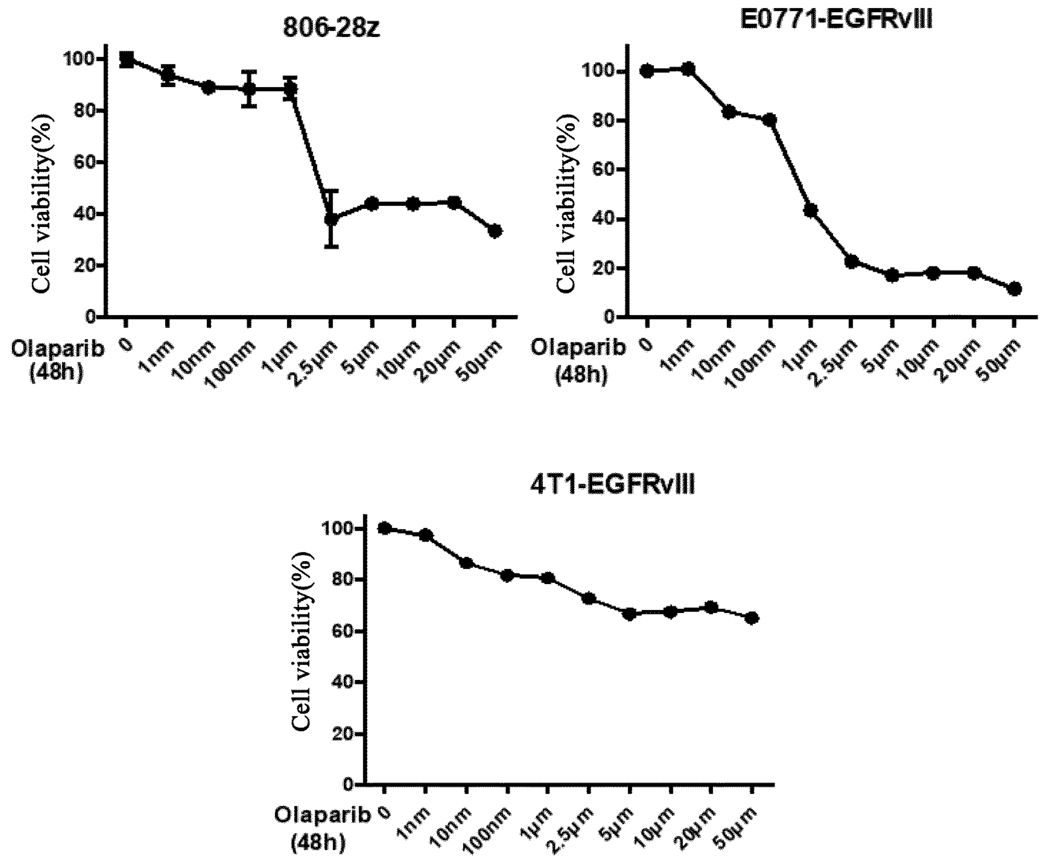
806-28z
E0771-EGFRvIII
4T1-EGFRvIII
Cell viability(%)
Olaparib (48h)
0
1nm
10nm
100nm
1μm
2.5μm
5μm
10μm
20μm
50μm

Fig. 2C

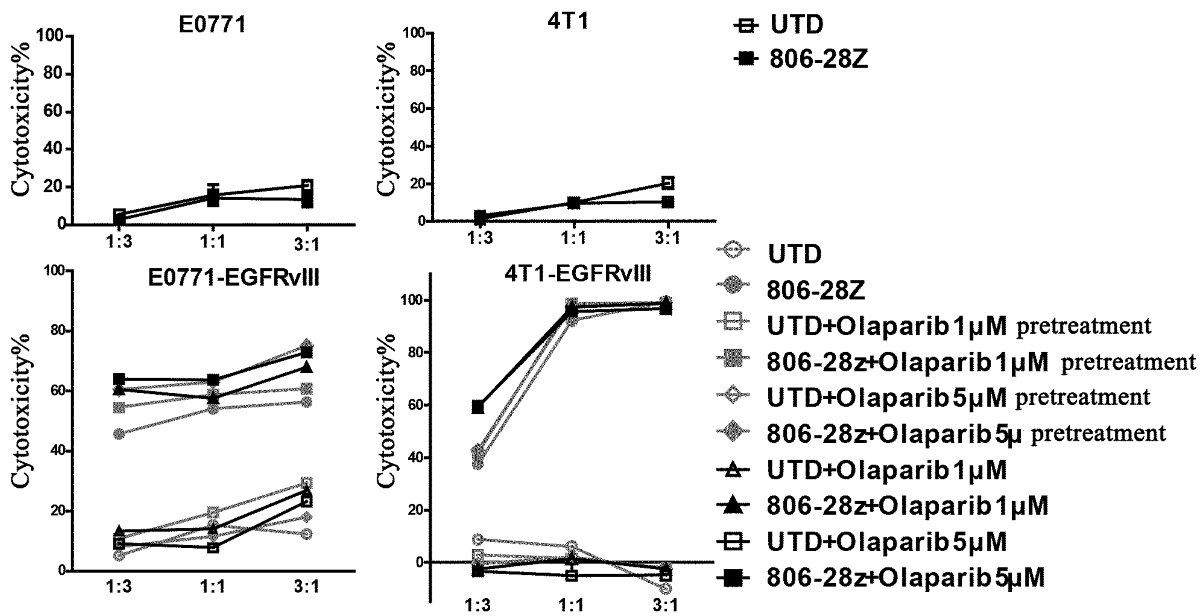
E0771
4T1
E0771-EGFRvIII
4T1-EGFRvIII
Cytotoxicity%
1:3
1:1
3:1
UTD
806-28Z
UTD
806-28Z
UTD+Olaparib1μM pretreatment
806-28z+Olaparib1μM pretreatment
UTD+Olaparib5μM pretreatment
806-28z+Olaparib5μ pretreatment
UTD+Olaparib1μM
806-28z+Olaparib1μM
UTD+Olaparib5μM
806-28z+Olaparib5μM

Fig. 3A

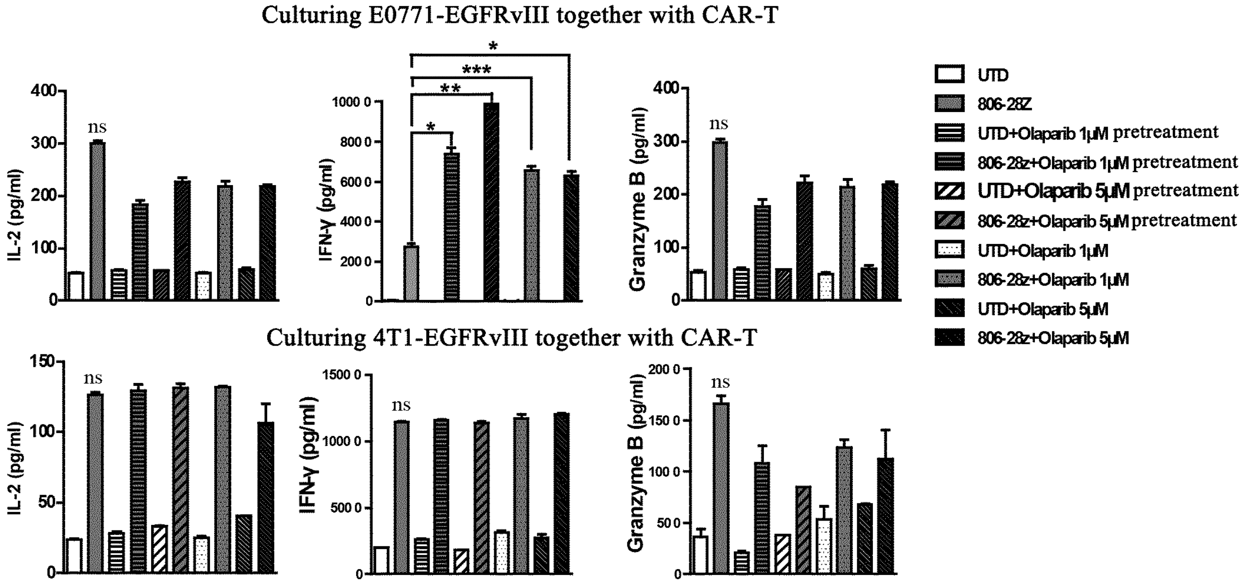
Culturing E0771-EGFRvIII together with CAR-T
Culturing 4T1-EGFRvIII together with CAR-T
IL-2 (pg/ml)
IFN-γ (pg/ml)
Granzyme B (pg/ml)
ns
UTD
806-28Z
UTD+Olaparib 1μM pretreatment
806-28z+Olaparib 1μM pretreatment
UTD+Olaparib 5μM pretreatment
806-28z+Olaparib 5μM pretreatment
UTD+Olaparib 1μM
806-28z+Olaparib 1μM
UTD+Olaparib 5μM
806-28z+Olaparib 5μM

Fig. 3B

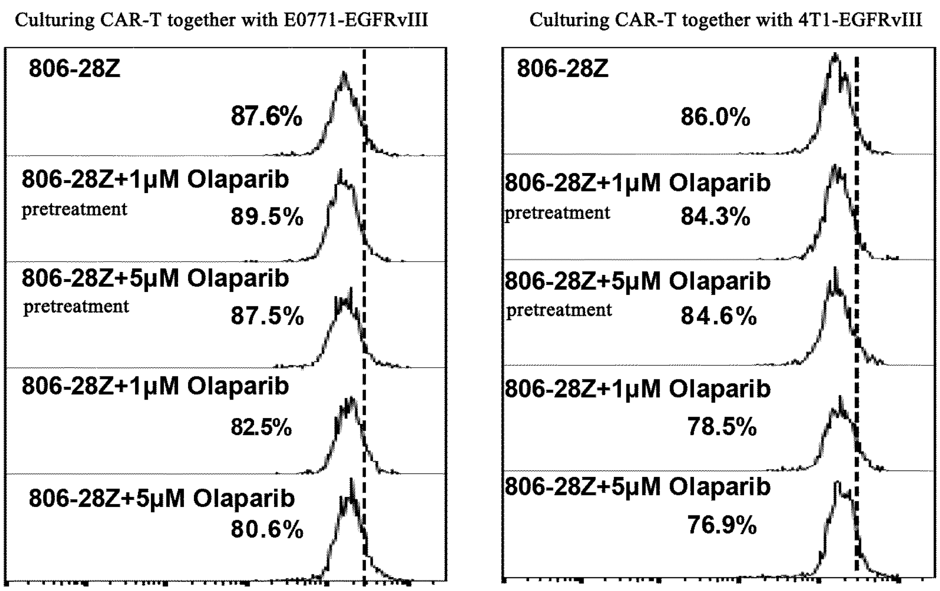
Culturing CAR-T together with E0771-EGFRvIII
Culturing CAR-T together with 4T1-EGFRvIII
806-28Z
87.6%
806-28Z+1μM Olaparib pretreatment
89.5%
806-28Z+5μM Olaparib pretreatment
87.5%
806-28Z+1μM Olaparib
82.5%
806-28Z+5μM Olaparib
80.6%
806-28Z
86.0%
806-28Z+1μM Olaparib pretreatment
84.3%
806-28Z+5μM Olaparib pretreatment
84.6%
806-28Z+1μM Olaparib
78.5%
806-28Z+5μM Olaparib
76.9%

Fig. 4A

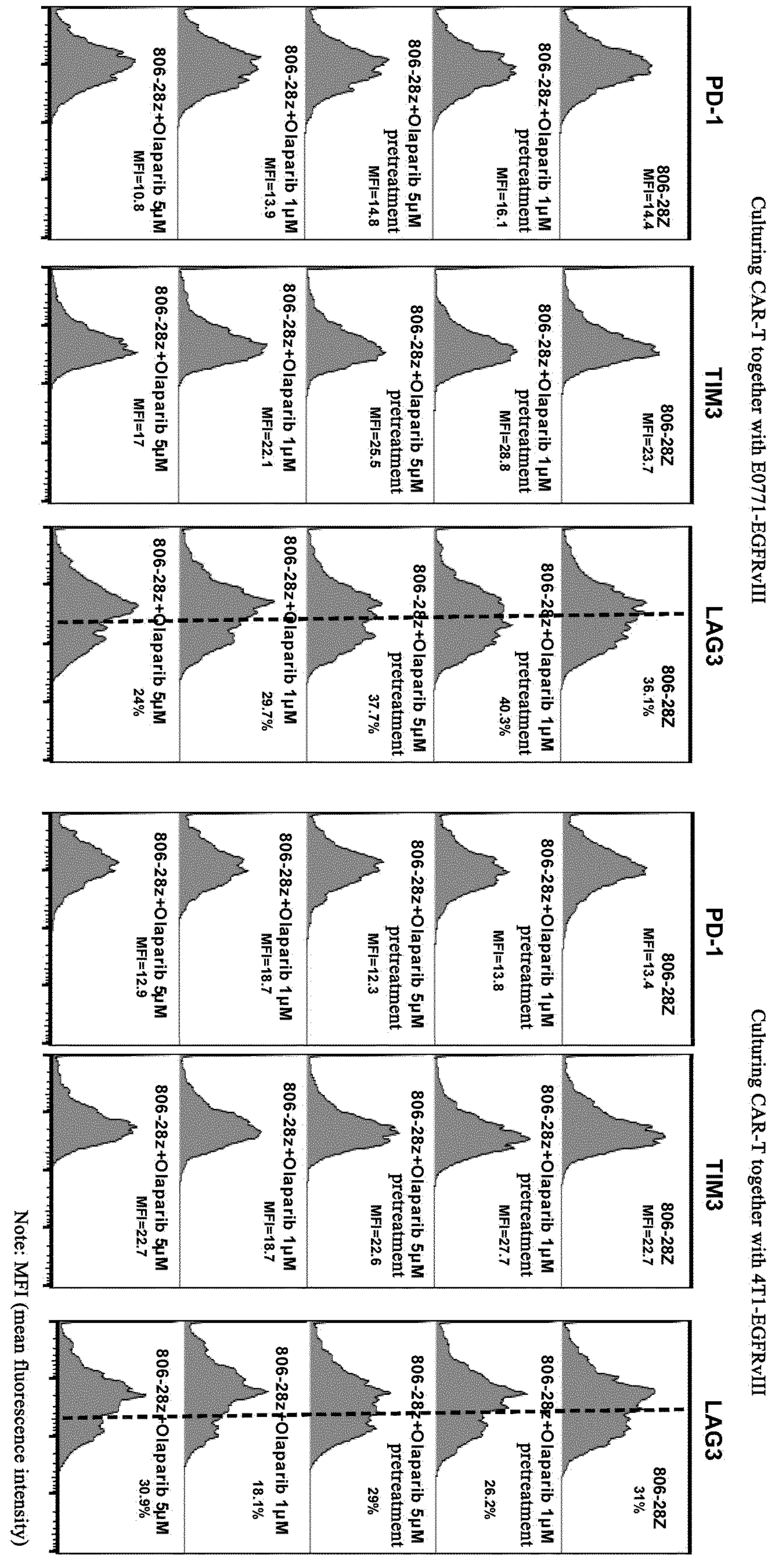
Culturing CAR-T together with E0771-EGFRvIII
PD-1
806-28Z MFI=14.4
806-28z+Olaparib 1µM pretreatment MFI=16.1
806-28z+Olaparib 5µM pretreatment MFI=14.8
806-28z+Olaparib 1µM MFI=13.9
806-28z+Olaparib 5µM MFI=10.8
TIM3
806-28Z MFI=23.7
806-28z+Olaparib 1µM pretreatment MFI=28.8
806-28z+Olaparib 5µM pretreatment MFI=25.5
806-28z+Olaparib 1µM MFI=22.1
806-28z+Olaparib 5µM MFI=17
LAG3
806-28Z 36.1%
806-28z+Olaparib 1µM pretreatment 40.3%
806-28z+Olaparib 5µM pretreatment 37.7%
806-28z+Olaparib 1µM 29.7%
806-28z+Olaparib 5µM 24%
Culturing CAR-T together with 4T1-EGFRvIII
PD-1
806-28Z MFI=13.4
806-28z+Olaparib 1µM pretreatment MFI=13.8
806-28z+Olaparib 5µM pretreatment MFI=12.3
806-28z+Olaparib 1µM MFI=18.7
806-28z+Olaparib 5µM MFI=12.9
TIM3
806-28Z MFI=22.7
806-28z+Olaparib 1µM pretreatment MFI=27.7
806-28z+Olaparib 5µM pretreatment MFI=22.6
806-28z+Olaparib 1µM MFI=18.7
806-28z+Olaparib 5µM MFI=22.7
LAG3
806-28Z 31%
806-28z+Olaparib 1µM pretreatment 26.2%
806-28z+Olaparib 5µM pretreatment 29%
806-28z+Olaparib 1µM 18.1%
806-28z+Olaparib 5µM 30.9%
Note: MFI (mean fluorescence intensity)

Fig. 4B

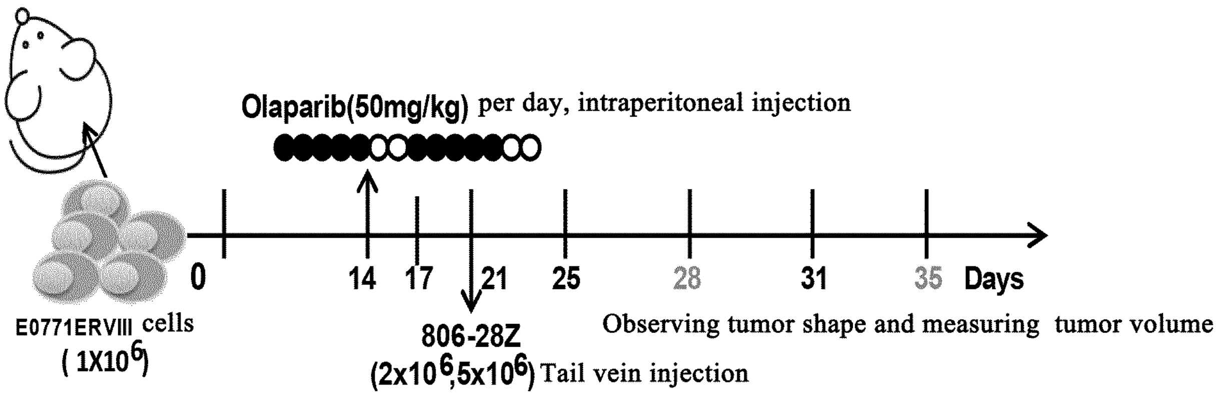


Fig. 5A

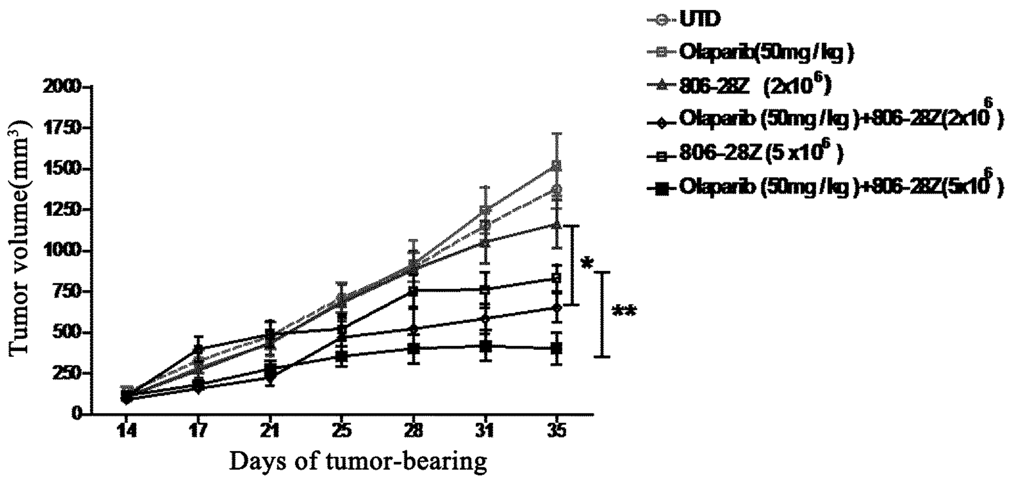


Fig. 5B

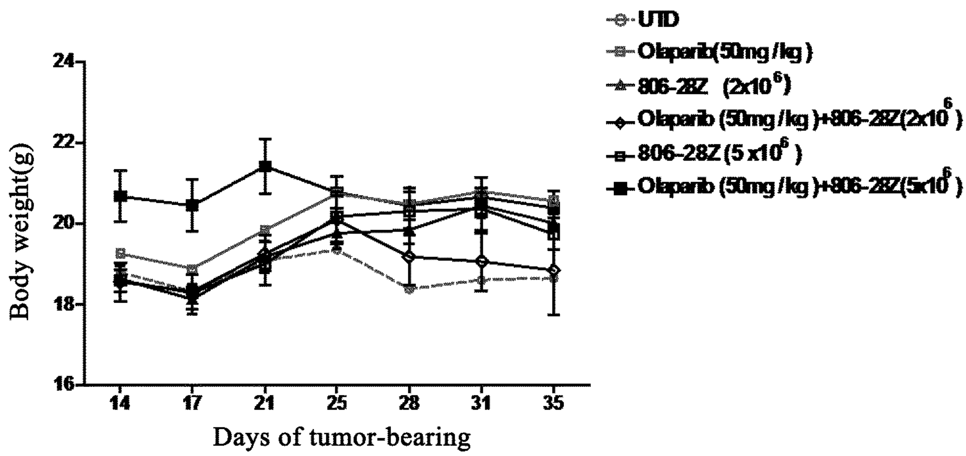


Fig. 5C

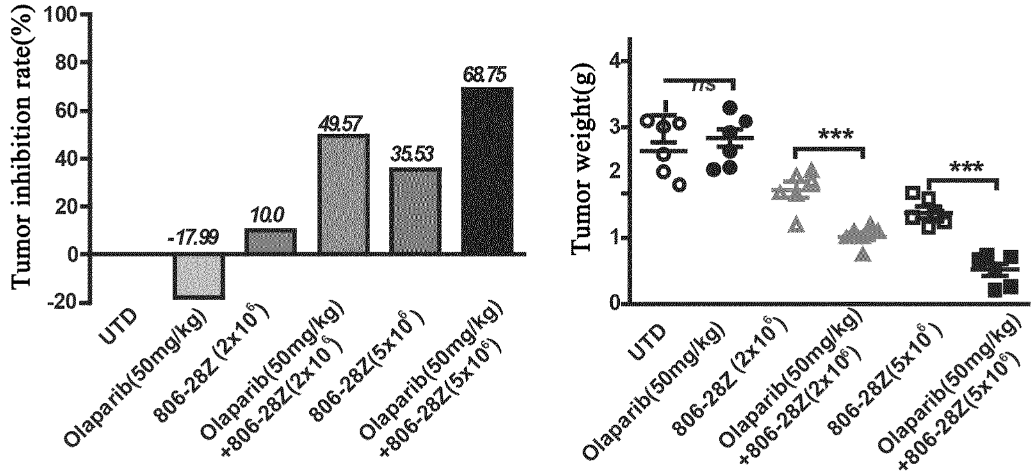
Tumor inhibition rate(%)
100
80
60
40
20
0
-20
-17.99
10.0
49.57
35.53
68.75
UTD
Olaparib(50mg/kg)
806-28Z (2x10⁶)
Olaparib(50mg/kg) +806-28Z(2x10⁶)
806-28Z(5x10⁶)
Olaparib(50mg/kg) +806-28Z(5x10⁶)
Tumor weight(g)
4
3
2
1
0
ns
***
***

Fig. 5D

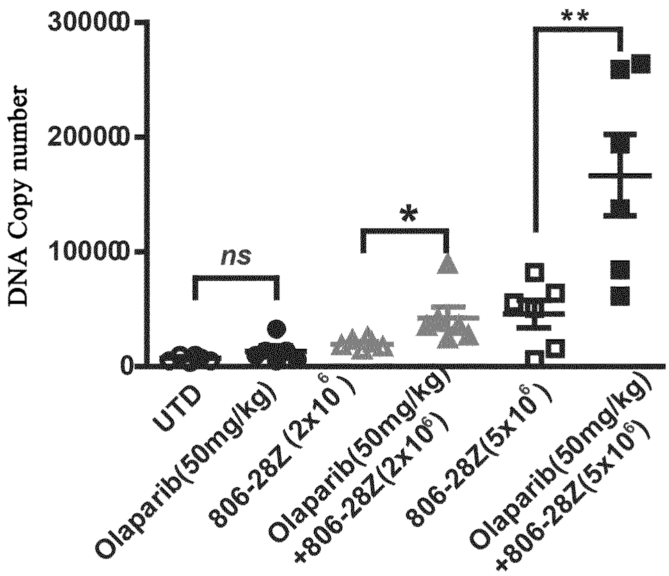
DNA Copy number
300000
200000
100000
0
ns
*
**
UTD
Olaparib(50mg/kg)
806-28Z (2x10⁶)
Olaparib(50mg/kg) +806-28Z(2x10⁶)
806-28Z(5x10⁶)
Olaparib(50mg/kg) +806-28Z(5x10⁶)

Fig. 5E

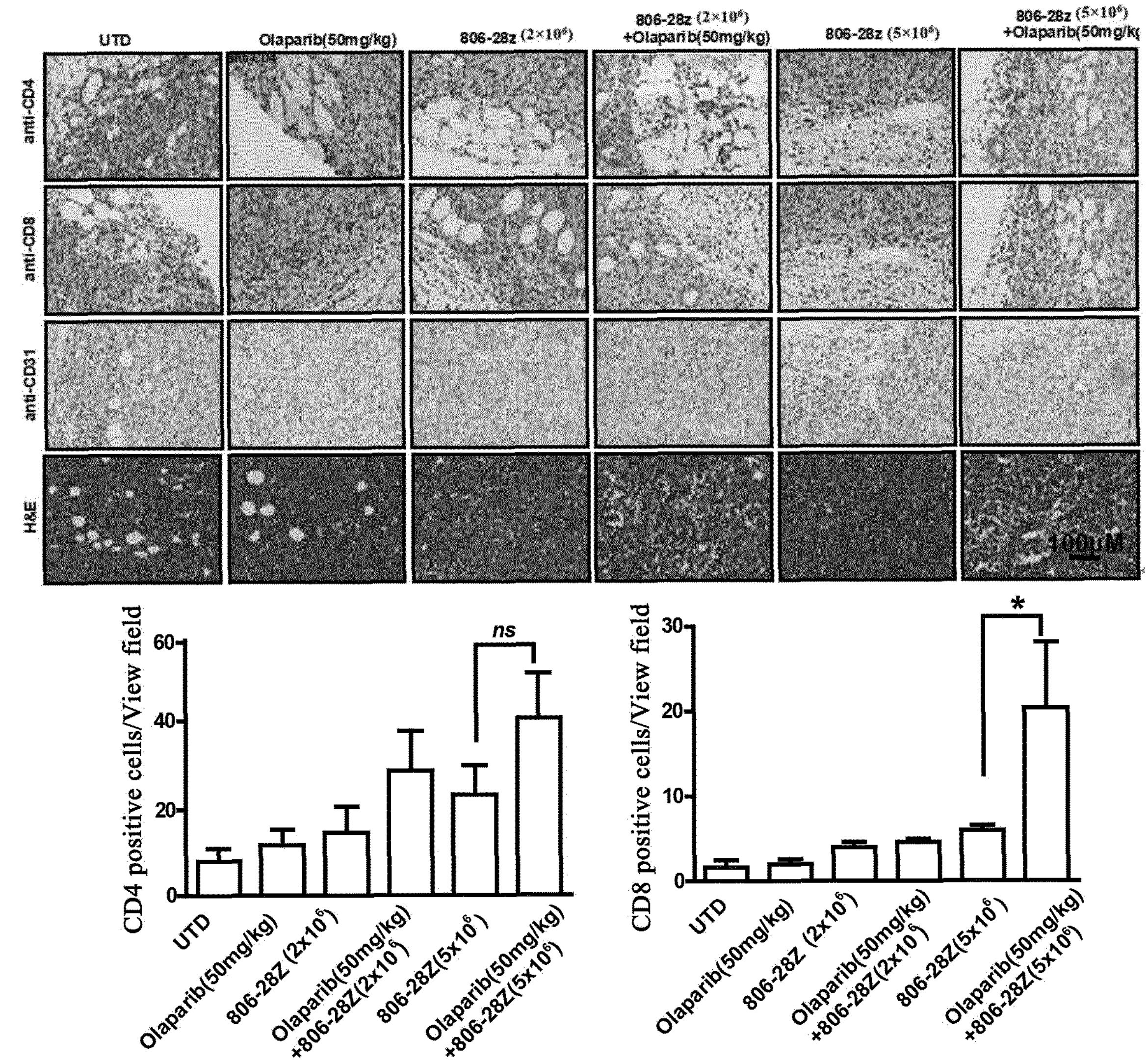
UTD
Olaparib(50mg/kg)
806-28z (2×10⁶)
806-28z (2×10⁶) +Olaparib(50mg/kg)
806-28z (5×10⁶)
806-28z (5×10⁶) +Olaparib(50mg/kg
anti-CD4
anti-CD8
anti-CD31
H&E
100μM
CD4 positive cells/View field
ns
CD8 positive cells/View field
*
UTD
Olaparib(50mg/kg)
806-28Z (2x10⁶)
Olaparib(50mg/kg) +806-28Z(2x10⁶)
806-28Z(5x10⁶)
Olaparib(50mg/kg) +806-28Z(5x10⁶)

Fig. 5F

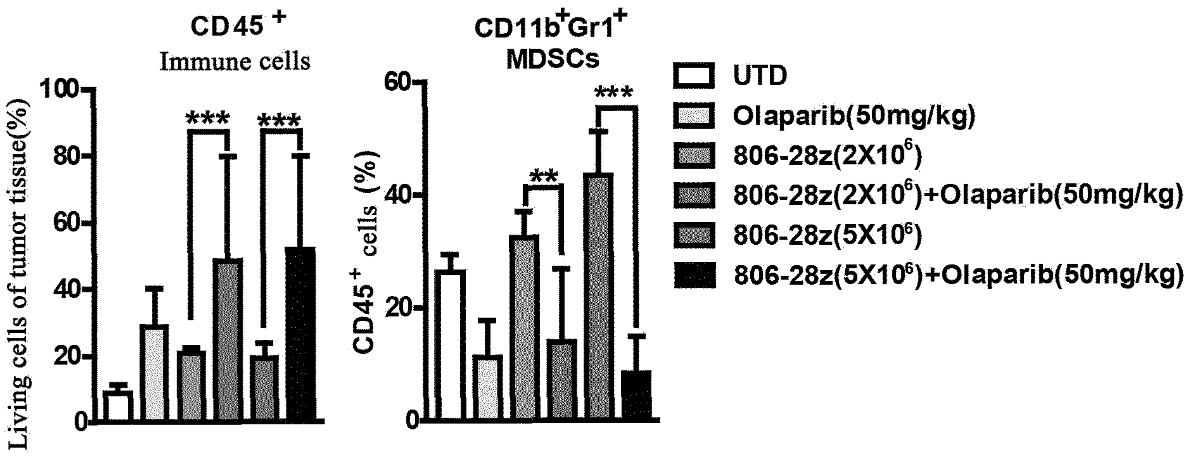
CD45 ⁺
Immune cells
Living cells of tumor tissue(%)
***
CD11b⁺Gr1⁺
MDSCs
CD45⁺ cells (%)
**
UTD
Olaparib(50mg/kg)
806-28z(2X10⁶)
806-28z(2X10⁶)+Olaparib(50mg/kg)
806-28z(5X10⁶)
806-28z(5X10⁶)+Olaparib(50mg/kg)

Fig. 5G

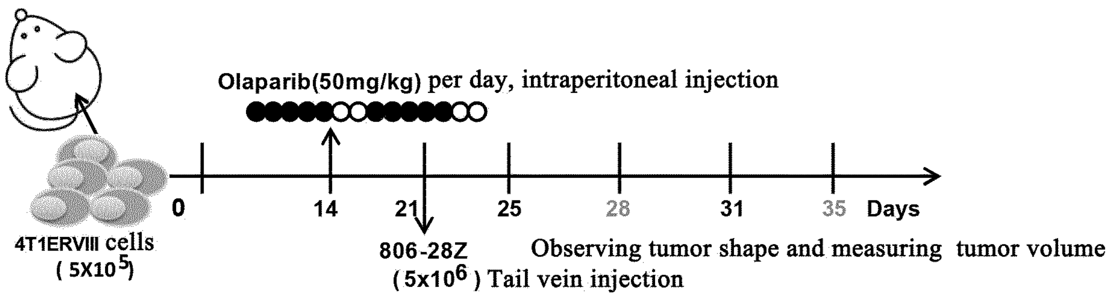


Fig.6A

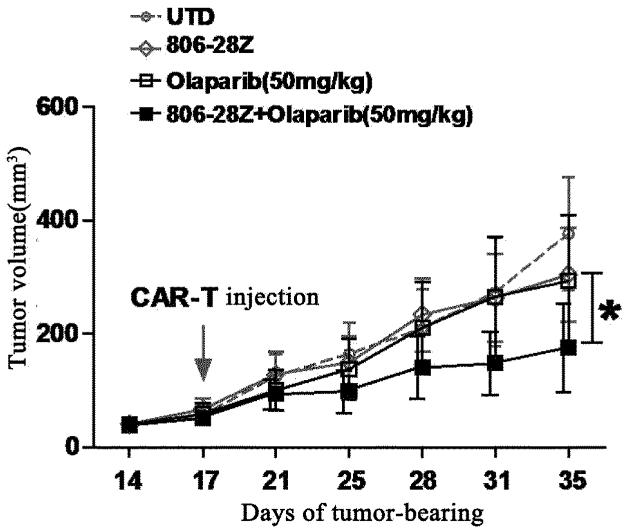


Fig. 6B

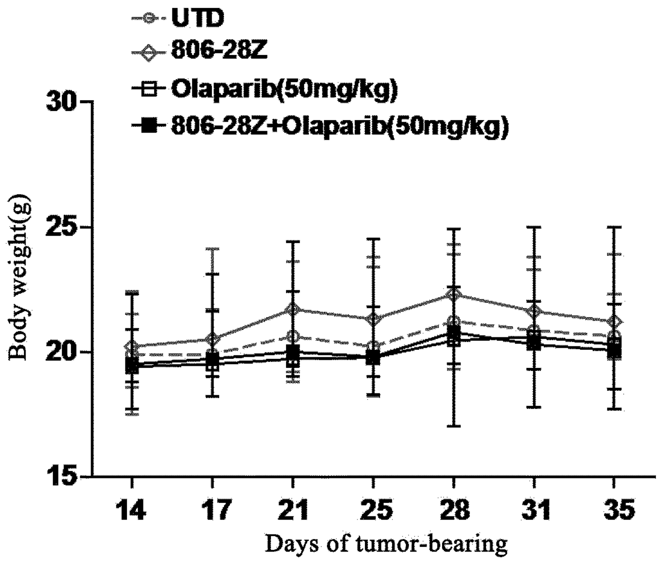


Fig. 6C

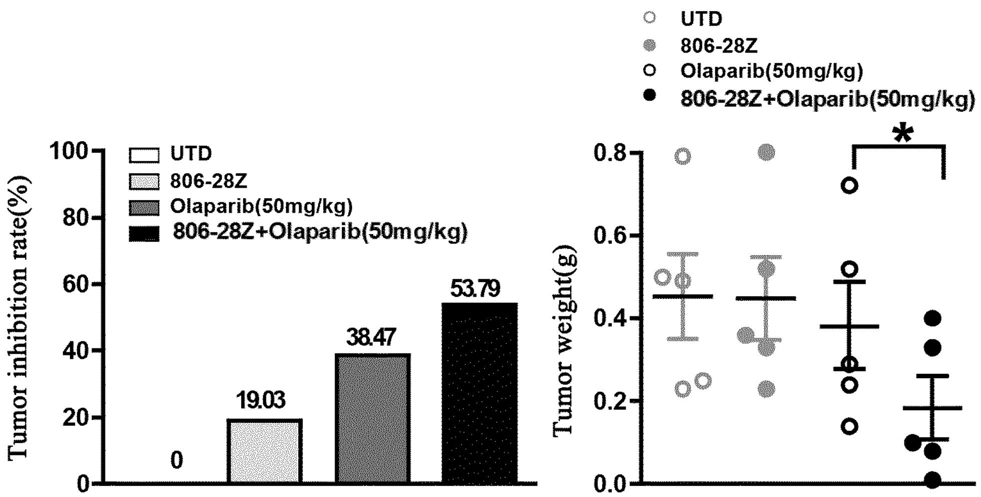
UTD
806-28Z
Olaparib(50mg/kg)
806-28Z+Olaparib(50mg/kg)
Tumor inhibition rate(%)
100
80
60
40
20
0
0
19.03
38.47
53.79
Tumor weight(g)
0.8
0.6
0.4
0.2
0.0
*

Fig. 6D

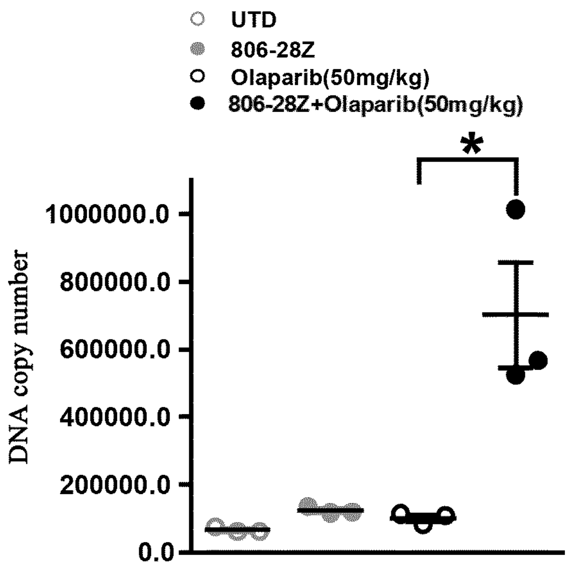
UTD
806-28Z
Olaparib(50mg/kg)
806-28Z+Olaparib(50mg/kg)
*
DNA copy number
1000000.0
800000.0
600000.0
400000.0
200000.0
0.0

Fig. 6E

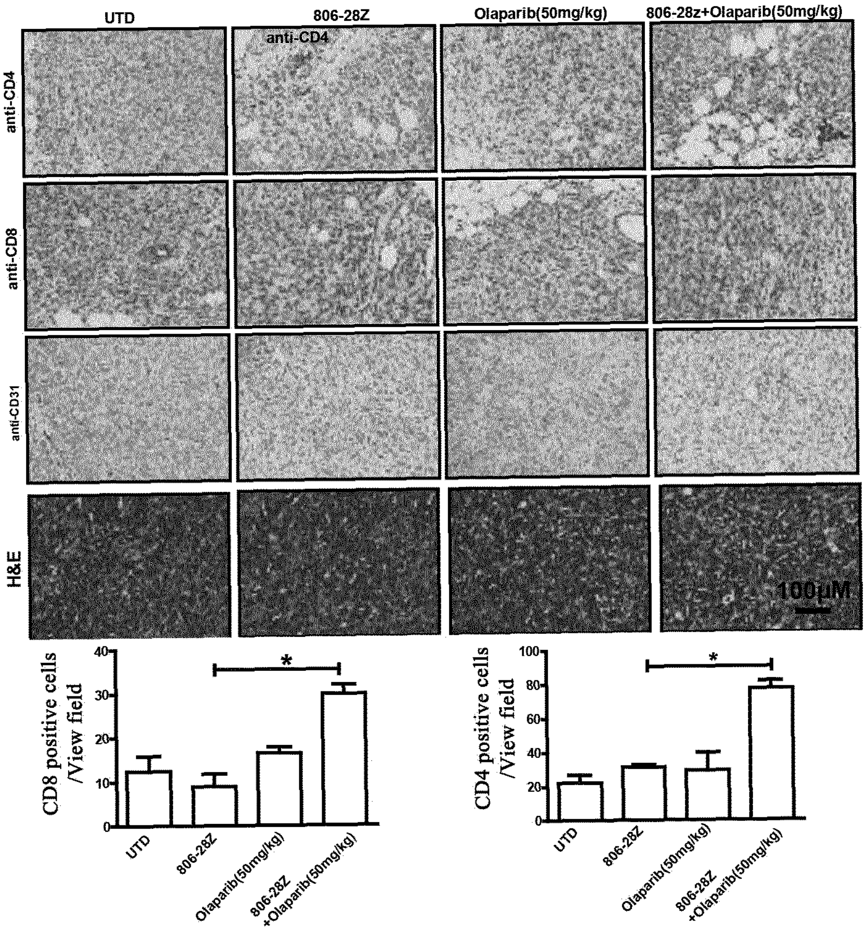
UTD
806-28Z
Olaparib(50mg/kg)
806-28z+Olaparib(50mg/kg)
anti-CD4
anti-CD4
anti-CD8
anti-CD31
H&E
100μM
CD8 positive cells /View field
CD4 positive cells /View field
*
*
UTD
806-28Z
Olaparib(50mg/kg)
806-28Z +Olaparib(50mg/kg)

Fig. 6F

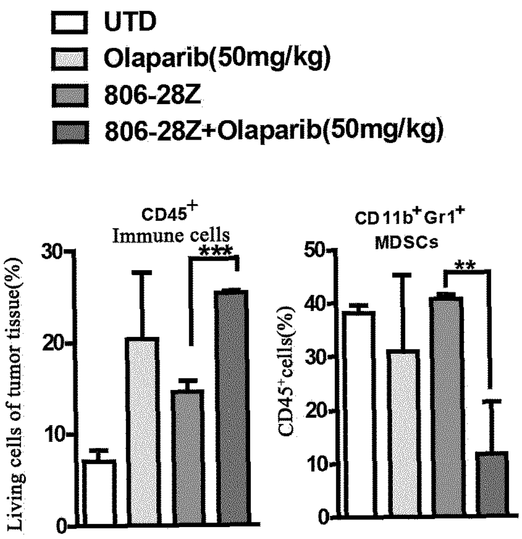
UTD
Olaparib(50mg/kg)
806-28Z
806-28Z+Olaparib(50mg/kg)
CD45+ Immune cells
CD11b+Gr1+ MDSCs
Living cells of tumor tissue(%)
CD45+cells(%)
***
**

Fig. 6G

**INTERNATIONAL SEARCH REPORT**

International application No.
**PCT/CN2019/124066**

**A. CLASSIFICATION OF SUBJECT MATTER**

A61P 35/00(2006.01)i; A61K 35/12(2015.01)i; A61K 31/00(2006.01)i

According to International Patent Classification (IPC) or to both national classification and IPC

**B. FIELDS SEARCHED**

Minimum documentation searched (classification system followed by classification symbols)

A61P, A61K

Documentation searched other than minimum documentation to the extent that such documents are included in the fields searched

Electronic data base consulted during the international search (name of data base and, where practicable, search terms used)

CNMED, CNABS, CPEA, DWPI, SIPOABS, AUABS, TWMED, ILABS, TWABS, HKABS, MOABS, SGABS, CNKI, NCBI, EMBL, GoogleScholar: T细胞, NK细胞, 免疫效应细胞, B细胞, PARP抑制剂, PARPi, pamiparib, fluzoparib, rucaparib, talazoparib, olaparib, mefuparib, niraparib, CAR-T, LAK, CTL

**C. DOCUMENTS CONSIDERED TO BE RELEVANT**

| Category* | Citation of document, with indication, where appropriate, of the relevant passages | Relevant to claim No. |
|---|---|---|
| X | CN 108601789 A (SENHWA BIOSCIENCES INC) 28 September 2018 (2018-09-28) abstract, description, page 5, claims 1-23 | 16-21, 25, 26 |
| A | CN 108601789 A (SENHWA BIOSCIENCES INC) 28 September 2018 (2018-09-28) see entire document | 22-24 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 February 2020** | **09 March 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

## INTERNATIONAL SEARCH REPORT

International application No.
**PCT/CN2019/124066**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **1-15**
because they relate to subject matter not required to be searched by this Authority, namely:

[1] Claims 1-15 relate to a tumour treatment method, which is a method for treatment of the human or animal body, and belongs to subject matter which does not require an international search, as listed in PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2019/124066**

| Patent document cited in search report | Publication date (day/month/year) | Patent family member(s) | Publication date (day/month/year) |
|---|---|---|---|
| CN 108601789 A | 28 September 2018 | CA 3005730 A1 | 26 May 2017 |
| | | IL 259423 D0 | 31 July 2018 |
| | | TW 201720833 A | 16 June 2017 |
| | | KR 20180113976 A | 17 October 2018 |
| | | JP 2018534321 A | 22 November 2018 |
| | | US 2019224209 A1 | 25 July 2019 |
| | | EP 3377068 A1 | 26 September 2018 |
| | | WO 2017087235 A1 | 26 May 2017 |
| | | AU 2016355268 A1 | 14 June 2018 |
| | | US 2017143737 A1 | 25 May 2017 |
| | | EP 3377068 A4 | 19 June 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4683195 A, Mullis
- CN 107058354 A
- CN 107460201 A
- CN 105194661 A
- CN 105315375 A
- CN 105713881 A
- CN 106146666 A
- CN 106519037 A
- CN 106554414 A
- CN 105331585 A
- CN 106397593 A
- CN 106467573 A
- CN 104140974 A
- WO 2017186121 A1
- WO 2018006882 A1
- WO 2015172339 A8
- WO 2018018958 A1


### Non-patent literature cited in the description


- **JESSICA HARTMANN et al.** Clinical development of CAR T cells-challenges and opportunities in translating innovative treatment concepts. *EMBO Molecule Medicine,* 01 August 2017
- **QIANG FU et al.** Progress of HER2 Signaling Pathway in Gastric Cancer and Clinical Application of Trastuzumab. *YAO PIN PING JIA,* 2012, vol. 9 (27), 8-12
- **FREDERICK M. AUSUBEL.** Current Protocols in Molecular Biology. Wiley and Son Inc, Library of Congress, 2000
- **SAMBROOK et al.** Molecular Cloning: A Laboratory Manual. Cold Spring Harbor Laboratory Press, 2001
- **M. J. GAITED.** *Oligonucleotide Synthesis,* 1984
- **B. D. HARRIES ; S. J. HIGGINSEDS.** *Nucleic Acid Hybridization,* 1984
- **B. D. HAMES ; S. J. HIGGINSEDS.** *Transcription And Translation,* 1984
- **R. I. FRESHNEY.** Culture Of Animal Cells. Alan R. Liss, Inc, 1987
- Immobilized Cells And Enzymes. IRL Press, 1986
- **B. PERBAL.** *A Practical Guide To Molecular Cloning,* 1984
- Methods In Enzymology. Academic Press, Inc,
- Gene Expression Technology
- Gene Transfer Vectors for Mammalian Cells. Cold Spring Harbor Laboratory, 1987
- Immunochemical Methods In Cell and Molecular Biology. Academic Press, 1987
- Hand book of Experimental Immunology. 1986, vol. I-IV
- Manipulating the Mouse Embryo. Cold Spring Harbor Laboratory Press, 1986
- Molecular Cloning Experiment Guide. Science Press, 2002
- **GAN HK et al.** Targeting of a conformationally exposed, tumor-specific epitope of EGFR as a strategy for cancer therapy. *Cancer Res,* 2012, vol. 72 (12), 2924-2930